(19) Europäisches Patentamt
European Patent Office
Office européen des brevets

(11) Publication number: 0 372 582 A2

# (12) EUROPEAN PATENT APPLICATION

(21) Application number: 89122711.8

(22) Date of filing: 08.12.89

(51) Int. Cl.5: C07D 477/00, A61K 31/40, //C07D205/08

(30) Priority: 09.12.88 US 281616

(43) Date of publication of application: 13.06.90 Bulletin 90/24

(84) Designated Contracting States: AT BE CH DE ES FR GB GR IT LI LU NL SE

(71) Applicant: BRISTOL-MYERS SQUIBB COMPANY
345 Park Avenue
New York, N.Y. 10154(US)

(72) Inventor: Banville, Jacques
1209 Girard
St. Hubert QC(CA)

(74) Representative: Kinzebach, Werner, Dr. et al
Patentanwälte Reitstötter, Kinzebach und Partner Sternwartstrasse 4 Postfach 86 06 49
D-8000 München 86(DE)

## (54) 6-Aminoalkyl carbapenems.

(57) The invention relates to carbapenem compounds of the above formulae

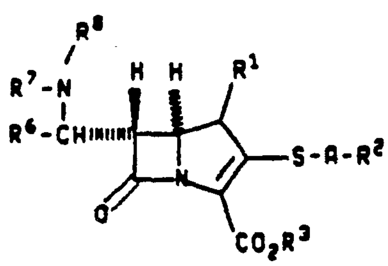

(V)

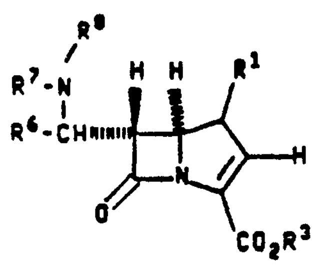

(XII)

wherein the substituents have the definitions given in claim 1. These compounds are useful for the treatment of infections.

EP 0 372 582 A2

## 6-AMINOALKYL CARBAPENEMS

### Field of the Invention

This invention relates to heterocyclic organic compcunds of the carbapenem series having a 1-aminoalkyl group in the 6-position

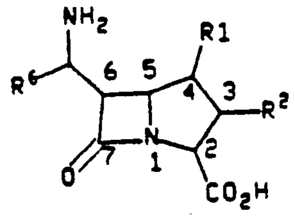

(I)

(Class 514, Subclass 302), and to drug, bio-affecting and body treating compositions (Class 514, subclass 210) containing these compounds since they possess antibiotic activity against bacteria, and other microorganisms.

### Nomenclature

The terminology for compounds of Formula I based upon the root name "carbapenem" employs a trivial system of nomenclature. The systematic name according to Chemical Abstracts, again referring to Formula I, is 4-$R^1$-3-$R^2$-6-(amino-$R^6$-methyl)-1-azabicyclo-[3,2,0]hept-2-ene-2-carboxylic acid. The positions are numbered in Formula I according to the Chemicl Abstracts system. The trivial "carbapenem" numbering system is as shown below in Formula IA.

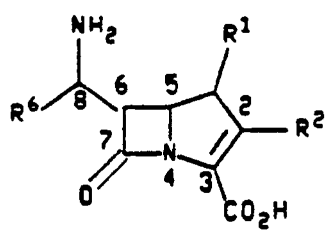

(IA)

### Description of the Prior Art

A great number of carbapenem antibiotics is known, the structures of which are generally characterized by a 1-hydroxyethyl group in the 6-position. This class of compounds is typefied by thienemycin, and its synthetic derivative imipenem which are the subjects of U. S. Patent Nos. 3,950,357 and 4,194,047 respectively patented April 13, 1976 and March 18, 1980.

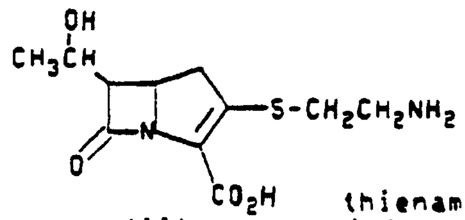

(II) thienamycin, imipenem is the N-formidoyl derivative

The present invention involves carbapenem antibiotic compounds of this type having an aminoalkyl group rather than a hydroxyethyl group at the 6-position, a 4$\beta$-alkyl substituent, and other structural characteristics.

The 6-aminoethyl analogs of thienamycin and imipenem have been described, in a conceptual vay, in European Patent Publication No. 17,992, published October 29, 1980. Refer to pages 6,7,8,95,96,and 153 and particularly to Example 22 (page 95) and Compound No. 18 on page 153. European Patent Publication 38,869 published November 4, 1981 on pages 46,47,56,77,78,79,80,83,84,88,89, and 90 contains a similar disclosure. The 4,4-dimethyl analog of the foregoing is conceptually illustrated at column 30, line 40 of U. S. Patent No. 4,341,706 patented July 27, 1982. 8-Azido carbapenems similar to Formula I above are disclosed as intermediates in the preparation of corresponding 8-(1,2,3-triazolo and tetrazolo) carbapenems, and 8-amino carbapenems in European Patent Publication No. 59,554 published September 8, 1982, and D. F. Corbett, Tetr. Letters 24, 5543 (1983). Refer to pages 15-18, 19-22, 35, and 36 of EP 59,554.

The following compounds are illustrated by example at the page/line locations indicated in European Patent Publication No. 10,316 published April 30, 1980, but characterizing data are not reported. The meanings indicated for $R^1$, $R^2$, and $R^6$ are with reference to Formula I above. Those structures are distinguished from the present compounds in that the $R^2$ substituent does not contain sulfur; and is not attached to the ring through sulfur.

Table 1

| Compounds of Formula I Disclosed in EP 10,136 | | | |
|---|---|---|---|
| $R^1$ | $R^2$ | $R^6$ | page/line |
| $CH_3$ | cyclopropyl | $CH_3$ | 67/1 - 80/11 |
| $CH_3$ | methyl | $CH_3$ | 84/19 |
| $CH_3$ | phenyl | $CH_3$ | 84/20 |
| $C_2H_5$ | cyclopropyl | $CH_3$ | 84/21 |
| $CH_3$ | isopropyl | H | 84/22 |
| $CH_3$ | 4-MeOphenyl | $C_2H_5$ | 84/23 |

European Patent Publication No. 50,927 published May 5, 1982, and S. Coulton, et al. J. Chem. Soc. Perkin Transact. 1, 3011 (1982), discloses a process for transforming a 6-hydroxyalkyl carbapenem corresponding to Formula I above into a 6-aminoalkyl carbapenem of Formula I with inversion of configuration, i.e. (S)OH becomes (R)$NH_2$, and (R)OH becomes (S)$NH_2$. The preparation of the following compound is disclosed on page 22.

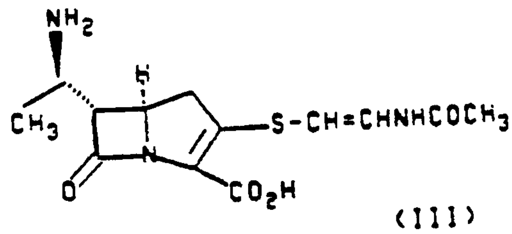

(III)

6-(1-Aminoethyl)carbapenems of Formula I above wherein $R^1$ is H, $R^6$ is methyl, and $R^2$ has the following meanings are disclosed by formula in Table V beginning at column 92, Compound Nos. 2, and 71-81 of U. S. Patent No. 4,543,257 patented September 24, 1985. Again, the $R^2$ substituents are not sulfur -- attached which is a significant structural difference from the present compounds.

$R^2$ is: phenyl
4-methoxyphenyl
4-hydroxymethylphenyl
4-aminomethylphenyl
1-propen-1-yl
3-diethylamino-1-propen-1-yl
1-cyclohept-1-ene
5-methyl-1-furyl
isothiazol-5-yl
thiazol-2-yl
1-methyltetrazol-5-yl

U. S. Patent Nos. 4,536,335, 4,642,341 and 4,644,061 patented respectively on August 20, 1985, February 30, 1987, and February 17, 1987 refer to 3-substituted carbapenem antibiotics carrying further substituents at the 4 and 6 positions and corresponding to Formula IV.

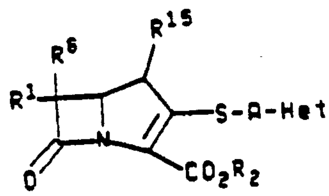

(IV)

$R^1$, $R^8$, and $R^{15}$ of Formula IV are inter alia substituted alkyl groups in which the substituents may be amino, or hydroxy. No specific 6-(1-aminoalkyl) compounds are named, pictured, or described, however. Het in Formula IV preferably refers to a quaternary ammonio hetercyclic group, and A may be an alkylene group. European Patent Publication 170,073 published February 5, 1986 refers to a similar series of compounds, but 6-(1-aminoalkyl) is not offered or suggested as an alternative to the 6-(1-hydroxyalkyl) substituent with which that disclosure is concerned.

## Summary Of The Invention

The present invention is concerned with the 6-(1-aminomethyl)-1-azabicyclo-[3,2,0]-hept-2-enes having Formulas V and XII

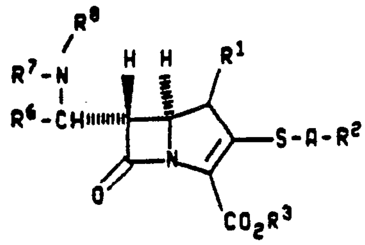

(V)

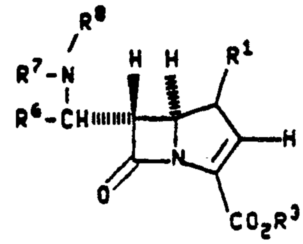

(XII)

wherein
$R^1$ is lower alkyl having 1 to 6 carbon atoms, R- or S- orientation,
A is a bond or it is a straight or branched alkylene group having 1 to 6 carbon atoms connecting the sulfur atom and $R^2$,
$R^2$ is hydrogen or a substituent other than amino but including methyl, hydroxy, cyano, $-CO_2R^3$, halo, carboxamido, carbamyl, phenyl, B-substituted phenyl, heterocyclo, or B-substituted heterocyclo, each of said heterocyclo having 4 to 8 ring members including at least one carbon, and from 1 to 4 heteroatoms selected from O, S, and N,
$R^3$ is H, lower alkyl having 1 to 6 atoms, aralkyl having 7 to 17 carbon atoms, an anionic charge, a salt forming group, a protective ester group, or a physiologically hydrolyzable ester group,
$R^6$ is hydrogen, lower alkyl having 1 to 6 carbon atoms, or phenyl, wherein each of said alkyl and said phenyl optionally has one or two substituents B,
$R^7$ and $R^8$ are selected independently from hydrogen, alkyl having 1 to 6 carbon atoms, aralkyl having 7 to 17 carbon atoms, said alkyl and aralkyl optionally having one or two substituents B, or one of $R^7$ and $R^8$ is carbacyl, sulfonyl, or phosphonyl having 1 to 17 carbon atoms, and the other is hydrogen
wherein said substituents B referred to herein are selected from halogen, nitro, methoxy, hydroxy, methyl, cyano, carboxy, carboxamido, sulfo, sulfonamido, carbamyl, and guanyl.

The compounds of Formula V and XII in which $R^6$ is other than hydrogen have the R- or S-configuration at the carbon atom bearing the $R^6$ substituent, and at the carbon atom bearing the $R^1$ substituent. Otherwise, the 1-azabicyclo [3.2.0]heptane ring structure of the products of Formula V has the same stereochemical configuration as the antibiotic thienamycin.

The compounds of Formula V are antibiotics and are toxic to or inhibit the growth of microorganisms when in contact therewith in a medium which otherwise supports the growth thereof. Sensitive microorganisms include Gram positive, Gram negative, and acid fast bacteria of both the aerobic and anaerobic types. These compounds may be used, for example, as animal feed additives for promotion of growth, as preservatives in food, as bactericides in industrial applications, for example in waterbased paint and in the white water of paper mills to inhibit the growth of harmful bacteria and as disinfectants for destroying or inhibiting the growth of harmful bacteria on medical and dental equipment. They are especially useful, however, in the treatment of infectious disease in humans and other animals caused by Gram-positive or Gram-negative bacteria.

The pharmaceutically active compounds of this invention may be used alone or formulated as pharmaceutical compositions comprising, in addition to the active carbapenem ingredient, a pharmaceutically acceptable carrier or diluent. The compounds may be administered by a variety of means; those of principal interest include: orally, topically including rectally or by inhalation or parenterally (intravenous or intramuscular injection). The pharmaceutical compositions may be in solid form such as capsules, tablets, powders, etc. or in liquid from such as solutions, suspensions or emulsions. Compositions for injection, the preferred route of delivery, may be prepared in unit dose form in ampules or in multidose containers and may contain formulatory agents such as suspending, stabilizing and dispersing agents. The compositions may be in ready to use form or in powder form for reconstitution at the time of delivery with a suitable vehicle such as sterile water.

The dosage to be administered depends to a large extent on the particular compound being used, the particular composition formulated, the route of administration, the nature and condition of the host and the particular situs and organism being treated. Selection of the particular preferred dosage and route of application, then is left to the discretion of the physician or veterinarian. In general, however, the compounds may be administered parenterally or orally to mammalian hosts in an amount of from about 5 to

200 mg/kg/day. Administration is generally carried out in divided doses, e.g. three to four times a day.

As in the case of other beta lactam antibiotics, the compounds of general Formulas V and XII are amphoteric and exist as zwitterions, and may be converted by known procedures to pharmaceutically acceptable salts which, for purposes of the present invention, are substantially equivalent to the parent compounds. Thus, for example, one may dissolve a compound of Formula V in a suitable inert solvent including water, and then add an equivalent of a pharmaceutically acceptable acid or base. The desired acid addition or base salt may be recovered by conventional procedures, e.g. solvent precipitation, lyophilization, etc.

A compound of Formula V wherein $R^3$ is hydrogen or an anionic charge, or a pharmaceutically acceptable salt may be converted by conventional procedures to a corresponding compound wherein $R^3$ is a physiologically hydrolyzable or protective ester group. A compound of Formula Y wherein $R^3$ is a conventional carboxyl protecting group may be converted to the corresponding compound wherein $R^3$ is hydrogen, an anionic charge or a physiologically hydrolyzable ester group, or a pharmaceutically acceptable salt thereof.

Included within the scope of this invention are methods for the treatment of infectious diseases of plants and animals including man with a compound of Formula V, and compositions useful for use in such methods.

The invention also includes a process for the synthesis of the compounds of Formula V which involves converting a 6-hydroxymethyl carbapenem or 3-hydroxymethyl azetidin-2-one precursor thereof to the corresponding azidomethyl compound, and thence to the aminomethyl compound as is more particularly described below.

## Detailed Description Of The Invention

The preparation of the compounds of the present invention is based upon any of several available beta lactam intermediates for the synthesis of carbapenem compounds of the thienamycin series having a 1-hydroxyalkyl group in the ring position adjacent to the lactam carbonyl group. The common feature in the process as applied to any of these intermediates is operation on the 1-hydroxyalkyl group to transform it into the 1-azidoalkyl group, and thence to the 1-aminoalkyl group as is shown in Scheme I.

**Scheme I**

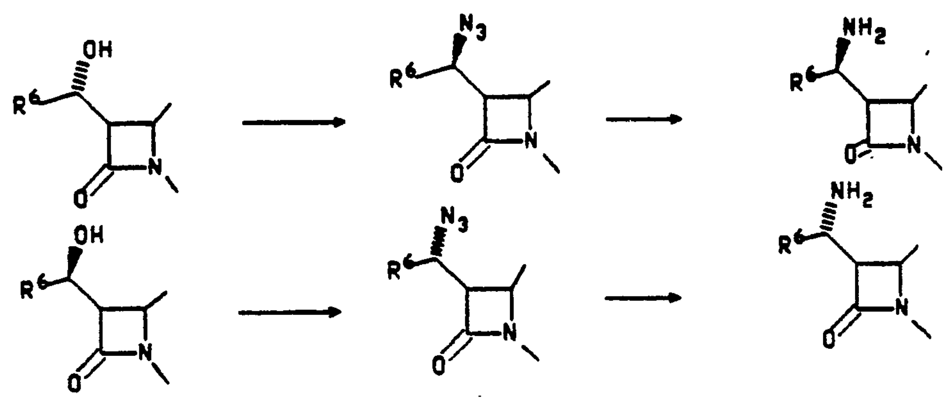

The method involves reaction of the 1-hydroxyalkyl starting material, in which other reaction sensitive substituents are protected by conventional protecting groups, with triphenylphosphine, hydrazoic acid ($HN_3$), and diethyl azodicarboxylate (DEAD). The azido group is substituted for the -OH group with inversion of configuration relative to the 1-hydroxyalkyl starting material i.e., the 1(S)-OH starting material yields the 1(R)-$N_3$ product, and the 1(R)-OH starting material yields the 1(S)-$N_3$ product. This method was first used by D.F. Corbett et al. (J. Chem. Soc. Perkin Trans. I, (1982), 3011, and Tetrahedron Letters 24, No. 49, pp. 5543-6 (1983)).

In cases where the hydroxyalkyl starting material of the required 1(S)- or 1(R)-configuration is not readily available, but the related starting material of the opposite configuration is available, the latter can be transformed into the former by the method of O. Mitsunobu Synthesis (1981), 1-28, or Mitsunobu et al., Bull.

Chem. Soc. Japan 49, 510 (1976). This method is similar to the above, and involves reaction of the hydroxy compound with triphenylphosphine, formic acid rather than hydrazoic acid, and DEAD to yield the formate ester of opposite configuration, i.e., 1(S)-OH yields 1(R)-OCHO, and 1(R)-OH yields 1(S)-OCH0.. The formate ester is then hydrolyzed to yield the required hydroxyalkyl starting material.

Intermediates of Formulas VI, VII, VIII, IX and X shown in Scheme II which follows wherein Z is OH are examples of available hydroxyalkyl substituted beta lactam intermediates which can be converted to the corresponding 1-azidoalkyl and 1-aminoalkyl products as described above, and thence to the products of the present invention by executing the remainder of the scheme with the corresponding aminoalkyl or azidoalkyl intermediate.

## SCHEME II

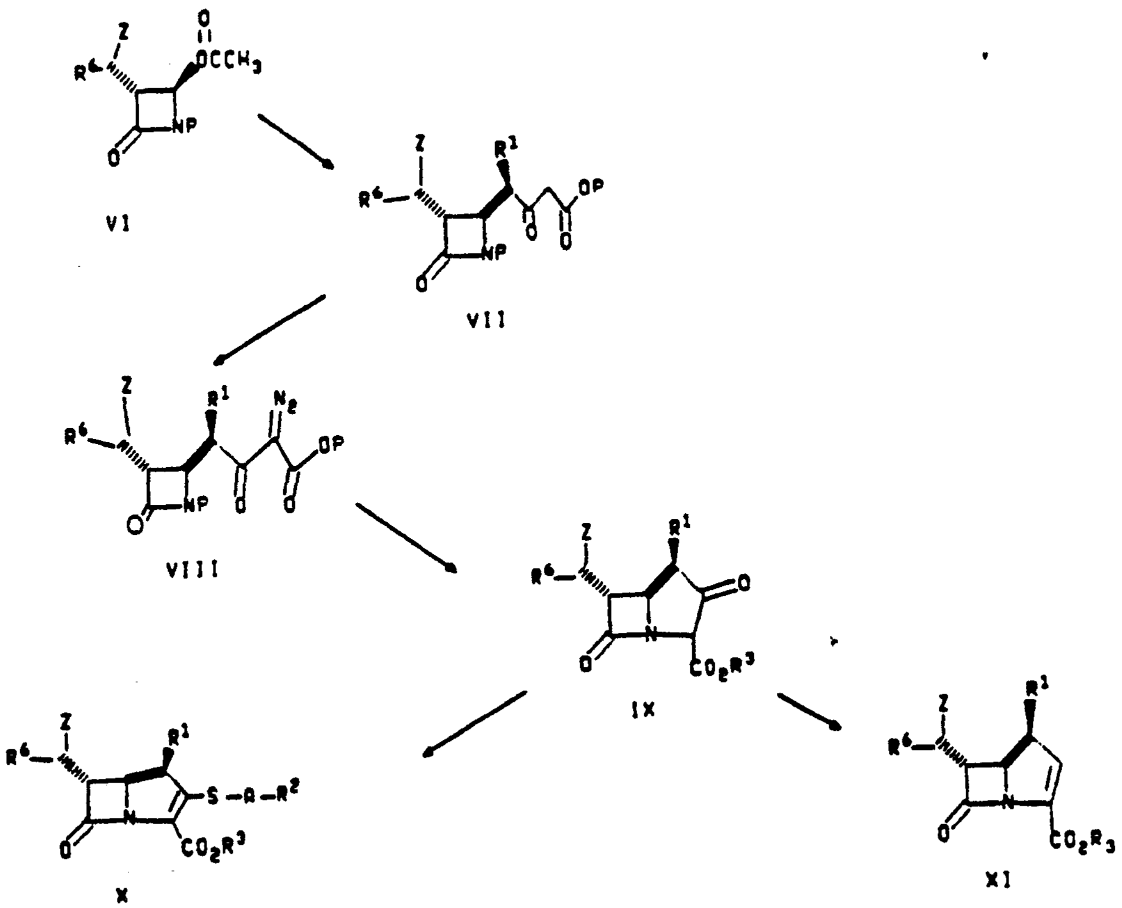

VI VII VIII IX X XI

In foregoing Scheme II, $R^1$, $R^2$, $R^3$, $R^6$, and A have the same meanings as previously. P is a carboxy or amino-protecting group as the case may be. Z is hydroxy, azido, amino, or $-NR^7R^8$. In Formulas VI, VII, VIII, IX, X, and XI the stereochemical configuration of the carbon atom bearing the $R^6$ group is intentionally not specified since either the R or the S configuration is suitable.

While any of the hydroxyalkyl intermediates of Formulas VI, VII, VIII, IX, X and XI may be converted to the corresponding azidoalkyl, or aminoalkyl intermediates, and thence further through the remainder of the sequence, using protecting groups as appropriate, to provide a product of the present invention of Formula V and Formula XII, we prefer to use compounds of Formulas VIII, IX, or X as starting materials. This is illustrated in the examples which follow.

The carbonyl group in the 3-position of the intermediate of Formula IX wherein Z is azido is subject to

reduction with sodium borohydride to yield the corresponding 3-ol compound. The latter on esterification with methanesulfonyl chloride yields the 3-O-mesylester which readily undergoes elimination of the elements of methanesulfonic acid to yield the compound of Formula XI. The latter conform in part to Formula XII, and may be converted to other compounds of Formula XII wherein $R^7$ or $R^8$ are other than hydrogen by alkylation of acylation of the amino, methyl group at the 6-position.

In summary, the process of the present invention involves reacting a first beta lactam compound of Formulas VI, VII, VIII, IX, or X wherein Z is OH with triphenylphosphine, hydrazoic acid, and diethyl azodicarboxylate under reaction conditions which are known to be appropriate for substitution of -OH by $-N_3$ to yield a second beta lactam compound of Formulas VI, VII, VIII, IX, or X wherein Z is azido, reducing the azido group of said second beta lactam compound by known means to provide a third beta lactam compound of Formulas VI, VII, VIII, IX, or X wherein Z is $-NH_2$. Either said second or said third beta lactam compound may be converted via the sequence VI---VII→ VIII→ IX→ X according to methods known to be suitable with said beta lactam compounds wherein Z is OH, with the use of protecting groups when necessary to avoid competing side reactions. Thereafter, if necessary, further transformations according to methods which are themselves known may be applied to convert a compound of Formula X wherein Z is azido or amino to a compound of Formula V.

For conversion of the 1-azidoalkyl compound to the 1-aminoalkyl compound, catalytic hydrogenation using a Pt or Pd catalyst at 1-3 atm. pressure of $H_2$ may be used. The Staudinger method is also applicable, and is in fact, preferred. The Staudinger method is a very mild, reducing method which is not destructive of other functional groups. According to this method, as practiced for instance by Bachi and Vaya (J. Org. Chem., 44, (1979), 4393), the phosphinimine is formed by reaction of the 1-azidoalkyl intermediate with triphenyphosphine. The phosphimimine is then exchanged with p-nitrobenzaldehyde to yield the Schiff base which is hydrolyzed.

Scheme III

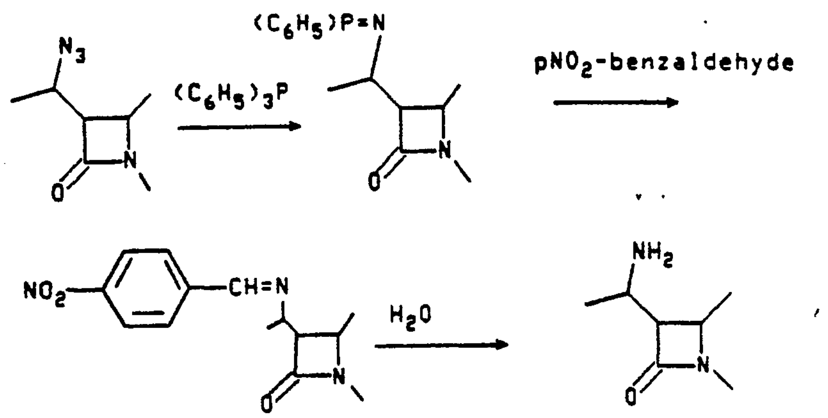


The following procedures illustrate the application of the process of this invention to the preparation of a variety of compounds of Formula V. In many instances, data illustrating the biological activity of the substances as antibiotics are presented. These data include minimum inhibitory concentrations ($MIC_{50}$) which are the concentrations determined by the tube dilution method in Nutrient Broth which reduces growth of the microorganism by 50%. It is determined by interpolation from a graph of concentration versus number of cells following exposure of the organism to the test compound under growth conditions. Another parameter such as optical density which is proportional to the cell count may be used. In some instances $MIC_{90}$ values are also given. The latter refers to 90% growth inhibition.

Chemical stability in aqueous solution at pH 7.4 and pH 2.0 is reported as half-life (t1/2). These values are determined by interpolation from a graph of concentration of test substance remaining in solution after storage under selected conditions for various periods of time.

$C_{max}$, t1/2, and AUC are blood concentration parameters determined after administering test compound to mice at a dose of 50 mg/kg of body weight. "$C_{max}$" is the maximum concentration achieved, "t1/2" is the half-life or time for half of the absorbed material to disappear from the blood stream.

The $PD_{50}$ values are protective doses, or the dose of test compound which secures survival of 50% of the animals of a test group, usually mice, during a designated period of time after being infected with an

inoculum of infectious organism which will result in death of all inoculated untreated animals during that test period.

The data show variously that the compounds have good Gram - and Gram + antibacterial activity, and stability at neutral and acidic pH. They are orally active, and more stable to the dehydropeptidase enzyme than the corresponding 6-(1-hydroxyethyl)carbapenems. They have improved activity against bacteria of the genus Pseudomonas over the analagous hydroxy compounds. Preferred compounds are listed below. Procedure Nos. are given in parenthesis.

(21) (5S,6R) 6-(1′R-aminoethyl)-4R-methyl-3-[(pyridin-3-yl)methylthio-7-oxo-1-azabicyclo-[3.2.0]-hept-2-ene-2-carboxylic acid

(26) (5S,6R) 6-(1′R-aminoethyl)-3-(2-cyanoethylthio)-4R-methyl-7-oxo-1-azabicyclo-[3.2.0]-hept-2-ene-2-carboxylic acid

## Description of Specific Embodiments

Referring to Scheme II, of the foregoing description, the application of compounds of Formula X wherein Z is OH to the process outlined in Scheme I is illustrated in Procedures 1-26. Operation on an intermediate of Formula VIII wherein Z is OH according to Scheme I, and thence applying the Scheme II sequence to the Formula VIII compounds wherein Z = $NH_2$ or $N_3$ is illustrated in Procedures 27-35.

## Abbreviations Used

DBN 1,5-diazabicyclo [4.3.0]non-S-ene.
DEAD diethyl azodicarboxylate
DIPEA diisopropylethylamine
DMAP 4-(N,N-dimethylaminopyridine
EEDQ 2-ethoxy-1-1ethoxycarbonyl-1,2-dihydroquinilone
EtOAc ethyl acetate
Gly glycine
Leu leucine
MsCl methanesulfonyl chloride
MeOH methanol
$pph_3$ triphenylphosphine
TEA triethylamine
THF tetrahydrofuran
TMS-Cl trimethylsilyl chloride

## Procedure 1

3S-(1′S-Hydroxyethyl)-4R-(1R-methyl-3-allyloxycarbonyl-2-oxo-3-diazopropyl)azetidin-2-one

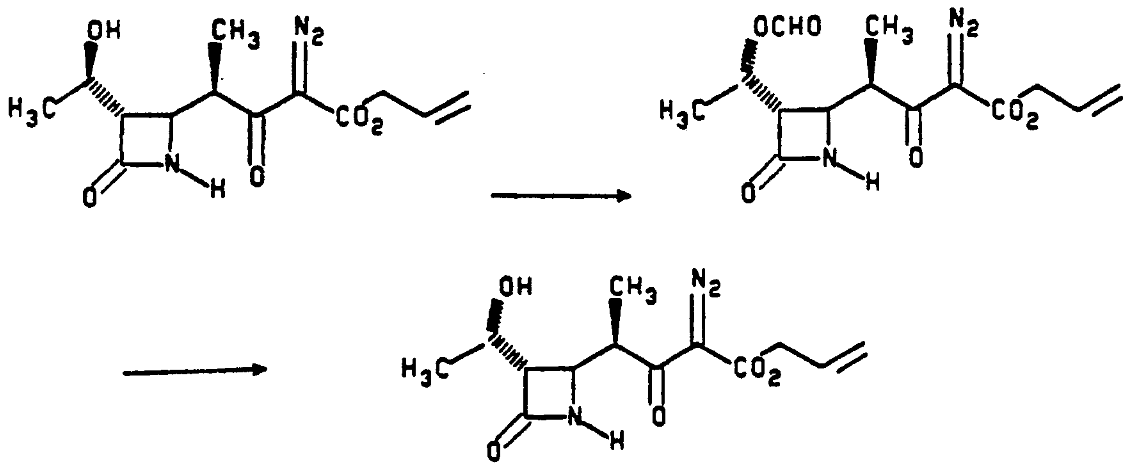

A solution of 3S-(1R-hydroxyethyl)-4R-(1R-methyl-3-allyloxycarbonyl-2-oxo-3-diazopropyl)azetidin-2-one (20.0 g, 67.7 mmol) in benzene (500 mL) was cooled to 5° C and treated with triphenylphosphine (28.4 g, 0.108 mol) and 98% formic acid (4.1 mL, 0.108 mol). Then diethyl azodicarboxylate (17 mL, 0.108 mol) was added dropwise over 10 min and the resulting mixture was stirred at 5-10° C for 1 h and then at 22° C for another hour. The reaction mixture was then filtrated to remove the diethyl hydrazinedicarboxylate. The filtrate was then diluted with ethyl acetate (500 mL), washed with saturated sodium bicarbonate, brine and then dried over anhydrous magnesium sulfate. The solvent was then evaporated under reduced pressure and the residual oil was chromatographed on a silicagel pad (10 x 10 cm) to remove any unreacted starting material. Elution with toluene and then with a gradient of ethyl acetate (0-40%) and toluene gave the intermediate formyl derivative. This crude product was dissolved with ethanol (400 mL), treated with 1.0N hydrochloric acid (80 mL) and stirred at 22° C for 20 h. The solvent was then concentrated under vacuum and the residue taken in ethyl acetate (600 mL). The organic phase was washed with saturated sodium bicarbonate brine and then dried over magnesium sulfate. Evaporation of the solvent and chromatography of the residue on silicagel (9 x 8 cm, elution toluene and ethyl acetate 7:3 to ethyl acetate) gave the title material as a white solid. Crystallization from a mixture of ethyl acetate and hexanes gave 6.67 g (33%) of the 1S-hydroxyethyl derivative: mp 118-119° C (dec).

ir (KBr)$\nu_{max}$: 3430 and 3200 (OH and NH), 2239 ($N_2$), 1730, 1712 and 1692 $cm^{-1}$ (C=O); ′Hmr ($CDCl_3$) δ: 1.18 (d, J=6.66, 3H $CH_3$-1 of diazopropyl), 1.30 (d, J=6.40, 3H, $CH_3CHOH$), 2.20 (d, J=5.12, 1H, OH), 2.99 (dd, $J_{H3,H4}$=1,74, $J_{H3,H1'}$=593, 1H, H-3), 3.75 (dd, $J_{H4,H3}$=174, $J_{H4,H1''}$=5.52, 1H, H-4), 3.80 (m, 1H, H-1 of diazopropyl), 4.06 (m, 1H, $CH_3CHOH$), 4,72 (m, 2H, $CH_2$ of allyl), 5.3-5.4 (m, 2H, CH of allyl), 5.8-6.1 ppm (m, 2H, NH and CH of allyl).

| | | | |
|---|---|---|---|
| Anal. Calcd. for $C_{13}H_{17}N_3O_5$: | C, 52.88; | H 5.80; | N, 14.23 |
| found: | C, 52.83; | H 5.80; | N, 14.22 |

Procedure 2

(5S,6S) Allyl 6-(1′S-hydroxyethyl)-4R-methyl-3,7-dioxo-1-azabicyclo[3.2.0]heptane-2-carboxylate

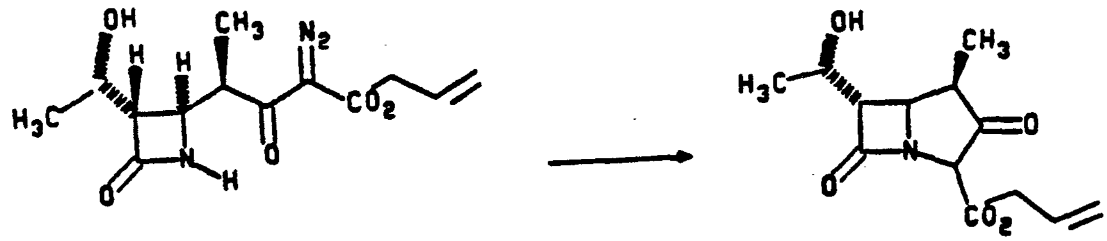

A solution of 3S-(1S-hydroxyethyl)-4R-(1R-methyl-3-allyloxycarbonyl-2-oxo-3-diazopropyl)azetidin-2-one (3.69 g, 12.49 mmol) in ethyl acetate (40 mL) was treated with rhodium octanoate dimer (0.040 g) and the resulting mixture was heated at 60-65°C for 40 min. The solvent was evaporated under reduced pressure and the residual oil was used as such in Procedure 3.
ir (NaCl film) $\nu_{max}$: 1755 (C=O of $\beta$-lactam), 1740 $cm^{-1}$ (C=O of ketone and ester);
$^{1}$Hmr ($CDCl_3$) $\delta$: 1.20 (d, J=7.8, 3H, $CH_3$-4), 1.38 (d, J=6.30, 3H, $CH_3CHOH$), 4.0-4.5 (m, 2H, H-5 and H-1′ overlapping), 4.6-4.8 (m, 3H, H-2 and $CH_2$ of allyl), 5.2-5.5 and 5.6-6.2 ppm (m, 3H, CH of allyl).

## Procedure 3

(5S,6S) Allyl 6-(1S-hydroxyethyl)-4R-methyl-3-methylthio-7-oxo-1-azabicyclo[3.2.0]hept-2-ene-2-carboxylate

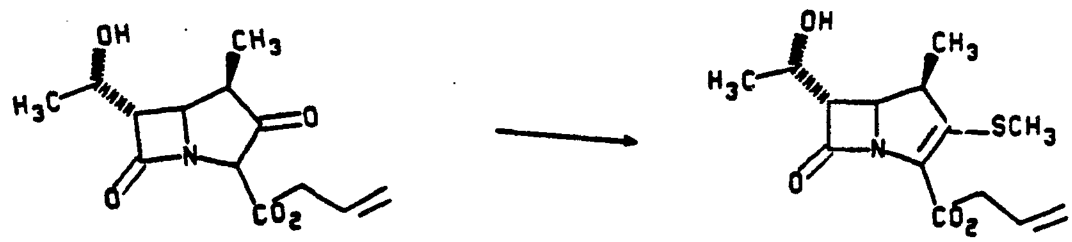

Method A: Without hydroxyl protection

A solution of (5S,6S) allyl 6-(1S-hydroxyethyl)-4S-methyl-3,7-dioxo-1-azabicyclo[3.2.0]heptane-2-carboxylate (3.34 g, 12.49 mmol) in acetonitrile (25 mL) was cooled to -5°C and treated with diphenyl chlorophosphate (2.79 mL, 13.48 mmol). Then N,N-diisopropylethylamine (2.34 mL, 13.48 mmol) was added followed by excess methyl mercaptan (~4.0 g, 0.08 mol). The reaction mixture was then stirred at 0±5°C for 3 h and then poured onto crushed ice. The mixture was then extracted with ethyl acetate (500 mL) and the organic phase was washed with water, cold saturated sodium bicarbonate and brine. After drying over magnesium sulfate, the solvent was evaporated under reduced pressure and the residue chromatographed on silica gel (4.5 x 12 cm). Elution with a mixture of toluene and ethyl acetate (1:1) first gave 3.10 g of an oil which, by ir and $^{1}$Hmr, corresponds to the thioester resulting from opening of the $\beta$-lactam of the enol phosphate by methyl mercaptan. Elution with the same solvent system gave 1.52 g (42%) of the title material which was obtained as an oil. Crystallization from a mixture of ethyl acetate and hexanes gave white prisms: mp 81-83°C; [$\alpha_D^{22}$ + 151° (c 1.0, $CHCl_3$);
uv (ETOH)$\lambda_{max}$: 320 (9,050);
ir (KBr)$\nu_{max}$: 3550 (OH), 1742 (C=O of $\beta$-lactam), 1690 $cm^{-1}$ (C=O of ester);
$^{1}$Hmr ($CDCl_3$) $\delta$: 1.24 (d, J=7.25, 3H, $CH_3$-4), 1.34 (d, J-6.34, 3H, $CH_3CHOH$), 2.37 (s, 3H, $SCH_3$-3), 3.30 (dd, $J_{H6,H5}$=2.50, $J_{H5H1'}$=6.20, 1H, H-6), 3.35 (m, 1H, H-4), 4.07 (dd, $J_{H5,H6}$=2.50, $J_{H5,H4}$=8.96, 1H, H-5), 4.15 (m, 1H, $CH_3CHOH$), 4.6-4.9 (m, 2H, $CH_3$ of allyl), 5.2-5.5 and 5.8-6.1 ppm (m, 3H, CH of allyl).

| | | | |
|---|---|---|---|
| Anal. calcd. for $C_{14}H_{19}NO_4S$: | C, 56.55; | H 6.44; | N, 4.71 |
| found: | C, 56.60; | H 6.51; | N, 4.73 |

Method B: Via silyl ether protection

A solution of (5S,6S) allyl 6-(1S-hydroxyethyl)-4R-methyl-3,7-dioxo-1-azabicyclo[3.2.0]heptane-2-carboxylate (23.90 g, 89.63 mmol) in acetonitrile (300 mL) was cooled to 0°C and treated with diphenyl chlorophosphate (20.5 mL, 98.9 mmol). The N,N-diisoprophylethyl amine (17.22 mL, 98.9 mmol) was added dropwise over 10 min followed by a small crystal of 4-N,N-dimethylaminopyridine. The reaction mixture was stirred at 0°C for 2.5 h and then N,N-diiospriopylethylamine (17.22 mL, 98.9 mmol), followed by chlorotrimethylsilane (12.67 mL, 99.8 mmol) were added dropwise over 10 min. After 15 min, the mixture was finally treated with N,N-diisopropylethylamine (17.22 mL, 98.9 mmol) and excess methyl mercaptan ~7.0 g, 0.145 mol). After 18 h at 0°C, the reaction mixture was diluted with ethyl acetate (1 L) and washed successively with brine, saturated sodium bicarbonate and brine. After drying over anhydrous magnesium sulfate, the mixture was concentrated under reduced pressure to give an oil. This oil was taken in a mixture of tetrahydrofuran (250 mL) and water (250 mL) and treated with acetic acid (5 mL). After 15 min at 22°C, the mixture was diluted with ethyl acetate (1 L) and the organic phase washed successively with saturated sodium bicarbonate and brine. After drying over anhydrous sodium sulfate, evaporation of the solvent under reduced pressure, gave an oil which was chromatographed on silicagel (9.5 x 11 cm). Elution with a mixture of dichloromethane and acetonitrile (0-30%) gave the title material as an oil (22.0 g, 82%) which was shown to be pure by ′Hmr. Crystallization from a mixture of ethyl acetate and hexanes gave white prisms (mp 81-83°C). Spectral data for this material (ir, ′Hmr) were identical to that given for Method A of this procedure.

Procedure 4

(5S,6S) Potassium 6-(1′S-hydroxyethyl)-4R-methyl-3-methylthio-7-oxo-1-azabicyclo[3.2.0]hept-2-ene-2-carboxylate

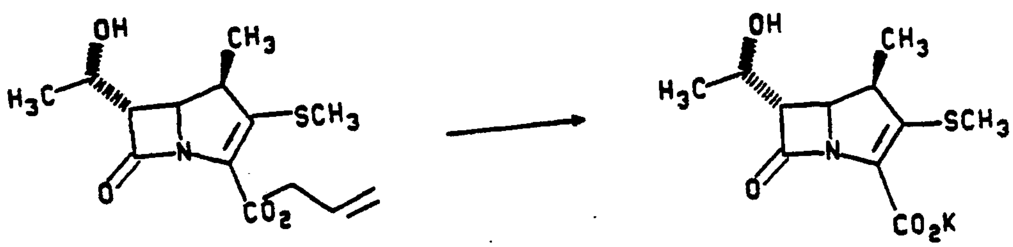

A solution of (5S,6S) allyl 6-(1S-hydroxyethyl)-4R-methyl-3-methylthio-7-oxo-1-azabicyclo[3.2.0]hept-2-ene-2-carboxylate (0.300 g, 1.0 mmol) in dichloromethane (15 mL) was treated at 22°C with triphenylphosphine (0.020 g) and tetrakis(triphenylphosphine)palladium (0) (0.020 g). Then a 0.5 M solution of potassium 2-ethylhexanoate (2.0 mL, 1.0 mmol) in ethyl acetate was added and the mixture was stirred for 30 min. The reaction mixture was extracted with water (2 x 20 mL) and the combined aqueous phases were chromatographed on reversed phase silicagel (μ-Bondapak c-18, 2.5 x 14 cm) using water and acetonitrile (0-5%) as eluent. The title material was obtained as a white amorphous powder: 0.249 g (83%); $[\alpha]_D^{22}$ + 166.2° (c 1.0, $H_2O$); hplc on μ-Bondapak c-18, 4 mm x 30 cm, 5% $CH_3CN$-$H_2O$ pH 7.4 phosphate buffer, flow rate 1 mL/min, uv detector at 300 nm, retention time 7.4 min, purity 99.9%;
uv ($H_2O$, pH 7.4 phosphate buffer) $\lambda_{max}$: 304 nm (10,690);
ir (KBr)$\nu_{max}$: 1748 (C=O of β-lactam), 1592 $cm^{-1}$ (C=O of carboxylate);
′Hmr ($D_2O$) δ: 1.19 (d, J-7.22, 3H, $CH_3$-4), 1.33 (d, J=6.47, 3H, $C_3$CHOH), 2.36 (s, 3H, $SCH_3$-3), 3.5 (m, 2H, H-6 and H-4 overlapping), 4.10 (dd, $J_{H5,H6}$=2.25, $J_{H5,H4}$=8.77, 1H, H-5), 4.20 ppm (m, becomes a doublet

$J_{H1',H6}$ = 5.20 upon irradiation of CH₃CHOH, 1H, CH₃CHOH).

By uv this carbapenem was found to have a half-life of 160 h at 37° C in pH 7.4 phosphate buffer.

## Procedure 5

(5S,6S) Allyl 3-Ethylthio-6-(1'S-hydroxyethyl)-4R-methyl-7-oxo-1-azabicyclo[3.2.0]hept-2-ene-2-carboxylate

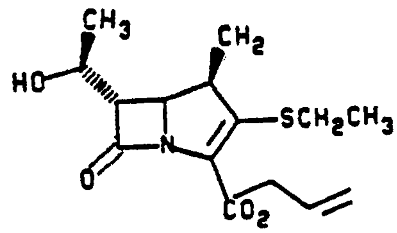

Method B of Procedure 3 was modified by the substitution of ethanthiol for the methyl mercaptan on a chemically equivalent basis. Chromatographic purification using toluene and ethyl acetate (1:1) gave 1.30 g (41%) of the title carbapenem as an oil;

ir (NaCl, film) $\nu_{max}$: 3480 (OH), 1765 (C=O of β-lactam) and 1702 cm⁻¹ (C=O of ester);

'Hmr (200 MHz, $CDCl_3$) δ: 1.26 (d, J=7.26 Hz, 3H, $CH_3$-4), 1.33 (t, J=7.44 Hz, 3H, $SCH_2CH_3$), 1.36 (d, J=6.33 Hz, 3H, $CH_3CHN$), 2.9 (m, 2H, $SCH_2CH_3$), 3.31 (dd, $J_{H6,H5}$=2.60 Hz, $J_{H6,H1}$=6.10 Hz, 1H, H-6), 3.34 (m, 1H, H-4), 4.09 (dd, $J_{H5,H6}$=2.60 Hz, $J_{H5,H4}$=9.03 Hz, 1H, H-5), 4.19 (m, 1H, $CH_3CHO$), 4.6-4.9 (m, 2H, $CH_2$ of allyl), 5.2-5.5 and 5.9-6.1 ppm (m, 3H, CH of allyl).

## Procedure 6

(5S,6S) Allyl 6-(1'R-Azidoethyl)-3-ethylthio-4R-methyl-7-oxo-1-azabicyclo[3.2.0]hept-2-ene-2-carboxylate

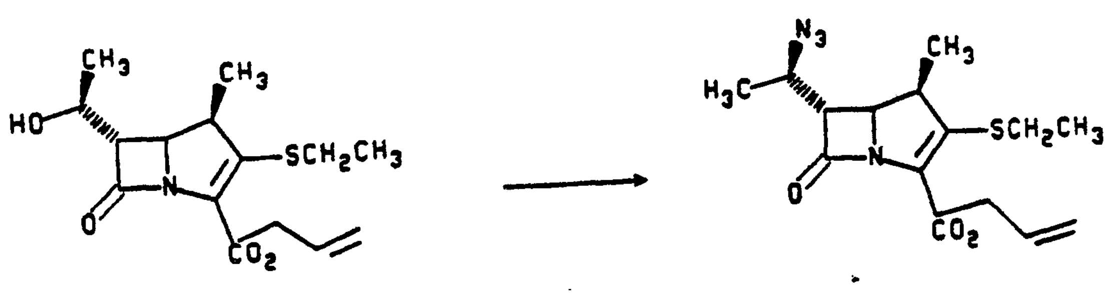

A solution of (5S,6S) allyl 3-ethylthio-6-(1'S-hydroxyethyl)-4R-methyl-7-xox-1-azabicyclo[3.2.0]hept-2-ene-2-carboxylate (1.30 g, 4.17 mmol) in dry tetrahydrofuran (THF) (60 mL) was cooled to 0.5° C and treated, under $N_2$, with triphenylphosphine (2.19 g, 8.34 mmol) and 7.0 mL (5.95 mmol) of a 0.85M solution of hydrazoic acid in toluene. Then diethyl azodicarboxylate (1.32 mL, 8.38 mmol) was added dropwise over 5 min and the resulting solution was stirred for 20 min. The reaction mixture was then quenched by addition of saturated aqueous $NaHCO_3$ and then extracted with ethyl acetate (EtOAc) (300 mL). The organic phase was then washed with brine, dried ($MgSO_4$) and evaporated under reduced pressure. The residue was triturated with toluene and filtered to remove the crystalline diethyl hydrazinedicarboxylate. Chromatography of the filtrate on silica gel (3 x 12 cm) using a mixture of toluene and EtOAc (8:2) gave 0.76 g (54%) of the title carbapenem as an oil;

ir (NaCl, film) $\nu_{max}$: 2108 ($N_3$), 1775 (C=O of $\beta$-lactam) and 1705 $cm^{-1}$ (C=O of ester);
$^{1}$Hmr (200 MHz, $CDCl_3$) $\delta$: 1.26 (d, J=7.31 Hz, 3H, $CH_3$-4), 1.33 (t, J-7.48 Hz, 3H, $SCH_3CH_3$), 1.47 (d, J=6.53 Hz, 3H, $CH_3CHN$), 2.84 (m, 2H, $SCH_2CH_3$), 3.19 (dd, $J_{H6,H5}$=2.47 Hz, $J_{H6,H1}$=9.28 Hz, 1H, H-6), 3.39 dq, $J_{H4,CH3}$=7.31 Hz, $J_{H4,H5}$=9.0 Hz, 1H, H-4), 3.90 (dq, $J_{H1'}$,CH3=6.53 Hz, $J_{H1'}$,H6=9.28 Hz, 1H, H-1'), 4.11 (dd, $J_{H5,H6}$=2.47 Hz, $J_{H5,H4}$=9.0 Hz, 1H, H-5), 5.6-5.9 (m, 2H, $CH_2$ of allyl), 5.2-5.5 and 5.9-6.1 ppm (m, 3H, CH of allyl).

## Procedure 7

(5S,6R) 6-(1'R-Aminoethyl)-3-ethylthio-4R-methyl-7-oxo-1-azabicyclo[3.2.0]hept-2-ene-2-carboxylic Acid (41116)

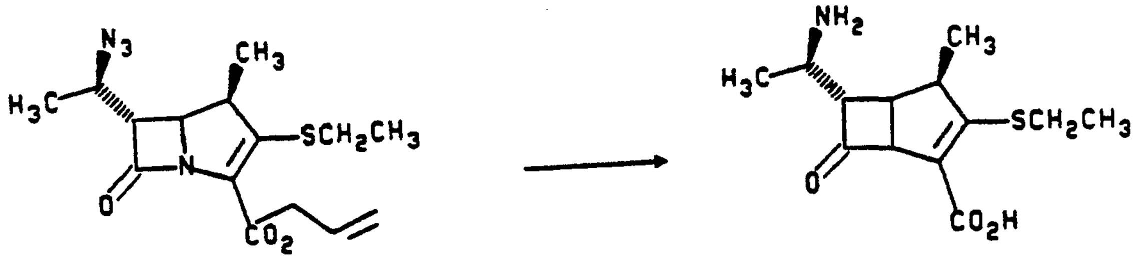

A solution of (5S,6S) allyl 6-(1'R-azidoethyl)-3-ethylthio-4R-methyl-7-oxo-1-azabicyclo[3.2.0]hept-2-ene-2-carboxylate (0.72 g, 2.14 mmol) and triphenylphosphine (0.72 g, 2.74 mmol) in dry benzene (15 mL) and under $N_2$ was heated under reflux for 45 min. The cooled reaction mixture was then treated with p-nitrobenzaldehyde (0.36 g, 2.38 mmol) and stirred at 22° C for 18 h. The solvent was then evaporated and the crude Schiff base was disolved in dry methylene chloride (25 mL). this solution was then treated with tetrakis(triphenylphosphine)palladium (0) (0.070 g) and 4.7 mL (2.35 mmol) of a 0.5M solution of potassium 2-ethylhexanoate in EtOAc at 22° C for 30 min. Then aqueous 0.2M pH 6.0 phosphate buffer (15 mL) was added and the resulting mixture was stirred vigorously for 1 h. The aqueous phase was then collected and the organic phase was extracted twice with water (2 x 20 mL). The combined aqueous extracts were concentrated to ~20 mL under vacuum (T <10° C) and chromatographed on reversed phase silica gel ($\mu$-Bondapak c-18, 3 x 13 cm). Elution with a mixture of water and acetonitrile (95:5) gave 0.24 g (42%) of the title carbapenem as a white amorphous solid after freeze drying: $[\alpha]_D^{22}$ + 122° (c 1.0, $H_2O$); hplc on $\mu$-Bondapak c-18, 4 mm x 30 cm, 2% $CH_3CN$-$H_2O$ pH 7.4 phosphate buffer, flow rate 2 mL/min, uv detector 304 nm, retention time 7.3 min, purity 99%;
uv ($H_2O$, pH 7.4 phosphate buffer) $\lambda_{max}$: 304 nm (8,700);
ir (KBr)$\nu_{max}$: 1750 (C=O of $\beta$-lactam) and 1590 $cm^{-1}$ (C=O of carboxylate);
$^{1}$Hmr (200 MHz, $D_2O$) $\delta$: 1.22 (d, J=7.01 Hz, 3H, $CH_3$-4), 1.27 (t, J=7.40 Hz, 3H, $SCH_2CH_3$) 1.47 (d, J=6.63 Hz, 3H, $CH_3CHN$), 2.84 (m, 2H, $SCH_2CH_3$), 3.47 (dq, $J_{H4,CH3}$=7.0 Hz, $J_{H4,H5}$=8.76 Hz, 1H, H-4), 3.58 (dd, $J_{H6,H5}$=2.35 Hz, $J_{H6,H1}$=8.19 Hz, 1H, H-6) 3.84 (dq, $J_{H1'}$,CH3=6.63 Hz $J_{H1'}$,H6=8.19 Hz, 1H, $CH_3CHN$) and 4.20 ppm (d, $J_{H5,H6}$=2.35 Hz, $J_{H5,H4}$=8.76 Hz, 1H, H-5).

By uv, at 37° C in a pH 7.4 phosphate buffer, this carbapenem was found to have a half-life of 4.3 h. By hplc at 37° C in a pH 2.0 citric acid buffer, the half-life was evaluated to be 168 min.

| M.I.C. | |
|---|---|
| Str. pneu A9585 | 1.000 |
| Str. pyo. A9604 | 2.000 |
| Str. faec.A20688 | 125.000 |
| Staph. aur. A9537 | 2.000 |
| Staph. serum | 16.000 |
| Staph aur. Pen + | 4.000 |
| Staph aur. MR | 125.000 |
| E. coli A15119 | 0.030 |
| E. Coli A20341 | 0.030 |
| K. pneu. A9664 | 2.000 |
| K. pneu.A20468 | 2.000 |
| Ent.clo.A9659 | 2.000 |
| Ent.clo.A9656 | 2.000 |
| P.mir. A9900 | 0.016 |
| P.vul. A21559 | 0.500 |
| P. morg. A15153 | 0.250 |
| P.rett.A22424 | 8.000 |
| Ser. mars. A20019 | 8.000 |
| Ps. aeru. A 9843 | 32.000 |
| Ps. aeru. CarbR | 32.000 |
| t1/2, pH 7.4 (h.): | 4.3 |
| t1/2, pH 2 (min.): | 168 |

## Procedure 8

(5S,6S) Allyl 6-(1′Azidoethyl)-4R-methyl-3-methylthio-7-oxo-1-azabicyclo[3.2.0]-2-ene-2-carboxylate

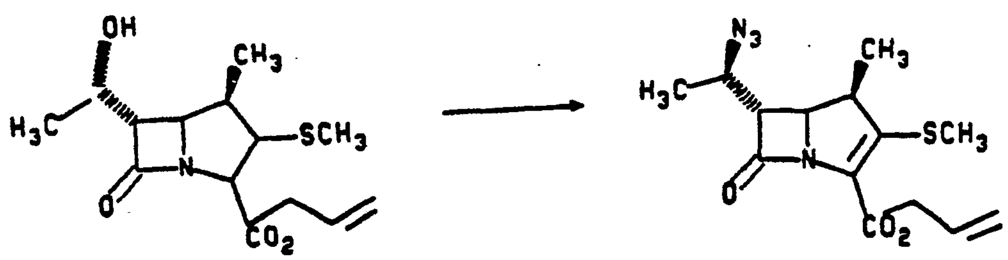

A solution of (5S,6S) allyl 6-(1′S-hydroxyethyl)-4R-methyl-3-methylthio-7-oxo-1-azabicyclo[3.2.0]hept-2-ene-2-carboxylate (1.45 g, 4.87 mmol) from Procedure 3 in dry tetrahydrofuran (60 mL) was cooled to 0 °C and treated with triphenylphosphine (2.55 g, 9.72 mmol) followed by a 0.58 M solution of hydrazoic acid (16.8 mL, 9.74 mmol) in toluene. Then diethyl azodicarboxylate (1.53 mL, 9.70 mmol) was added dropwise over 3 min. After 10 min at 0 °C, the reaction mixture was quenched by addition of saturated sodium bicarbonate (100 mL) and ethyl acetate (400 mL). The organic phase was washed with brine, dried over anhydrous magnesium sulfate and evaporated under reduced pressure. The semi-solid residue was triturated with toluene (20 mL) and filtered to remove the diethyl hydrazinedicarboxylate. The filtrate was chromatographed on silicagel (3 x 14 cm) using toluene and an ethyl acetate (0-5%) gradient. The title azido compound was obtained as a clear oil (1.145 g, 73%). Crystallization from a mixture of ethyl acetate, ether and hexanes gave white prisms: mp 70-71 °C; $[\alpha]_D^{22}$ + 148° (c 1.0, $CHCl_3$);
uv (EtOH) λ: 318 nm (10,650);
ir (KBr)$\nu_{max}$: 2130 and 2090 ($N_3$), 1770 (C=O of β-lactam) and 1705 $cm^{-1}$ (C=O of ester);

$^{1}$Hmr ($CDCl_3$) δ: 1.24 (d, J=7.25, 3H, $CH_3$-4), 1.45 (d, J=6.52, 3H, $CH_3CHN_3$), 2.38 (s, 3H, $SCH_3$-3), 3.18 (dd, $J_{H6,H5}$=2.45, $J_{6,H1}$=9.32, 1H, H-6), 3.39 (dq, $J_{H4,H5}$=8.95, $J_{H4,CH3}$=7.25, 1H, H-4), 3.89 (dq, $J_{H1'}$,H6=9.32, $J_{H1'}$,CH3=6.52, 1H, H-1'), 4.09 (dd, $J_{H5,H6}$=2.45, $J_{H5,H4}$=8.95, 1H, H-5), 4.6-4.9 (m, 2H, $CH_2$ of allyl), 5.2-5.5 and 5.9-6.1 (m, 3H, CH of allyl).

| Anal. calcd. for $C_{14}H_{18}N_4O_3S$: | | | | |
|---|---|---|---|---|
| | C, 52.16; | H, 5.63; | N, 17.38; | S, 9.95 |
| found: | C, 52.16; | H, 5.75; | N, 17.40; | S, 9.91 |

## Procedure 9

(5S,6R) 6-(1'R-Aminoethyl)-4R-methyl-3-methylthio-7-oxo-1-azabicyclo[3.2.0]hept-2-ene-2-carboxylic Acid (40591)

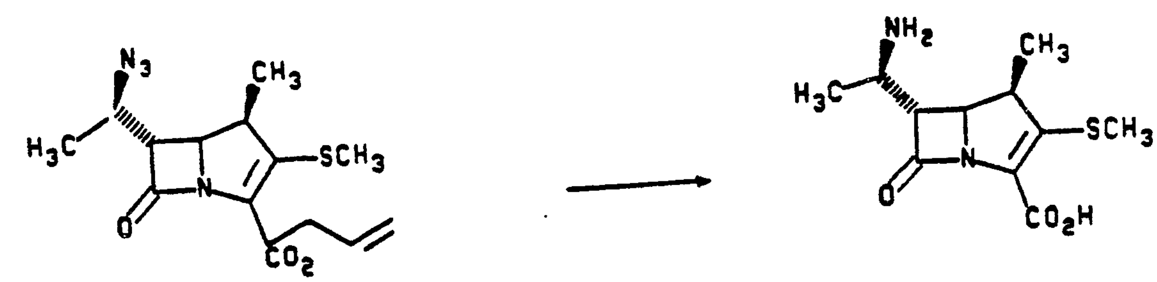

Method A: by hydrogenation

A solution of (5S,6S) allyl 6-(1'R-azidoethyl)-4R-methyl-3-methylthio-7-oxo-1-azabicyclo[3.2.0]hept-2-ene-2-carboxylate (1.14 g, 3.53 mmol) from Procedure 8 in dichloromethane (30 mL) was cooled to 0°C and treated with tetrakis (triphenylphosphine) palladium (0) (0.080 g) and then a 0.5M solution of potassium 2-ethylhexanoate (7.0 mL, 3.5 mmol) in ethyl acetate. After 15 min at 0°C, the reaction mixture was extracted with water (3 x 20 mL) and the combined aqueous extracts were chromatographed on reversed phase silicagel (μ-Bondapak c-18, 3.5 x 15 cm). Elution was done with water and an acetonitrile gradient (0-5%). The uv active fractions were combined and concentrated to ~50 mL under vacuum. Water (100 mL), 0.2M pH 7.0 phosphate buffer (30 mL) and 30% palladium on celite (2.0 g) were added and the mixture was hydrogenated at 22°C in a Parr apparatus for 15 min. The catalyst was filtered and the filtrate was chromatographed on reversed phase silicagel (μ-Bondapak c-18, 3.5 x 10 cm). Elution with a mixture of water and acetonitrile (0-5%) and lyophilization of the uv active fractions gave a white solid (0.44 g). A second chromatography of this material gave 0.170 g of the title material as an amorphous powder. Crystallization from water (2mL) gave 0.072 g (8%) of the pure product as white prisms: hplc on μ-bondapak c-18, 4 mm x 30 cm, 2% $CH_3CN$-$H_2O$ pH 7.4 phosphate buffer, flow rate 1 mL/min, uv detector at 304 nm, retention time 6.35 min, purity 99.7%;
uv ($H_2O$, pH 7.4 phosphate buffer) $\lambda_{max}$: 301 nm (10,841)
ir (KBr)$\nu_{max}$: 1732 (C=O of β-lactam), 1570 $cm^{-1}$ (C=O of carboxylate)
$^{1}$Hmr ($D_2O$) δ: 1.22 (d, J=7.25, 3H, $CH_3$-4), 1.49 (d, J=6.69, 3H, $CH_3CHNH_2$), 2.37 (s, 3H, $SCH_3$-3), 3.51 (m, 1H, H-4), 3.59 (dd, $J_{H6,H5}$=2.30, $J_{H6,H1'}$=8.25, 1H, H-6), 3.87 (dq, $J_{H1'}$,H6=8.25, $J_{H1'}$,CH3=6.69, 1H, H1'), 4.19 ppm (dd, $J_{H5,H6}$=2.30, $J_{H5,H4}$=8.67, 1H, H-5).

By uv, this carbapenem was found to have a half-life of 6.8 h at 37°C in a pH 7.4 phosphate buffer. At 22°C in a pH 7.4 phosphate buffer the half-life was evaluated to be 39 h.

Method B via the phosphinimine

A solution of (5S,6R) allyl 6-(1'R-azidoethyl)-4R-methyl-3-methylthio-7-oxo-1-azabicyclo[3.2.0]hept-2-ene-2-carboxylate (4.28 g, 13.27 mmol) in dry benzene (85 mL) was treated with triphenylphosphine (4.28 g, 16.31 mmol) and the resulting solution was heated under reflux for 45 min. The cooled solution was then treated with p-nitrobenzaldehyde (2.20 g, 14.6 mmol), stirred at 22° C for 4 h and then allowed to stand at 5° C for 16 h. The solvent was then evaporated *in vacuo* and the residual crude Schiff base was dissolved in $CH_2Cl_2$ (100 mL). Then tetrakis(triphenylphosphine) palladium (0) (0.400 g) and 29 mL (14.5 mmol) of a 0.5M solution of potassium 2-ethylhexanoate in ethyl acetate were added and the resulting solution was stirred at 22° C for 20 min. Then 100 mL of 0.2M pH 6 aqueous phosphate buffer were added and the two phases system was stirred vigorously for 2 h. The aqueous phase was then collected and maintained under vacuum to remove traces of organic solvent. Chromatography of the aqueous phase on reversed phase silicagel (μ-Bondapak c-18, 5 x 13 cm, elution $H_2O$ to $H_2O$-$CH_3CN$ 5%) gave 1.42 g (41%) of the title amino acid as a white powder after freeze drying. This material was pure by 'Hmr and hplc (98%). Crystallization from water (~10 mL) gave 0.960 g (28%) of the title compound as white prisms;
uv ($H_2O$, pH 7.4 phosphate buffer) $\lambda_{max}$: 304 nm (8,770);

| Anal. calcd. for $C_{11}H_{16}N_2O_3S$: | | | | | |
|---|---|---|---|---|---|
| | C, 51.54; | H, 6.29; | N, 10.93; | S, 12.51 | |
| found: | C, 47.43; | H, 6.48; | N, 10.85; | S, 11.11; | $H_2O$ 6.55% |

The mother liquors (0.538 g) still contained some amino acid (94% by hplc).

| M.I.C. | | |
|---|---|---|
| Str. Pneu. A 9585 | 16.000 | |
| Str. pyo. A 9604 | 16.000 | |
| Str. faec. A20688 | 125.000 | |
| Staph aur. A9537 | 16.000 | |
| Staph serum | 32.000 | |
| Staph aur. Pen+ | 16.000 | |
| Staph aur. MR | 125.000 | |
| E. coli A15119 | 0.130 | |
| E. coli A20341 | 0.130 | |
| K. pneu. A9664 | 0.500 | |
| K. pneu. A20468 | 0.500 | |
| Ent. clo.A9659 | 0.500 | |
| Ent. clo.A9656 | 0.500 | |
| P.mir. A9900 | 0.060 | |
| P.vul. A21559 | 0.130 | |
| P.morg. A15153 | 0.130 | |
| P.rett. A22424 | 8.000 | |
| Ser.mars. A20019 | 0.250 | |
| Ps. aeur. A9843 | 1.000 | |
| Ps. aeur. CarbR | 4.000 | |
| PD50, | IM | PO |
| S. aur. | | |
| E. coli | 25.00 | 12.50 |
| t1/2, pH 7.4 (h.): | | 6.8 |
| t1/2, pH 2 (min.): | | 212 |
| DHP: | | 0.19 |
| Cmax, PO/50: | | 9 |
| t1/2: | | 18 |
| AUC; | | 5 |

Procedure 10

(5S,6S) 3-Benzylthio-6-(1′S-hydroxyethyl)-4R-metyl-7-oxo-1-azabicyclo[3.2.0]hept-2-ene-2-carboxylate

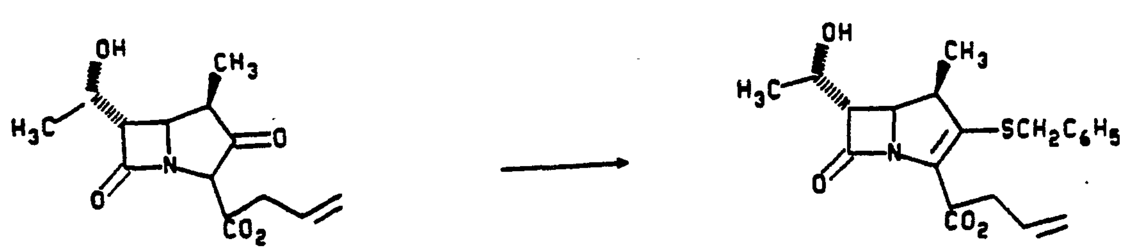

Method A of Procedure 3 was modified by the substitution of benzyl mercaptan in equivalent amounts for the methyl mercaptan specified there. Elution of the product from silica gel was with a mixture of toluene and EtOAc (8:2) and gave 2.54 g (67%) of the title carbapenem as a clear oil:
ir (NaCl, film)$\nu_{max}$: 3480 (OH), 1765 (C=O of $\beta$-lactam), 1700 $cm^{-1}$ (C=O of ester);

'Hmr (200 MHz, $CDCl_3$) δ: 1.25 (d, J=7.29 Hz, 3H, $CH_3$4), 1.33 (d, J=6.25 Hz, eH, $CH_3CHO$), 3.30 (dd, $J_{H5,H5}$=2.53 Hz, $J_{H6,H1'}$=5.87 Hz, 1H, H-6), 3.31 (m overlapping with H-6, 1H, H-4), 4.01 (dd, $J_{H5,H6}$=2.53 Hz, $J_{H5,H4}$=9.09 H, 1H, H-5), 4.06 (AB q overlapping with H-5 and H-1, 2H, $CH_2$ of benzyl), 4.15 (m, 1H, $CH_3CHO$), 4.75 (m, 2H, $CH_2$ of allyl), 5.2-5.5 and 5.9-6.1 (m, 3H, CH of allyl) and 7.2-7.4 ppm (m, 5H, aromatics).

## Procedure 11

Allyl (5S,6S)-6-(1'R-Azidoethyl)-3-benzylthio-4R-methyl-7-oxo-1-azabicyclo[3.2.0]hept-2-ene-2-carboxylate

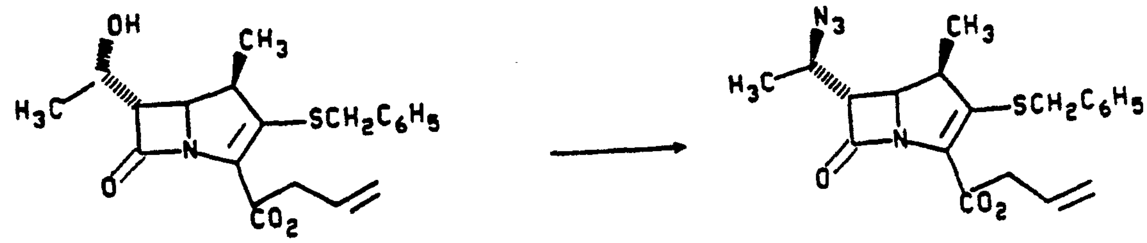

Allyl (5S,6S)-3-benzylthio-6-(1'S-hydroxyethyl)-4R-methyl-7-oxo-1-azabicyclo[3.2.0]hept-2-ene-2-carboxylate from Procedure 10 was treated with triphenylphosphine, hydrazoic acid, and diethyl azodicarboxylate as described in Procedure 6. After removal of the crystalline diethyl hydrazinedicarboxylate, chromatography of the filtrate on silica gel (3.5 x 12 cm, elution toluene: EtOAc, 8:2) gave 0.92 g (34%) of the title carbapenem as an oil;

ir (NaCl, film)$\nu_{max}$: 2115 ($N_3$), 1775 (C=O of β-lactam) and 1705 $cm^{-1}$ (C=O of ester);

'Hmr (200 MHz, $CDCl_3$) δ: 1.25 (d, J=7.29 Hz, 3H, $CH_3$-4), 1.46 (d, J=6.52 Hz, 3H, $CH_3CNHN$), 3.18 (dd, $J_{H6,H5}$=2.52 Hz, $J_{H6,H1}$=9.17 Hz, 1H, H-4), 3.88 (dq, $J_{H1'}$,CH3=6.52 Hz, $J_{H1'}$,H6=9.17 Hz, 1H, $CH_3CHN_3$), 4.02 (dd, $J_{H5,H6}$=2.52 Hz, $J_{H5,H4}$=9.09 Hz, 1H, H-5), 4.07 (ABq, $J_{AB}$=13.16 Hz, Δv=5.8 Hz, 2H, $CH_2$ of benzyl, 4.75 (m, 2H, $CH_2$ of allyl), 5.2-5.5 and 5.9-6.1 (m, 3H, CH of allyl) and 7.3 ppm (m, 5H, aromatics).

## Procedure 12

(5S,6S) 6-(1'R-Aminoethyl)-3-benzylthio-4R-methyl-7-oxo-1-azabicyclo[3.2.0]hept-2-ene-2-carboxylic Acid (41118)

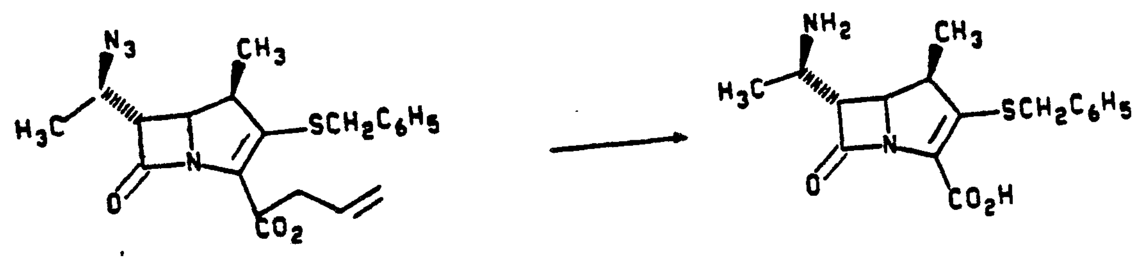

Allyl (5S,6S)-6-(1'R-azidoethyl)-3-benzylthio-4R-methyl-7-oxo-1-azabicyclo[3.2.0]hept-2-ene-2-carboxylate (0.60 g, 1.50 mmol) from Procedure 11 was treated with triphenylphosphine and p-nitrobenazldehyde as described in Procedure 9, Method B. The crude Schiff base was recovered as an oil;

ir (NaCl, film)$\nu_{max}$: 1770 (C=O of β-lactam) and 1705 $cm^{-1}$ (C=O of ester);

'Hmr (200 MHz, $CDCl_3$-) δ: 1.28 (d, J=7.31 Hz, 3H, $CH_3$-4), 1.43 (d, J=6.39 Hz, 3H, $CH_3CHN$), 3.30 (m, 1H,

H-4), 3.52 (dd, $J_{H6,H5}$ = 2.56 Ha, $J_{H6,H1}$ = 7.86 Hz, 1H, H-6, 3.88 (m, 1H, $CH_3CHN$), 4.02 (dd overlapping with $CH_2$ of benzyl, $J_{H5,H6}$ = 2.57 Hz, 1H, H-5), 4.40 (broad s, 2H, $CH_2$ of benzyl), 4.7 (m, 2H, $CH_2$ of allyl), 5.2-5.5 and 5.9-6.1 (m, 3H, CH of allyl), 7.89 (d, J = 8.82 Hz, 2H, H ortho of p-nitrobenzylidene), 8.28 (d, J = 8.82 Hz, H meta of p-nitrobenzylidene) and 7.43 ppm (s, 1H, CH = N).

The crude Schiff base allyl ester was then deallylated and hydrolyzed by treatment with tetrakis-(triphenylphosphine) palladium (0) and potassium 2-ethyl hexanoate as described. Chromatography on reversed phase silica gel (μ-Bondapak c-18, 3 x 13 cm, and elution with a mixture of water and $CH_3CN$ 7:3) gave 0.15 g (30%) of the title carbapenem as a white amorphous powder after freeze drying: hplc on μ-Bondapak c-18, 4 mm x 30 cm, 15% $CH_3CN$-$H_2O$, pH 7.4 phosphate buffer, flow rate 2 mL/min, uv detector 304 nm, retention time 7.2 min, purity 99%;

uv ($H_2O$, pH 7.4 phosphate buffer) $\lambda_{max}$: 304 nm (9,550);

ir (KBr)$\nu_{max}$: 1750 (C = O of β-lactam) and 1580 $cm^{-1}$ (C = O of carboxylate);

′Hmr (200 MHz, $D_2O$) δ: 1.19 (d, J-7.22 Hz, 3H, $CH_3$-4), 1.45 (d, J = 6.62 Hz, 3H, $CH_3CHN$), 3.42 (m, 1H, H-4), 3.57 (dd, $J_{H6,H5}$ = 2.34 Hz, $J_{H6,H1'}$Y = 8.22 Hz, 1H, H-6), 3.80 (dd, $J_{H1',CH3}$ = 6.62 Hz, $J_{H1',H6}$ = 8.22 Hz, 1H, $CH_3CHN$), 4.06 (dd overlapping with $CH_2$ of benzyl, $J_{H5,H6}$ = 2.34 Hz, 1H, H-5), 4.10 (ABq, $J_{AB}$ = 13.48, Δv = 15.4 Hz, 2H, $CH_2$ of benzyl) and 7.4 ppm (m, 5H, aromatics).

By uv, at 37° C in a pH of 7.4 phosphate buffer, this carbapenem has a half-life of 4.1 h. By hplc in a pH 2.0 citric acid buffer at 37° C, the half-life was evaluated to be 144 min.

| M.I.C. | |
|---|---|
| Str. pneu A9585 | 0.500 |
| Str. pyo. A9604 | 16.000 |
| Str. faec. A20688 | 125.000 |
| Staph. aur. A9537 | 1.000 |
| Staph. serum | 32.000 |
| Staph aur. Pen + | 2.000 |
| Staph aur. MR | 125.000 |
| E. coli A15119 | 0.030 |
| E. coli A20341 | 0.030 |
| K. pneu. A9664 | 0.500 |
| K. pneu. A20468 | 0.250 |
| Ent. clo. A9659 | 0.130 |
| Ent. clo. A9656 | 0.250 |
| P. mir. A9900 | 0.008 |
| P. vul. A21559 | 0.016 |
| P. morg. A15153 | 0.130 |
| P. rett. A22424 | 2.000 |
| Ser. mars. A20019 | 0.130 |
| Ps. aeru A9843 | 32.000 |
| Ps. aeru CarbR | 32.000 |
| t1/2, pH 7.4 (h.): | 4.1 |
| t1/2, pH 2 (min.): | 144 |
| Cmax, PO/50: | 33 |
| t1/2: | 13 |
| AUC: | 11 |

## Procedure 13

(5S,6S)Allyl 6-(1′S-Hydroxyethyl)-4R-methyl-3-[(1-methyl-1,2,3-triazol-4-yl)methylthio]-7-oxo-1-azabicyclo-[3.2.0]hept-2-ene-2-carboxylate

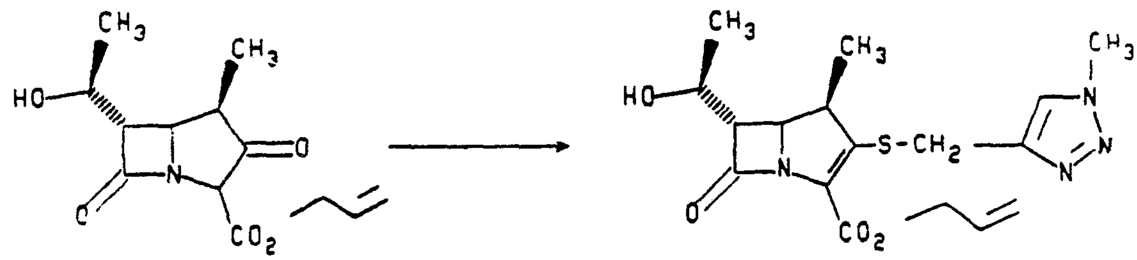

A solution of (5S,6S) allyl 6-(1′S-hydroxyethyl)-4R-methyl-3,7-dioxo-1-azabicyclo[3.2.0]heptane-2-carboxylate from Procedure 2 (11.0 mmol) in dry $CH_3CN$ (45mL) was cooled to 0-5°C and treated under $N_2$ with diphenyl chlorophosphate (2.44 mL, 11.55 mmol) and N, N-diisopropylethylamine (2.0 mL, 11.55 mmol). 4-N,N-Dimethylaminopyridine (0.003g) was then added and the solution was stirred for 30 min. Then 1-methyl-4-thiomethyl-1,2,3-triazole(1.50 g, 11.06 mmol) in $CH_3CN$ (5 mL) and N, N-diisopropylethylamine (2.3 mL, 13.2 mmol) were added and the mixture was stirred at 0-5°C for 1h. The reaction mixture was then diluted with EtOAc (300 mL), washed with brine and dried ($MgSO_4$). Evaporation of the solvent gave an oil which was chromatographed on silica gel (200 g). Elution with EtOAc and then $CH_3CN$ gave 2.74 g (66%) of the title carbapenem as a solid. Recrystallization from a mixture of EtOAc and hexanes gave white needles: mp 117° C; [a] $^{23}_{D}$+ 14° (c 1.0, $CHCl_3$);
uv (EtOH)$\lambda_{max}$: 320 nm (12,670); ir (KBr) $_{max}$: 1785 (C=O of β-lactam) and 1712 $Cm^{-1}$ (C=O of ester);
′Hmr (200 MHz, $CDCl_3$)δ: 1.27 (d, J=7.24 Hz, 3H, $CH_3$-4), 1.34 (d, J=6.33 Hz, 3H, $CH_3CHO$), 1.82 (broad 1H,OH), 3.29 (dd, $J_{H6,H5}$ = 2.58 $H_z$, $J_{H6,H1,}$ = 6.02 Hz, 1H, H-6), 3.69 (dq, $J_{4,CH3}$= 7.24 Hz, $J_{H4,H5}$=9.1 Hz, 1H, H-4), 4.02 (dd, $J_{H5,H6}$=2.58 Hz,, $J_{H5,H4}$=9.1 Hz, 1H, H-5), 4.07 (s, 3H, $CH_3$-1 of triazole, 4.10 (ABq, $J_{AB}$= 15.02 Hz, = 67.8 Hz, 2H, $SCH_2$-3), 4.15 (m, 1H, $CH_3CHO$), 4.73 (m, 2H, $CH_2$ of allyl), 5.2-5.5 and 5.9-6.1 (m, 3H, CH of allyl) and 7.49 ppm (s, 1H, H-5 of triazole).

| | | | | |
|---|---|---|---|---|
| Anal. calcd. for $C_{17}H_{22}N_4O_4S$: | C 53.95, | H 5.86, | N 14.80, | S 8.47; |
| found: | C 53.75, | H 5.87, | N 14.64, | S 8.59. |

## Procedure 14

(5S,6S)Allyl 6-(1′R-Azidoethyl)-4R-methyl-3-[(1-methyl-1,2,3-triazol-4-yl)methylthio]-7-oxo-1-azabicyclo-[3.2.0]hept-2-ene-2-carboxylate

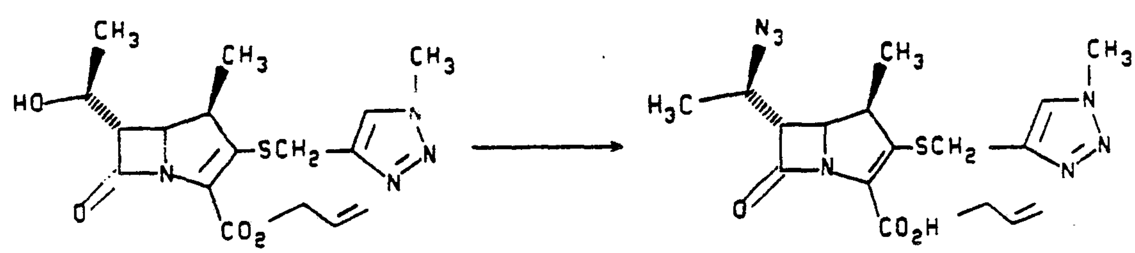

Allyl 6-(1′S-hydroxyethyl)-4R-methyl-3-[(1-methyl-1,2,3-triazol-4-yl)methylthiol]-7-oxo-1-azabicyclo-[3.2.0]hept--2-ene-2-carboxylate (1.74 g, 4.60 mmol) was treated with triphenylphosphine acid, and diethyl azodicarboxylate as described in Procedure 8. Chromatography on silicagel (3 x 14 cm, elution $CH_2Cl_2$:$CH_3CN$ 92:8) gave 1.08 g (58%) of the title carbapenem as a solid. Recrystallization from EtOAc

gave white prisms:
mp 134-135° C; $[\alpha]_D^{22}$ − 14° (c 1.0, $CHCl_3$);
uv (EtOH)$\lambda_{max}$: 320 nm (12,250);
ir (KBr)$\nu_{max}$: 2080 and 2118 ($N_3$), 1780 (C=O of β-lactam) and 1692 $cm^{-1}$ (C=O of ester);
$^1$Hmr (200 MHz, $CDCl_3$) δ: 1.28 (d, J=7.28 Hz, 3H, $CH_3$-4), 1.45 (d, J=6.54 Hz, 3H, $CH_3$CHN), 3.20 (dd, $J_{H6,H5,}$ = 2.54 Hz, $J_{H6,H1}$ = 8.57 Hz, 1H, H-6), 3.74 (dq, $J_{H4,CH3}$ = 7.28 Hz, $J_{H4,H5}$ = 9.10 Hz, 1H, H-4), 3.90 (m, 1H, $CH_3$CHN), 4.03 (dd, overlapping with $CH_3$ of triazole, $J_{H5,H6}$ = 2.54 Hz, 1H, H-5), 4.08 (s, 3H, $CH_3$-1 of triazole), 4.11 (ABq, $J_{AB}$ = 14.92 Hz,Δr = 64.3 Hz, 2H, $SCH_2$-3), 4.73 (m, 2H, $CH_2$ of allyl), 5.2-5.5 and 5.9-6.1 (m, 3H, CH of allyl) and 7.48 ppm (s, 1H, H-5 of triazole).

| | | | | |
|---|---|---|---|---|
| Anal. calcd. for $C_{17}H_{21}N_7O_3S$: | C 50.61, | H 5.25, | N 24.30, | S 7.95: |
| found: | C 50.21, | H 5.23, | N 24.19, | S 8.11 |

Procedure 15

(5S,6R) 6-(1′R-Aminoethyl)-4R-methyl-3-(1-methyl-1,2,3-triazol-4-yl)methylthio]-7-oxo-1--azabicyclo[3.2.0]-hept-2-ene-2-carboxylic Acid (41178)

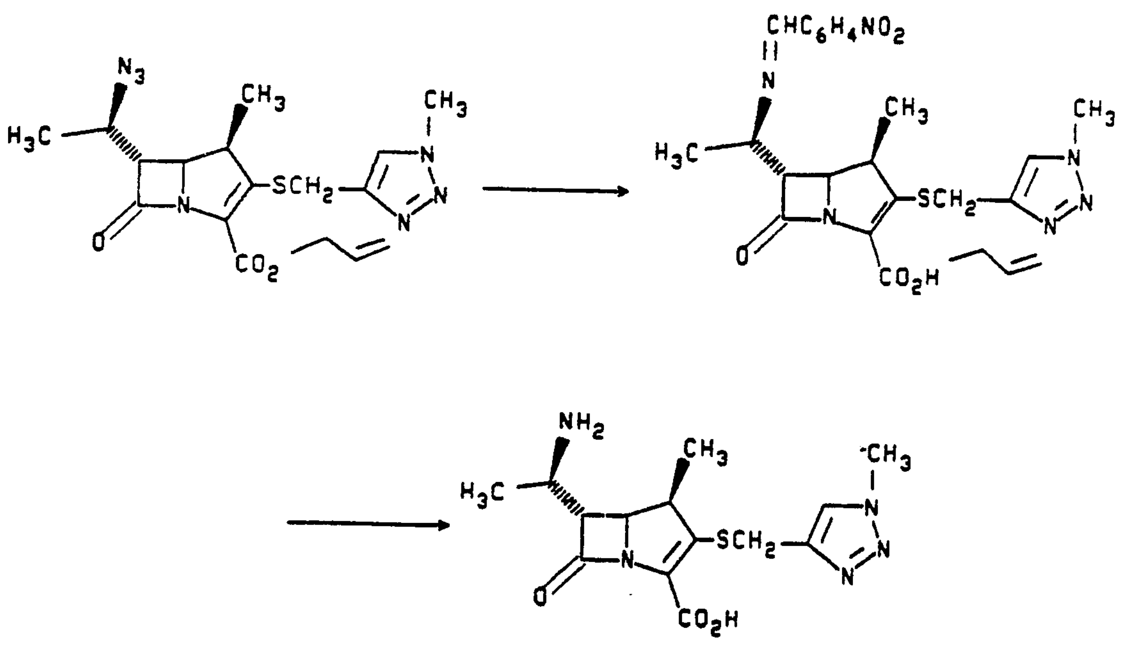

Allyl 6-(1′-R-azidoethyl)-4R-methyl-3-[(1-methyl-1,2,3-triazol-4-yl)methylthio]-7-oxo-1-azabicyclo[3.2.0]-hept-2-ene-2-carboxylate from Procedure 14 was converted with triphenylphosphine into the intermediate phosphinimine as described in Procedure 9B, and a portion thereof recovered as an oil:
ir (NaCl)$\nu_{max}$: 1765 (C=O of β-lactam) and 1700 $CM^{-1}$ (C=O of ester);
$^1$Hmr (200 MHz, $CDCl_3$) δ: 1.25 (d, J=6.03 Hz, 3H, $CH_3$ CHN), 1.30 (d, J=7.28 Hz, 3H, $CH_3$-4), 3.6 (m, 3H, H-1′, H-4 and H-6 overlapping), 3.90 (broad d, 1H, H-5), 4.07 (s, 3H, $CH_3$-1 of triazole), 4.11 (ABq, $J_{AB}$ = 14.95 Hz, Δν= 58.66 Hz, 2H, $SCH_2$-3), 4.74 (m, 2H $CH_2$ of allyl), 5.2-5.5 and 5.9-6.1 (m, 3H, CH of allyl) and 7.2-7.8 ppm (m, aromatics). The phosphinimine in benzene solution was treated with nitro-benzaldehyde as described in procedure 9B to yield the Schiff base:
ir (NaCl, film)$\nu_{max}$: 1770 (C=O of $\beta$-lactam) and 1705 $cm^{-1}$ (C=O of ester);
$^{1}$Hmr (200 MHz, $CDCl_3$ with $OP(Ph)_3$) $\delta$: 1.30 (d, J=7.28 Hz, 3H, $CH_3$-4), 1.41 (d, J=6.38 Hz, 3H, $CH_3$CHN), 3.53 (dd, $J_{H6,H5}$= 2.63 Hz, $J_{H6,H1'}$=7.73 Hz, 1H, H-6), 3.69 (dq, $J_{H4,CH3}$= 7.28 Hz, $J_{H4,H5}$= 9.30 Hz,1H, H-4), 3.85 (m overlapping with $SCH_2$, 1H, $CH_3$CHN), 4.05 (dd overlapping with $CH_3$ of triazole, $J_{H5,H6}$= 2.63 Hz, $J_{H5,H4}$= 9.30 Hz, 1H, H5) 4.06 (s, 3H, $CH_3$-1 of triazole), 4.07 (ABq, $J_{AB}$= 14.90 Hz, $\Delta\nu$ = 67.8 Hz, 2H, $SCH_2$-3), 4.72 (m, 2H, $CH_2$ of allyl), 5.2-5.5 and 5.8-6.1 (m, 3H, CH of allyl), 7.90 (d, J=8.81 Hz, 2H, H ortho of p-nitrobenzyl), 8.25 (d, J=8.81 Hz, 2H, H meta of p-nitrobenzyl) and 8.40 ppm (s, 1H, CH=N). The crude Schiff base was disolved in dry $CH_2Cl_2$ and further treated as described in Procedure 9B with tetrakis (triphenylphosphine) palladium (0) and potassium 2-ethylhexonate. Chromatography of the aqueous product on reversed phase silicagel ($\mu$-Bondapak c-18, 3 x 14 cm), and elution with a mixture of water and acetonitrile (95:5) gave 0.429 g (52%) of the title carbapenem as a white amorphous powder after lyophilization: $[\alpha]^{24}$ + 8° (c 1.0, $H_2O$); hplc on $\mu$-Bondapak c-18, 4mm x 30 cm, elution 8% $CH_3CN$-$H_2O$ pH 7.4 phosphate buffer, flow rate 1 mL/min, uv detector 300 nm, retention time 4.9 min, purity 99%;
uv ($H_2O$, pH 7.4 phosphate buffer) $\lambda_{max}$: 302 nm (8971);
ir (KBr)$\nu_{max}$: 1755 (C=O of $\beta$-lactam) and 1690 $cm^{-1}$ (C=O of ester);
$^{1}$Hmr (200 MHz, $D_2O$) $\delta$: 1.18 (d,, J=7.24 Hz, 3H, $CH_3$-4), 1.47 (d, J=6.62 Hz, 3H, $CH_3$CHN), 3.46 (m, 1H, H-4), 3.60 (dd, $J_{H6,H5}$= 2.45 Hz, $J_{H6,H1'}$= 8.29 Hz, 1H, H-6), 3.85 (dq, $J_{H1'}$, $CH_3$=6.62 Hz, $J_{H1'}$,H6=829 Hz, 1H, $CH_3$CHN), 4.07 (s,3H, $CH_3$-1 of triazole), 4.22 (dd, $J_{H5,H4}$= 8.99 Hz, 1H, H-5), 4.11 (ABq, $J_{AB}$= 14.84 Hz, $\Delta\nu$ = 37.0 Hz, 2H, $SCH_2$-3) and 7.87 ppm (s, 1H, H-5 of triazole).

By uv, at 37° C in a pH 7.4 phosphate buffer, the half-life of this carbapenem was 3.1 h. By hplc at 37° C in a pH 2.0 citric acid buffer, the half-life was evaluated to be 144 min.

| M.I.C. | | |
|---|---|---|
| Str. pneu. A9585 | 1.000 | |
| Str. pyo. A9604 | 1.000 | |
| Str. faec. A20688 | 125.000 | |
| Staph. aur. A9537 | 4.000 | |
| Staph. serum | 4.000 | |
| Staph aur. Pen+ | 4.000 | |
| Staph aur. MR | 32.000 | |
| E. coli A15119 | 0.030 | |
| E. coli A20341 | 0.030 | |
| K. pneu. A9664 | 0.130 | |
| K. pneu. A20468 | 0.130 | |
| Ent. clo. A9659 | 0.130 | |
| Ent. clo. A9656 | 0.030 | |
| P. mir. A9900 | 0.030 | |
| P. vul. A21559 | 0.060 | |
| P. morg. A15153 | 0.130 | |
| P. rett. A22424 | 4.000 | |
| Ser. mars. A20019 | 0.130 | |
| Ps. aeru. A9843 | 2.000 | |
| Ps. aeru. CarbR | 16.000 | |
| PD50 | IM | PO |
| E. coli | | 21.80 |
| t1/2, pH 7.4 (h.): | | 3.1 |
| t1/2, pH 2 (min.): | | 144 |
| Cmax, PO/50: | | 29 |
| t1/2: | | 24 |
| Auc; | | 25 |
| U.R. | | 11 |

Procedure 16

Allyl (5R,6S)-6-(1'S-Hydroxyethyl)-4R-methyl-3-[(pyridin-2-yl)methylthio]-7-oxo-1-azabicyclo[3.2.0]hept-2-ene-2-carboxylate

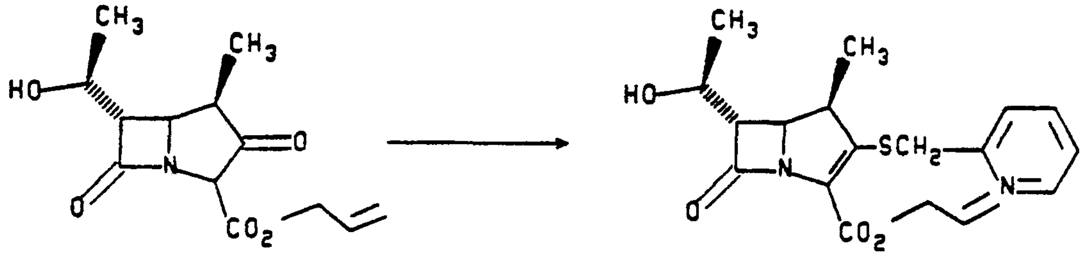

A solution of allyl (5S,6S)-6-(1'S-hydroxyethyl)-4R-methyl-3,7-dioxo-1-azabicyclo[3.2.0]heptane-2-carboxylate from Procedure 1 (11.0 mmol) was treated as described in Procedure 13, but substituting 2-mercaptomethylpyridine for the thiomethyltriazole specified there. The crude product was recovered as an oil which was chromatogrpahed on silica gel (9 x 12 cm) to give 1.87 g 45%) of the title carbapenem as a foam:

ir (NaCl, film)$\nu_{max}$: 1765 (C=O of $\beta$-lactam) and 1700 $cm^{-1}$ (C=O of ester);

'Hmr (200 MHz, $CDCl_3$) $\delta$: 1.23 (d, J=7.26 Hz, 3H, $CH_3$-4), 1.23 (d, J=7.26 Hz, 3H, $CH_3$-4), 1.35 (d, J=6.33 Hz, 3H, $CH_3CHO$), 1.95 (d, J=4.51 Hz, 1H, OH), 3.29 (dd, $J_{H6,H5}$=2.58 Hz, $J_{H6,H1'}$= 6.02 Hz, 1H, H-6), 3.66 (dq, $J_{H4,CH3}$= 7.26 Hz, $J_{H4,H5Y}$= 9.07 Hz, 1H, H-4), 4.00 (dd, JH5,H6 = 2.58 Hz, $J_{H5,H4}$= 9.07 Hz, 1H, H-5), 4.13 (m, 1H, $CH_3CHO$), 4.24 (ABq), $J_{AB}$= 14.17 Hz, $\Delta\nu$= 39.7 Hz, 2H,$SCH_2$-3), 4.74 (m, 2H, $CH_2$ of allyl), 5.2-5.5 and 5.9-6.1 (m, 3H, CH of allyl), 7.19 (m, 1H, H-5 of pyridine), 7.40 (d,J=7.86 Hz, H-3 of pyridine), 7.67 (m, 1H, H-4 of pyridine) and 8.50 ppm (m, 1H, H-6 of pyridine).

Procedure 17

(5S,6S) Allyl 6-(1'R-azidoethyl)-4R-methyl-3-[(pyridin-2-yl)methylthio]-7-oxo-1-azabicyclo[3.2.0]hept-2-ene-2-carboxylate

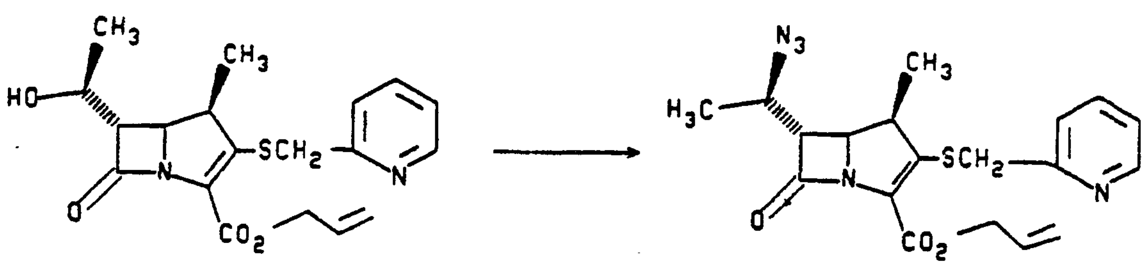

Allyl 6-(1'S-hydroxyethyl)-4R-methyl-3-[pyridin-2-yl)methylthio]-7-oxo-1-azabicyclo[3.2.0]hept-2-ene-2-carboxylate (1.61 g, 4.29 mmol) was treated with triphenylphosphine, hydrazoic acid, and diethylazodicarboxylate as described in Procedure 8. Chromatography on silica gel (3.5 x 11 cm), and elution with a mixture of $CH_2Cl_2$ and $CH_3CN$ (9:1) gave 0.90 g (53%) of the title carbapenem as an oil:

ir (NaCl, film)$\nu_{max}$: 2118 ($N_3$), 1770 (C=O of $\beta$-lactam), and 1710 $cm^{-1}$ (C=O of ester);

'Hmr (200 MHz, $CDCl_3$) $\delta$: 1.23 (d, J=7.0 Hz, 3H, $CH_3$-4), 1.45 (d, J=6.53 Hz, 3H, $CH_3CHN$), 3.18 (dd, $J_{H6,H5}$= 2.50 Hz, $J_{H6,H1'}$= 8.88 Hz, 1H, H-6), 3.71 (dq,, $J_{H4,CH3}$= 7.0 Hz, $J_{H4,H5}$= 9.08 Hz, 1H, H-4), 3.87 (dq, $J_{H1'}$,CH3= 6.53 Hz, $J_{H1'}$,H6= 8.88. 1H, $CH_3CHN$), 4.01 (dd, $J_{H5,H6}$= 2.50 Hz, $J_{H5,H4}$= 9.08 Hz,1H, H-5), 4.14 (ABq, $J_{AB}$= 14.14 Hz, $\Delta\nu$= 38.3 Hz, 2H, $SCH_2$-3), 4.73 (m, 2H, $CH_2$ of allyl), 5.2-5.5 and 5.9-6.1

(m, 3H, CH of allyl), 7.20 (m, 1H, H-5 of pyridine), 7.40 (broad d, J = 7.8 Hz, H-3 of pyridine), 7.68 (m, 1H, H-4 of pyridine), and 8.51 ppm (broad d, J = 4.5 Hz, 1H, H-6 of pyridine).

## Procedure 18

(5S,6R) 6-(1′R-Aminoethyl)-4R-methyl-3-[(pyridin-2-yl)methylthio]-7-oxo-1-azabicyclo[3.2.0]hept-2-ene-2-carboxylic Acid (41212)

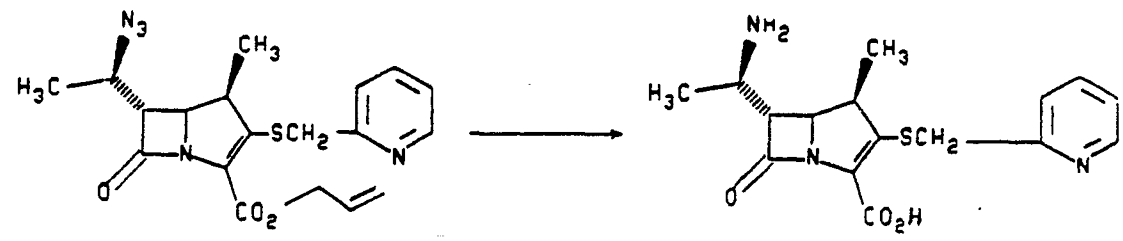

The method of Procedure 9B was employed. A solution of (5S,6S) allyl 6-(1′R-azidoethyl)-4R-methyl-3-[(pyridin-2-yl)methylthio]-7-oxo-1-azabicyclo[3.2.0]hept-2-ene-2-carboxylate (1.08 g, 2.70 mmol) and triphenylphosphine (0.85 g, 3.24 mmol) in dry benzene (25 mL) was heated under reflux for 20 minutes. The reaction mixture was then cooled to 22 °C and the crude phosphinimine was treated with p-nitrobenzaldehyde (0.45 g, 2.97 mmol). After 21 h at 22 °C, the solvent was evaporated to give the crude Schiff base as an oil which was used as such for the next step:
ir (NaCl, film)$\nu_{max}$: 1770 (C=O of $\beta$-lactam) and 1707 $cm^{-1}$ (C=O of ester).

A solution of the crude Schiff base (2.70 mmol) in dry $CH_2Cl_2$ (40 mL) was treated at 22 °C and under $N_2$ with tetrakis(triphenylphosphine)palladium(0) (0.10 g) and 6.0 mL (3.0 mmol) of a 0.5M solution of potassium 2-ethylhexanoate in EtOAc. After 20 minutes, 40 ml of a 0.2M pH 6.0 aqueous phosphate buffer was added and the esulting mixture was stirred vigorously for 30 minutes. The aqueous phase was collected and the organic phase was extracted with water (3 x 20 mL). The combined aqeuous extracts were concentrated to ~40 mL in vacuo (T, 10 °C) and the residue was chromatographed on reveresed phase silica gel ($\mu$-Bondapak c-18, 3 x 12 cm). Elution with a mixture of water was acetonitrile (9:1) gave 0.260 g (29%) of the title carbapenem as a white amorphous solid after freeze drying: $[\alpha]_D^{22}$ + 21 ° (c, 1.0, $H_2O$); hplc on $\mu$-Bondapak c-18, 4 mm x 30 cm, 8% $CH_3CN$-$H_2O$ pH 7.4 phosphate buffer, flow rate 1 mL/min, uv detector at 304 n, retention time 9.8 min, purity 99%;
uv ($H_2O$, pH 7.4 phosphate buffer) $\lambda_{max}$: 266 (6,600) and 304 nm (9,760);
ir (KBr)$\nu_{max}$: 1755 (C=O of $\beta$-lactam) and 1590 $cm^{-1}$ (C=O of carboxylate);
′Hmr (200 MHz, D2O) $\delta$: 1.13 (d, J = 7.26 Hz, 3H, $CH_3$-4), 1.44 (d, J = 6.62 Hz, 3H, $CH_3CHN$), 3.40 (dq, $J_{H4,CH3}$ = 7.26 Hz, $J_{H4,H5}$ = 8.86 Hz, 1H, H-4), 3.56 (dd, $J_{H6,H5}$ = 2.46 Hz, $J_{H6,H1'}$ = 8.25 Hz, 1H, H-6), 3.80 (dq, $J_{H1',CH3}$ = 6.62 Hz, $J_{H1',H6}$ = 8.25 Hz, 1H, $CH_3CHN$), 4.06 (dd, $J_{H5,H6}$ = 2.46 Hz, $J_{H5,H4}$ = 8.86 Hz, 1H, H-5), 4.17 (ABq, $J_{AB}$ = 14.09 Hz, $\Delta\nu$ = 20.3 Hz, 2H, $SCH_2$-3), 7.36 (m, 1H, H-5 of pyridine), 7.53 (broad d, J = 7.5 Hz, 1H, H-3 of pyridine), 7.85 (m, 1H, H-4 of pyridine) and 8.45 ppm (m, 1H, H-6 of pyridine.

By uv at 37 °C, in a pH 7.4 phosphate buffer, this carbapenem was found to have a half-life of 2.8 h. By hplc at 37 °C in a pH 2.0 citric acid buffer the half-life was evaluated to be 120 minutes.

| M.I.C. | | |
|---|---|---|
| Str. pneu. A9585 | 0.500 | |
| Str. Pyo. A9604 | 0.500 | |
| Str. faec. A20688 | 125.000 | |
| Staph. aur. A9537 | 2.000 | |
| Staph. serum | 4.000 | |
| Staph. aur. Pen + | 4.000 | |
| Staph. aur. MR | 32.000 | |
| E. coli A15119 | 0.030 | |
| E. coli A20341 | 0.016 | |
| K. pneu. A9664 | 0.130 | |
| K. pneu. A20468 | 0.130 | |
| Ent. clo. A9659 | 0.130 | |
| Ent. clo. A9656 | 0.030 | |
| P. mir. A9900 | 0.016 | |
| P. vul. A21559 | 0.016 | |
| P. morg. A15153 | 0.030 | |
| P. rett. A22424 | 2.000 | |
| Ser. mars. A20019 | 0.060 | |
| Ps. aeru. A9843 | 2.000 | |
| Ps. aeru. CarbR | 16.000 | |
| PD50, | IM | PO |
| E. coli | | 11.20 |
| S. pneu | | 16.5 |
| Cmax, IM/20: | | 24 |
| t1/2: | | 15 |
| AUC: | | 11 |
| Cmax, PO/50: | | 33 |
| t1/2: | | 24 |
| AUC; | | 32 |
| U.R.: | | 17 |

Procedure 19

Allyl(5S,6S)-6-(1′S-Hydroxyethyl)-4R-methyl-3-[(pyridin-3-yl)methylthio]-7-oxo-1-azabicyclo[3.2.0]hept-2-ene-2-carboxylate

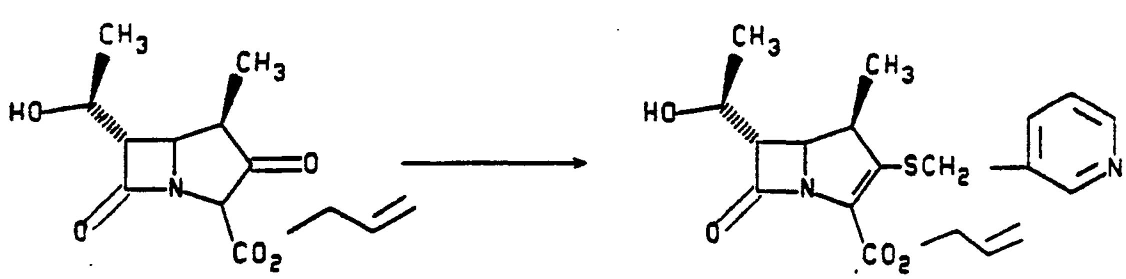

A solution of allyl(5S,6S)-6-(1′S-hydroxyethyl)-4R-methyl-3,7-dioxo-1-azabicyclo[3.2.0]heptane-2-carboxylate from Procedure 2 (11.0 mmol) was treated as described in Procedure 13, but substituting 3-mercaptomethylpyridine (2.05 g, 16.4 mmol) for the thiomethyltriazole specified there. The crude product was recovered as an oil which was chromatographed on silica gel (250 g). Elution with EtOAc and then $CH_3CN$ gave 2.58 g (62%) of the title carbapenem as foam:
ir (NaCl, film)$\nu_{max}$: 1760 (C = O of $\beta$-lactam) and 1700 $cm^{-1}$ (C = O of ester);
′Hmr (200 MHz, $CDCl_3$) $\delta$: 1.25 (d, J = 7.27 Hz, 3H, $CH_3$-4), 1.35 (d, J-6.35 Hz, 3H, $CH_3CHN$), 3.31 (dd, $J_{H6,H5}$ = 2.55 Hz, $J_{H6,H1'}$ = 5.89 Hz, 1H, H-6), 3.31 (m overlapping with H-6, 1H, H-4), 4.02 (dd overlapping with $SCH_2$, $J_{H5,H6}$ = 2.55 Hz, 1H, H-5), 4.05 (ABq, $J_{AB}$ = 13.3 Hz, $\Delta\nu$ = 11.7 Hz, 2H, $SCH_2$-3), 4.16 (m, 1H, $CH_3CHN$) 4.75 (m, 2H, $CH_2$ of allyl), 5.2-5.5 and 5.9-6.1 (m, 3H, CH of allyl), 7.28 (dd, $J_{H5,H6}$ = 4.79 Hz, 1H, H-5 of pyridine), 7.70 (broad, $J_{H4,H5}$ = 7.9 Hz, 1H, H-4 of pyridine), 8.53 (broad d, $J_{H6,H5}$ = 4.79 Hz, 1H, H-6 of pyridine) and 8.56 ppm (~d, J = 2.0 Hz, 1H, H-2 of pyridine).

## Procedure 20

Allyl(5S,6S)-6-(1′R-Azidoethyl)-4R-methyl-3-[(pyridin-3-yl methylthio]-7-oxo-1-azabicyclo[3.2.0]hept-2-ene-2-carboxylate

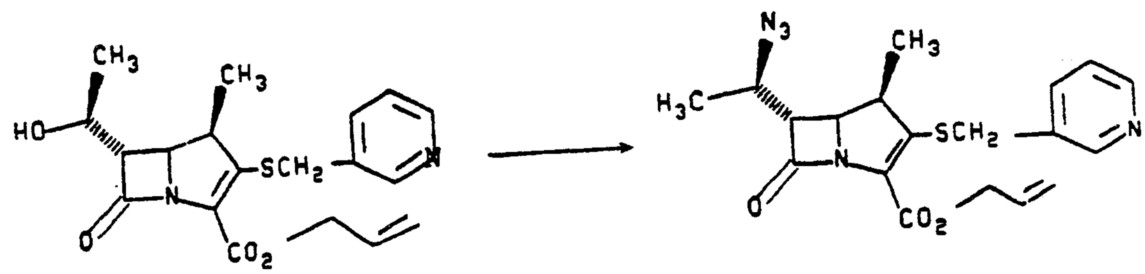

Allyl 6-(1′S-hydroxyethyl)-4R-methyl-3-[(pyridin-3-yl)methylthio]-7-oxo-1-azabicyclo [3.2.0]hept-2-ene-2-carboxylate (1.99 g, 5.31 mmol) was treated with triphenylphospine, hydrazoic acid, and diethyl azodicarboxylate as described in Procedure 8. Chromatography twice on silica gel (3 x 14 cm, first column $CH_2Cl_2$: $CH_3CN$ 7:3, second column toluene-AcOEt 1:1 as eluent) to give 0.62 g (30%) of the title carbapenem as an oil. Crystallization from EtOAc-hexanes gave white prisms:
mp 65-66 °C; $[\alpha]_D^{24}$ + 26° (C 1.0, $CHCl_3$);
uv (EtOH)$\lambda_{max}$: 264 (5,370) and 320 nm (13,960);
ir (KBr)$\nu_{max}$: 2120 and 2080 ($N_3$), 1786 (C = O of $\beta$-lactam) and 1700 $cm^{-1}$ (C = O of ester);
′Hmr (200 MHz, $CDCl_3$) $\delta$: 1.26 (d,J = 7.30 Hz,, 3H, $CH_3$-4), 1.46 (d, J = 6.52 Hz, 3H, $CH_3CHN$), 3.19 (dd,, $J_{H6,H5}$ = 2.58 Hz, $J_{H6,H1'}$ = 9.04 Hz, 1H, H-6), 3.34 (dq, $J_{H4,CH3}$ = 7.30 Hz, $J_{H4,H5}$ = 9.16 Hz, 1H, H-4), 3.89 (dq, $J_{H1'}$,CH3 = 6.52 Hz, $J_{H1'}$,H6 = 9.04 Hz, 1H, $CH_3CHN$), 4.03 (dd overlapping with $SCH_2$-3, $J_{H5,H6}$ = 2.58 Hz, 1H, H-5), 4.06 (ABq, $J_{AB}$ = 13.46 Hz, $\Delta\nu$ = 9.3 Hz, 2H, $SCH_2$-3), 4.75 (m, 2H, $CH_2$ of allyl), 5.2-5.5 and 5.9-6.1 (m, 3H, CH of allyl), 7.28 (dd, $J_{H5,H4}$ = 7.81 Hz, $J_{H5,H6}$ = 4.9 Hz, H-5 of pyridine), 7.69 (broad d, $J_{H4,H5}$ = 7.81 Hz, 1H, H-4 of pyridine), 8.54 (dd, $J_{H6,H5}$ = 4.9 Hz, $J_{H6,H4}$ = 1.37 Hz, 1H, H-6 of pyridine) and 8.57 ppm (d, J = 1.9 Hz, 1H, H-2 of pyridine).

| | | | | |
|---|---|---|---|---|
| Anal. calcd. for $C_{19}H_{21}N_5O_3S$: | C 57.13, | H 5.30, | N 17.53, | S 8.03; |
| found: | C 57.14, | H 5.34, | N 17.59, | S 8.15 |

## Procedure 21

(5S,6R)-6-(1′R-Aminoethyl)-4R-methyl-3-[(pyridin-3-yl)methyl-thio]-7-oxo-1-azabicyclo[3,2,0]hept-2-ene-2-carboxylic Acid (41214)

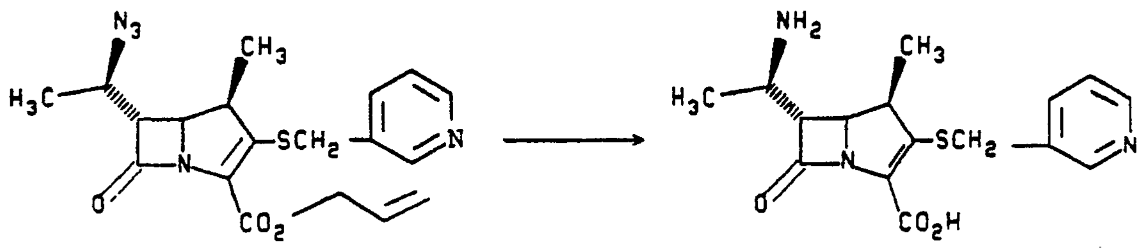

A solution of allyl (5S,6S)-6-(1′R-azidoethyl)-4R-methyl-3-[(pyridin-3-yl)methylthio]-7-oxo-1-azabicyclo-[3.2.0]hept-2-ene-2-carboxylate (0.540 g, 1.35 mmol) was treated with triphenylphosphine (0.389 g, 1.48 mmol), and p-nitrobenzaldehyde (0.224 g, 1.48 mmol) according to Procedure 9B. The crude Schiff base was an oil which was used as such for the next step:

ir (NaCl, film)$\nu_{max}$: 1770 (C=O of $\beta$-lactam) and 1705 $cm^{-1}$ (C=O of ester);

′Hmr (200 MHz, $CDCl_3$, with $(Ph)_3PO$) $\delta$: 1.28 (d, J=7.27 Hz, 3H, $CH_3$-4), 1.42 (d, J=6.37 Hz, 3H, $CH_3CHN$), 3.27 (dq, $J_{H4,CH3}$= 7.27 Hz, $J_{H4,H5}$= 9.30 Hz, 1H, H-4), 3.53 (dd, $J_{H6,H5}$= 2.64 Hz, $J_{H6,H1'}$= 7.83 Hz, 1H, H-6), 3.87 (m, 1H, $CH_3CHN$), 4.02 (ABq, $J_{AB}$= 12.0 Hz, $\Delta\nu$= 9.0 Hz, 2H, $SCH_2$-3), 4.02 (overlapping with $SCH_2$, 1H, H-5), 4.74 (m, 2H, $CH_2$ of allyl), 5.2-55 and 5.9--6.1 (m, 3H, CH of allyl), 7.90 (d, J=8.78 Hz, 2H, H ortho of p-nitrobenzylidene), 8.22 (d, J=8.78 Hz, 2H, H meta of p-nitrobenzylidene), 8.41 (s, 1H, CH-N) and 8.5 ppm (m, 2H, H-2 and H-6 of pyridine.

A solution of the crude Schiff base (1.35 mmol) in dry $CH_2Cl_2$ (10 mL) was treated at 22°C and under $N_2$ with triphenylphosphine (0,050 g), tetrakis(triphenylphosphine) palladium (0) (0.050 g) and 3.4 mL (1.7 mmol) of a 0.5M solution of potassium 2-ethylhexanoate in ethyl acetate. After 45 min. 20 mL of 0.2M, pH 6.0 phosphate buffer were added and the product was recovered from the aqueous phase as in Procedure 9B using chromatography on reversed phase silica gel ($\mu$-Bondapak C-18, 3 x 12 cm). Elution with a mixture of water and acetonitrile (9:1) gave 0.182 g (40%) of the title carbapenem as a white amorphous solid after freeze drying: $[\alpha]^{22}$ + 9° (c 1.0, $H_2O$); hplc on $\mu$-Bondapak c-18, 30 cm x 4 mm, 8% $CH_3CN$-$H_2O$ pH 7.4 phosphate buffer, flow rate 1 mL/min, uv detector 300 nm, retention time 10.3 min, purity 99%;

uv ($H_2O$, pH 7.4 phosphate buffer)$\lambda_{max}$: 270 (5,600) and 304 nm (8,950);

ir (KBr)$\nu_{max}$: 1755 (C=O of $\beta$-lactam) and 1580 $cm^{-1}$ (broad, C=O of carboxylate);

′Hmr (200 Hz, $D_2O$) $\delta$: 1.16 (d, J=7.19 Hz, 3H, $CH_3$-4), 1.46 (d, J=6.61 Hz, 3H, $CH_3CHN$), 3.35 (m, 1H, H-4), 3.58 (dd, $J_{H6,H5}$- 2.06 Hz, $J_{H6,H1'}$= 8.19 Hz, 1H, H-6), 3.83 (m, 1H, $CH_3CHN$), 4.06 (dd overlapping with $SCH_2$, $J_{H5,H6}$= 2.06 Hz, 1H, H-5), 4.12 (ABq, $J_{AB}$= 14.12 Hz, $\Delta\nu$= 24.4 Hz, 2H, $SCH_2$-3), 7.44 (dd, $J_{H5,H4}$= 7.9 Hz, $J_{H5,H6}$= 4.9 Hz, 1H, H-5 of pyridine), 7.9 (d, $J_{H4,H5}$= 7.9 Hz, 1H, H-4 of pyridine), 8.42 (d, $J_{H6,H5}$= 4.9 Hz, 1H, H-6 of pyridine) and 8.52 ppm (broad s, 1H, H-2 of pyridine).

By uv at 37°C in a pH 7.4 phosphate buffer, the carbapenem has a half-life of 3 h. By hplc, at 37°C in a pH 2.0 citric acid buffer, the half-life was evaluated to be 144 min.

| M.I.C. | | |
|---|---|---|
| Str. pneu. A9585 | 0.500 | |
| Str. pyo. A9604 | 0.500 | |
| Str. faec. A20688 | 63.000 | |
| Staph. aur. A9537 | 1.000 | |
| Staph. serum | 4.000 | |
| Staph. aur. Pen+ | 2.000 | |
| Staph. aur. MR | 32.000 | |
| E. coli A15119 | 0.030 | |
| E. coli A20341 | 0.030 | |
| K. pneu. A9664 | 0.130 | |
| K. pneu. A20468 | 0.130 | |
| Ent. clo. A9659 | 0.130 | |
| Ent. clo. A9656 | 0.060 | |
| P. mir. A9900 | 0.008 | |
| P. vul. A21559 | 0.016 | |
| P. morg.A15153 | 0.030 | |
| P. rett.A22424 | 2.000 | |
| Ser. mars. A20019 | 0.060 | |
| Ps. aeru A9843 | 1.000 | |
| Ps. aeru. CarbR | 16.000 | |
| PD50, | IM | PO |
| E. coli | | 8.10 |
| t1/2, pH 7.4 (h.): | | 3 |
| t1/2, pH 2 (min.): | | 144 |
| DHP: | | |
| Cmax, IM/20: | | 18 |
| t1/2: | | 12 |
| AUC: | | 7 |
| Cmax, PO/50: | | 25 |
| t1/2: | | 23 |
| AUC; | | 21 |
| U. R.: | | 5 |

## Procedure 22

(5S,6S) Allyl 6-(1′R-Azidoethyl)-3-(2-fluoroethylthio)-4R-methyl-7-oxo-1-azabicyclo[3.2.0]hept-2-ene-2-carboxylate

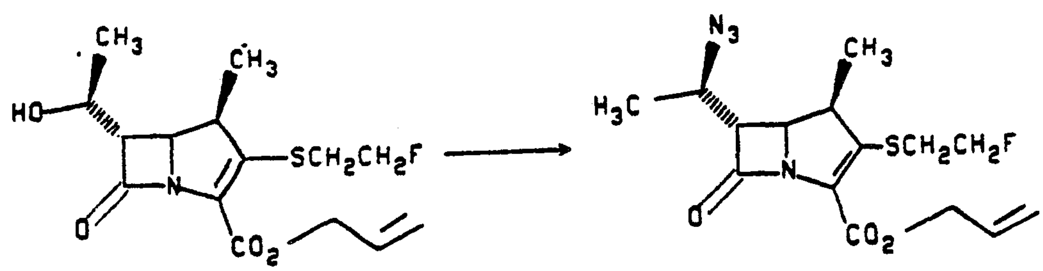

A solution of (5S,6S) allyl 3-(2-fluoroethylthio)-6-(1′S-hydroxyethyl)-4R-methyl 1-7-oxo-1-azabicyclo-[3.2.0]hept-2-ene-2-carboxylate (1.79 g., 5.43 mmol), prepared as shown in Procedure 3 but using 2-fluoroethanelthiol, in dry tetrahydrofuran (75 mL) was treated with triphenylphosphine, hydrazoic acid, and diethyl azodicarboxylate as shown in Procedure 8. Chromatography of the toluene solution on silica gel (5X12 cm) using a mixture of toluene and EtOAc (9:1) as eluent to give 1.10 g (57%) of the title carbapenem as an oil: ir (NaCl, film) $\nu_{max}$: 2120 ($N_3$), 1770 (C=O of $\beta$-lactam) and 1710 $cm^{-1}$ (C=O of ester); ′Hmr (200 MHz, $CDCl_3$) $\delta$: 1.26 (d, J=7.30 Hz, 3H, $CH_3$-4), 1.47 (d, J=6.54 Hz, 3H, $CH_3CHN$), 2.8-3.3 (m, 2H, $SCH_2$), 3.21 (dd, $J_{H6,H5}$=2.51 Hz, $J_{H6,H1'}$=9.11 Hz, 1H, H-6), 3.45 (dq, $J_{H4,CH3}$=7.30 Hz, $J_{H4,H5}$=9.12 Hz, 1H, H-4), 3.91 (dq, 3.91 (dq, $J_{H1'}$,CH3=6.54Hz, $J_{H1'}$,H6= 9.11 Hz, 1H, $CH_3CHN$, 4.13 (dd, $J_{H5,H6}$=2.51 Hz, $J_{H5,H4}$=9.12 Hz, 1H, H-5), 4.3-4.9 (m, 4H, $CH_2$ of allyl and $SCH_2CH_2F$), 5.2-5.5 and 5.9-6.1 ppm (m, 2H and 1H, CH of allyl).

## Procedure 23

(5S,6R)-6-1′Aminoethyl)-3-(2-fluoroethylthio)-4R-methyl-7-oxo-1-azabicyclo[3.2.0]hept-2-ene-2-carboxylic Acid (41416)

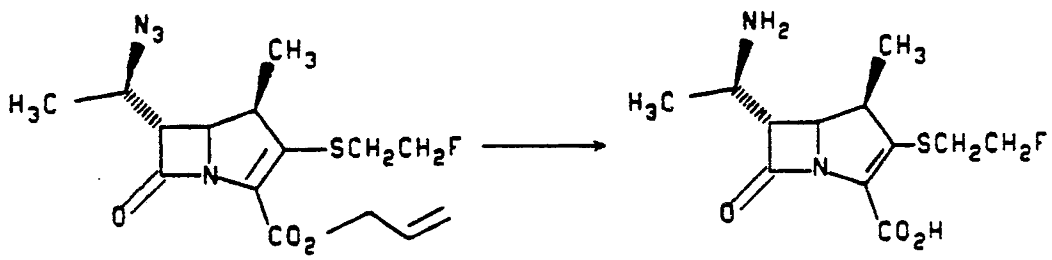

A solution of allyl (5S,6S)-6-(1′R-azidoethyl)-3- (2-fluoroethylthio)-4R-methyl-7-oxo-1-azabicyclo[3.2.0]-hept-2-ene-2-carboxylate (1.10 g, 3.1 mmol) was treated with triphenylphosphine (0.98 g, 3.73 mmol), and p-nitrobenzaldehyde (0.52 g, 3.44 mmol), as described in Procedure 9B to form the Schiff base which was used without purification for the next step:
′Hmr (200 MHz, $CDCl_2$, mixed with $Ph_3PO$) $\delta$: 1.28 (d, J=7.33 Hz, 3H, $CH_3$-4), 1.44 (d, J=6.39 Hz, 3H, $CH_3CHN$), 2.8-33 (m, 2H, $SCH_2$), 3.36 (m, 1H, H-4), 3.54 (dd, $J_{H6,H5}$,= 2.61 Hz, $J_{H6,H1}$=7.96 Hz, 1H, H-6), 3.89 (m, 1H, $CH_3CHN$), 4.08 (dd, $J_{H5,H6}$=2.61 Hz, $J_{H5,H4}$=9.34 Hz, 1H, H-5), 4.3-4.9 (m, 4H, $CH_2$ of allyl and $CH_2F$), 5.2-5.5 and 5.9-6.1(m, 2H and 1H, CH of allyl), 7.9 (d, J=9 HZ, 2H, H ortho or p-nitrobenzyl), 8.3 (d, J=9 Hz, H meta of p-nitrobenzyl) and 8.45 ppm (s, 1H, CH=N).

A solution of the crude Schiff base (3.10 mmol) in dry $CH_2Cl_2$ (40 mL) was treated with tetrakis (triphenylphosphine)palladium [0] (0.11 g), and potassium 2-ethylhexanoate as in Procedure 9B. Chromatography on reversed phase silica gel($\mu$-Bondapak $C_{18}$, 3x14 cm), and elution with water gave 0.36 g (40%) of the title material as a white amorphous powder after freeze drying: $[\alpha]^{22}$ + 96° (c 1.0, $H_2O$); hplc on $\mu$-Bondapak $C_{18}$, 4 mm x 30 cm, 5% $CH_3CN$-$H_2O$, pH 7.4 phosphate buffer, flow rate 1 mL/min, uv detector 304 nm, retention time 6.9 min, purity 99%;
uv ($H_2$), pH 7.4 phosphate buffer)$\lambda_{max}$: 300 nm (8,430);
ir (KBr)$\nu_{max}$:1760 (C=O of $\beta$-lactam) and 1585 $cm^{-1}$ (C=O of carboxylate);
′Hmr (200 MHz, $D_2O$) $\delta$: 1.22 (d, J=7.26 Hz, 3H, $CH_3$-4), 1.46 (d, J=6.61 Hz, 3H, $CH_3$-4), 2.9-3.4 (m, 2H, $SCH_2$), 3.5 (dq, $J_{H4,CH3}$=7.26 Hz, $J_{H4,H5}$=8.80 Hz, 1H, H-4), 3.60 (dd, $J_{H6,H5}$=2.38 Hz, $J_{H6,H1'}$=8.18 Hz, 1H, H-6), 3.83 (dq, $J_{H1'}$,CH3=6.61 Hz, $J_{H1'}$,H6=8.18 Hz, 1H, $CH_3CHN$), 4.20 (dd, $J_{H5,H6}$= 2.38 Hz, $J_{H5,H4}$=8.80 Hz, 1H, H-5), and 4.67 ppm (dm, $J_{H,F}$=47 Hz, 2H, $CH_2F$).

By uv, at 37° C in a pH 7.4 phosphate buffer, the half-life was 3.6 h. By hplc, at 37° C in a pH 2.0 citric acid buffer, the half-life was evaluated to be 155 min.

| M.I.C. | |
|---|---|
| Str. pneu. A9585 | 0.500 |
| Str. pyo. A9604 | 1.000 |
| Str. faec. A20688 | 125.000 |
| Staph. aur. A9537 | 2.000 |
| Staph. serum | 16.000 |
| Staph aur. Pen+ | 4.000 |
| Staph aur. MR | 125.000 |
| E. coli A15119 | 0.030 |
| E. coli A20341 | 0.030 |
| K. pneu. A9664 | 0.030 |
| K. pneu. A20468 | 0.130 |
| Ent. clo. A9659 | 0.130 |
| Ent. clo. A9656 | 0.030 |
| P. mir. A9900 | 0.016 |
| P. vul. A21559 | 0.030 |
| P. morg. A15153 | 0.016 |
| P. rett. A22424 | 4.000 |
| Ser. mars. A20019 | 0.030 |
| Ps. aeru. A9843 | 0.500 |
| Ps. aeru. CarbR | 16.000 |

| M.I.C. (cont.) | | |
|---|---|---|
| PD50, | IM | PO |
| E. coli | | 7.20 |
| S. pneu. | | >25 |

| | |
|---|---|
| t1/2, pH 7.4: | 3.6 |
| t1/2, pH 2: | 155 |
| Cmax, PO/50: | 24 |
| t1/2: | 17 |
| AUC; | 16 |

Procedure 24

(5S,6S)-Allyl 3-(2-Cyanoethylthio)-6-(1′S-hydroxyethyl)-4R-methyl-7-oxo-1-azabicyclo[3.2.0]hept-2-ene-2-carboxylate

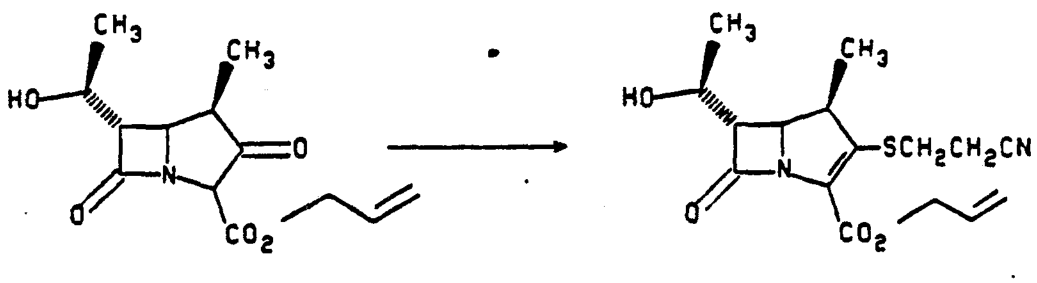

(5S,6S) Allyl 6-(1′S-hydroxyethyl)-4R-methyl-3,7-dioxo-1-azabicyclo[3,2,0]heptane-2-carboxylate from Procedure 2 (16.9 mmol)was converted with β-mercaptopropionitrile (2.95 g, 33.85 mmol) into the title compound by the process of Procedure 3, Method B. Chromatography on silica gel (9 x 12 cm), and elution with a mixture of toluene and EtOAc (1:1 to EtOAc) gave 3.37 g (59%) of the title carbapenem as an oil: ir (NaCl, film)$\nu_{max}$: 3500 (OH), 2260 (C=N), 1770 (C=O of β-lactam) and 1705 $cm^{-1}$ (C=O of ester); ′Hmr (200 MHz, $CDCl_3$) δ: 1.27 (d, J=7.31 Hz, 3H, $CH_3$-4), 1.36 (d, J=6.36 Hz, 3H, $CH_3CHOH$), 2.6-3.3 (m, 4H, $CH_2CH_2CN$), 3.35 (dd,$J_{H6,H5}$=2.6 Hz, $J_{H6,H1'}$=5.83 Hz, 1H, H-6), 3.4 (m, overlapping with H-6, 1H, H-4), 4.18 (dd, $J_{H5,H6}$= 2.6 Hz, $J_{H5,H4}$=9.21 Hz, 1H, H-5), 4.18 (m, overlapping with H-5, 1H, $CH_3CHOH$), 4.75 (m, 2H, $CH_2$ of allyl), 5.2-5.5 and 5.9-6.1 ppm (m, 3H, CH of allyl).

## Procedure 25

(5S,6S) Allyl 6-(1′R-Azidoethyl)-3-(2-cyanoethylthio)-4R-methyl-7-oxo-1-azabicyclo[3.2.0]hept-2-ene-2-carboxylate

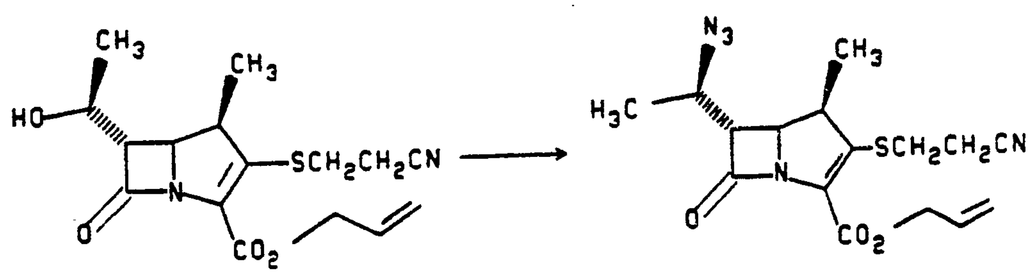

(5S,6S) Allyl 3-(2-cyanoethylthio)-6-(1′S-hydroxyethyl-4R-methyl-7-oxo-1-azabicyclo[3.2.0]hept-2-ene-2-carboxylate (3.0 g, 8.9 mmol) was treated with triphenylphosphine hydrazoic acid, and diethyl azodicarboxylate according to Procedure 8. Chromatography gave 1.24 g (38%) of the title carbapenem as an oil: ir (NaCl, film)$\nu_{max}$: 2250 (C=N), 2118 ($N_3$), 1775 (C=O of β-lactam) and 1708 $cm^{-1}$ (C=O of ester); ′Hmr (200 MHz, $CDCl_3$) δ: 1.28 (d, J=7.30 Hz, 3H, $CH_3$-4), 1.47 (d, J=6.51 Hz, 3H, $CH_3CHN$), 2.6-3.2 (m, 4H $SCH_2CH_2CN$), 3.23 (dd, $J_{H6,H5}$=2.58 Hz, $J_{H6,H1'}$=8.92 Hz, 1H, H-6), 3.43 (dq, $J_{H4,CH3}$=7.30 Hz, $J_{H4,H5}$=9.23 Hz, 1H, H-4), 3.92 (dq, $J_{H1'}$,CH3=6.51 Hz, $J_{H1'}$,H6=8.92 Hz, 1H, $CH_3CHN_3$), 4.18 (dd, $J_{H5,H6}$=2.58 Hz, $J_{H5,H4}$=9.23 Hz, 1H, H-5), 4.76 (m, 2H, $CH_2$ of allyl), 5.2-5.5 and 5.9-6.1 ppm (2m, 2H and 1H, CH of allyl).

## Procedure 26

(5S,6R)-6-(1′R-Aminoethyl)-3-(2-cyanoethylthio)-4R-methyl-7-oxo-1-azabicyclo[3.2.0]hept-2-ene-2-carboxylic Acid (41646)

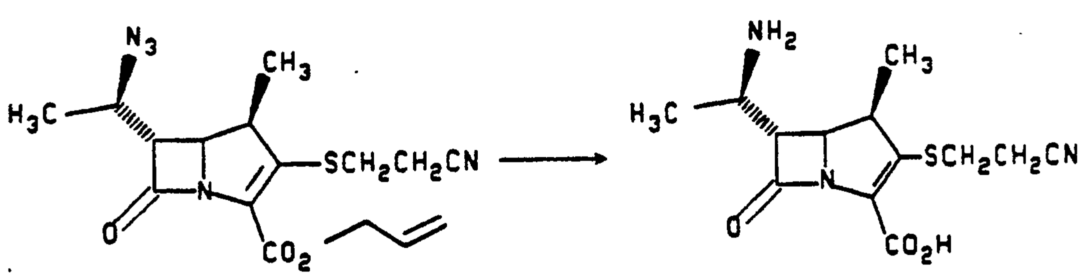

A solution of (5S,6S) allyl 6-(1′R-azidoethyl)-3-(2-cyanoethylthio)-4R-methyl-7-oxo-1-azabicyclo[3.2.0]-hept-2-ene-2-carboxylate (1.24 g, 3.43 mmol), was treated with triphenylphosphine. The resulting phosphinimine was then treated with p-nitrobenzaldehyde according to Procedure 9B to give the crude Schiff base as an oil which was used without purification for the next step:
ir (NaCl, film)$\nu_{max}$: 1775 (C=O of $\beta$-lactam) and 1708 $cm^{-1}$ (C=O of ester);
′Hmr (200 Hz, $CDCl_3$, mixture with $Ph_3PO$) $\delta$: 1.29 (d, J=7.28 Hz, 3H, $CH_3$-4), 1.44 (d, J=6.39 Hz, 3H, $CH_3CHN$), 2.6-3.3 (m, 4H, $SCH_2CH_2CN$), 3.37 (m, 1H, H-4), 3.57 (dd, $J_{H6,H5}$=2.69 Hz, $J_{H6,1'}$=7.90 Hz, 1H, H-6), 3.9 (m, 1H, $CH_3CHN$, 4.15 (~dd, 1H, H-5), 4.74 (m, 2H, $CH_2$ of allyl), 5.2-5.5 and 5.8-6.1 (m, 3H, CH of allyl), 7.91 (d, J=8.83 Hz, 2H, H ortho of benzyl) and 8.27 ppm (d, J-8.83 Hz, 2H, H meta of benzyl).

A solution of (5S,6S) allyl 3-(2-cyanoethylthio)-4R-methyl-6-(1′R-p-nitrobenzylideneaminoethyl)-7-oxo-1-aza bicyclo[3.2.0]hept-2-ene-2-carboxylate (1.60 g, 3.43 mmol), the crude Schiff base produced in the preceding paragraph,in dry $CH_2Cl_2$ (75 mL) was treated with tetrakis (triphenylphosphine) palladium and potassium 2-ethylhexanoate as described in Procedure 9B. Chromatography on reversed phase silaca gel ($\mu$-Bondapak $C_{18}$, 3.5 x 10 cm, elution with water) gave 0.32 g (32%) of the title carbapenem as a white amorphous powder after lyophilization: $[\alpha]^{22}$ + 89° (c 1.0, $H_2O$); hplc on $\mu$-Bondapak $C_{18}$, 4mm x 30 cm, elution 5% $CH_3CN$:$H_2O$ pH 7.4 phospharte buffer, flow rate 1 mL/min, uv detector 305 nm, retention time 5 min, purity 99%;
uv ($H_2O$, pH 7.4 phosphate buffer) $\lambda_{max}$: 300 nm (7,860);
ir(KBr)$\nu_{max}$:1755 (C=O of $\beta$-lactam) and 1590 $cm^{-1}$ (C=O of carboxylate);
′Hmr (200 MHz, $D_2O$) $\delta$: 1.23 (d, J=7.26 Hz, 3H, $CH_3$-4), 1.45 (d, J=6.61 Hz, 3H, $CH_3CHN$), 2.8-3.3 (m, 4H, $SCH_2CH_2CN$), 3.50 (m, 1H, H-4), 3.61 dd, $J_{H6,H5}$=1.46 Hz, $J_{H6,H1'}$=8.20 Hz, 1H,H-6), 3.81 (dq, $J_{H1',CH3}$=6.61Hz, $J_{H1',H6}$=8.20 Hz, 1H, H-1′) and 4.24 ppm (dd, $J_{H5,H6}$=2.46 Hz, $J_{H5,H4}$=9.0 Hz, 1H, H-5).

By uv at 37°C in a pH 7.4 phosphate buffer, the half-life was 2.8 h. At 37°C, in a pH 2.0 citric acid buffer, the half-life was evaluated to be 176 min.

| M.I.C. | |
|---|---|
| Str. pneu. A9585 | 1.000 |
| Str. pyo. A9604 | 1.000 |
| Str. faec. A20688 | 125.000 |
| Staph. aur. A9537 | 2.000 |
| Staph. serum | 63.000 |
| Staph aur. Pen+ | 125.000 |
| Staph aur. MR | 125.000 |
| E. coli A15119 | 0.008 |
| E. coli A20341 | 0.016 |
| K. pneu. A9664 | 0.030 |
| K. pneu. A20468 | 0.060 |
| Ent. clo. A9659 | 0.060 |
| Ent. clo. A9656 | 0.016 |
| P. mir. A9900 | 0.008 |
| P. vul. A21559 | 0.016 |
| P. morg. A15153 | 0.016 |
| P. rett. A22424 | 2.000 |
| Ser. mars. A20019 | 0.016 |
| Ps. aeru. A9843 | 0.250 |
| Ps. aeru. CarbR | 16.000 |
| t1/2, pH 7.4 (h.): | 2.8 |
| t1/2, pH 2 (min.): | 176 |

## Procedure 27

**3S-(1R-Azidoethyl)-4R-(1R-methyl-3-allyloxycarbonyl-2-oxo-3-diazopropyl)azetidin-2-one**

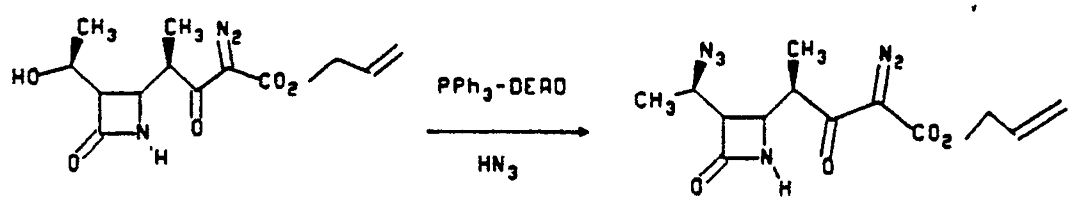


A solution of 3S-(1S-hydroxyethyl)-4R-(1R-methyl-3-allyloxycarbonyll-2-oxo-3-diazopropyl)azetidine-2-one (5.00 g, 16.9 mmol, Procedure 1) in a mixture of tetrahydrofuran (50 mL) and toluene (50 mL) was cooled to -15°C and treated with triphenylphosphine (7.10 g, 27.1 mmol) and 51 mL (27.1 mmol) of a 0.53 solution of hydrazoic acid in toluene. Then 4.71 g (27.1 mmol) of diethyl azodicarboxylate were added dropwise over 5 min and the resulitng mixture was stirred for 30 min. The reaction was then quenced by addition of water and EtOAc (400 mL). The organic phase was washed with brine, dried over anhydrous $MgSO_4$ and concentrated under vacuum. The residue was triturated with ether and filtered to remove the crystalline diethyl hydrazinedicarboxylate.

The filtrate was then chromatographed on silica gel (4 x 12 cm). Elution with a mixture of toluene and EtOAc (8:2) gave the title material as a solid. Recrystallization from EtOAc-hexanes gave 4.34 g (80%) of white needles: mp 94-94.5°C; $[\alpha]_D^{22}$ -33° (c 1.0, $CHCl_3$); uv (EtOH) $\lambda_{max}$: 224 (10,280) and 258 nm (7,400); ir (KBr) $\nu_{max}$: 3200 (NH), 2158 ($N_2$), 2100 ($N_3$), 1738 (C=O of β-lactam), 1728 and 1710 (C=O of ketone and ester) and 1645 cm (amide); ′Hmr (200 MHz, $CDCl_3$) δ: 1.18 (d, J=6.94 Hz, 3H, $CH_3$-1 of diazopropyl), 1.42 (d, J=6.61 Hz, 3H, $CH_3$ of azidoethyl), 3.01 (dd, $J_{H3,H4}$=2.2 Hz, $J_{H3,\ H1}$=7.33 Hz, 1H, H-3 of azetidinone), 3.77 (dd, $J_{H4,H3}$=2.2 Hz, $J_{H4,H1}$=4.60 Hz, 1H, H-4 of azetidinone), 3.85 (m, 1H, H-1 of azidoethyl), 3.93 (dq, $J_{H,\ CH3}$=6.94 Hz, $J_{H,H4}$=4.60 Hz, 1H, H-1 of diazopropyl), 4.73 (m, 2H, $CH_2$ of allyl), 5.3-5.5 and 5.9-6.1 ppm (2 x m, 2 x 2H, CH of allyl and HN). Anal. calcd. for $C_{13}H_{16}N_6O_4$: C 48.75, H 5.03, N 26.24; found: C 48.78, H 5.06, N 26.26.

Procedure 28

(5R,6S) Allyl 6-(1′R-Azidoethyl)-4R-methyl-3,7-dioxo-1-azabicyclo[3.2.0]heptane-2-carboxylate

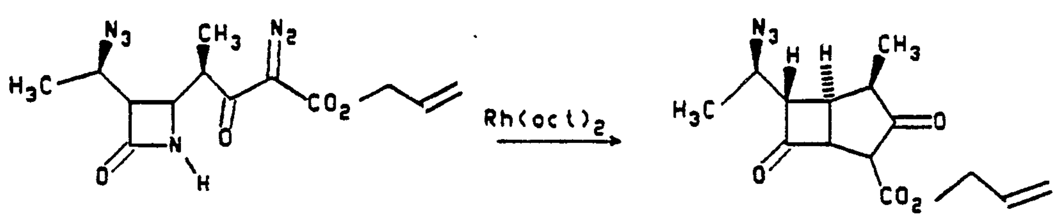


A solution of 3S-(1R-azidoethyl)-4R-(1R-methyl-3-allyoxycarbonyl-2-oxo-3-diazopropyl)azetidinone (0.50 g, 1.56 mmol, Procedure 27) in ethyl acetate (5 mL) was treated under $N_2$ with rhodium (II) octanoate dimer (0.005 g) and heated at 60°C for 30 min. The solvent was then evaporated under vacuum and the residual oil was used as such: ir (NaCl, film) $\nu_{max}$: 2120 ($N_3$), 1770 (C=O of β-lactam) and 1745 cm (C=O of β-keto ester); ′Hmr (200 MHx, $CDCl_3$) δ: 1.22 (d, J=7.79Hz, 3H, $CH_3$-4), 1.50 (d, J-6.51 Hz, 3H, $CH_3CHN$), 2.84 (m, 1H, H-4), 3.21 (dd, $J_{H6,H5}$=2.31 Hz, $J_{H6,H1'}$=8.96 Hz, 1H, H-6), 4.02 (dq, $J_{H1'}$, H6=8.96 Hz, $J_{H1'}$,CH3=6.51 Hz, 1H, $CH_3CHN$), 4.17 (dd, $J_{H5,H6}$= 2.31 Hz, $J_{H5,H4}$= 7.85 Hz, 1H, H-5), 4.66 (s, 1H, H-2), 4.67 (m, 2H, $CH_2$ of allyl), 5.2-5,4 and 5.8-6.1 ppm (2 x m, 2H and 1H, CH of allyl).

Procedure 29

(5S,6S) Allyl 6-(1′R-azidoethyl)-3-(2-cyanoethylthio)-4R-methyl-7-oxo-1-azabicyclo[3.2.0]hept-2-ene-2-car-

boxylate

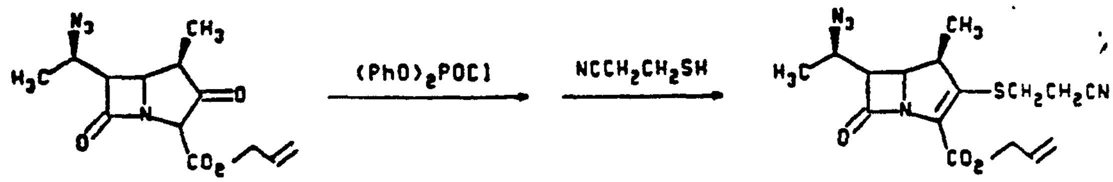


A solution of the crude bicyclic ketone (1.56 mmol, Procedure 28) in dry acetonitrile (5 mL) was cooled to 0-5°C and treated simultaneously with diphenyl chlorophosphate (0.47 g, 1.73 mmol) and N,N-diisopropylethylamine (0.22 g, 1.72 mmol) added dropwise over 5 min. A small crystal of 4-N,N-dimethylaminopyridine was then added and the resulting mixture was stirred for 1 h. The solution was then cooled to -25°C and treated again with N,N-diisopropylethylamine (0.22 g, 1.72 mmol) and β-mercaptopropionitrile (0.27 g, 3.12 mmol). After 1 h, the reaction mixture was quenched by addition of 0.2M pH 7.0 phosphate buffer (50 mL) and ethyl acetate (50 mL). The organic phase was then washed by brine, dried over anhydrous $MgSO_4$ and evaporated under reduced pressure. Chromatography of the residue on silica gel (column dimensions, 4 x 11 cm, elution solvent toluene: EtOAc 8:2) gave 0.50 g (89%) of the title carbapenem. $^1$Hmr and ir of this material are identical to the ones already described (Procedure 25). This product may be reduced and hydrodyzed to the 1'(R)-aminoethyl acid by the method of Procedure 26.

## Procedure 30

3S-(1'S-Azidoethyl)-4R-(1R-methyl-3-allyloxycarbonyl-2-oxo-3-diazopropyl)azetidin-2-one

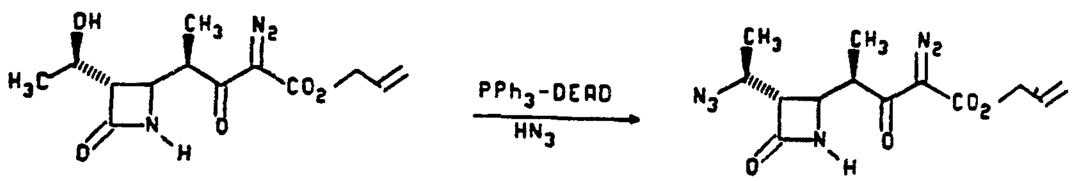


A solution of 3S-(1R-hydroxyethyl)-4R-(1R-methyl-3-allyloxycarbonyl-2-oxo-3-diazopropyl)azetidin-2-one (10.0 g, 33.9 mmol, the starting material used in Procedure 1) in dry tetrahydrofuran (500 mL) was cooled to -40°C and treated with triphenylphosphine (14.2 g, 54.2 mmol) and 69 mL (58.0 mmol) of a 0.84M solution of hydrazoic acid in toluene. Then 9.1 mL (57.8 mmol) of diethyl azodicarboxylate were added dropwise over 10 min and the resulting mixture was slowly warmed to 0°C over 1.5 h. The reaction mixture was then diluted with EtOAc (500 mL) washed with water and brine and dried over anhydrous $MgSO_4$. The solvent was then concentrated under reduced pressure and the residue was triturated with toluene (50 mL) and filtered to remove the diethyl hydrazinedicarboxylate. The filtrate was concentrated again, triturated with ether (50 mL) and filtered to remove the triphenylphosphine oxide. The filtrate was then concentrated and chromatographed on silica gel (7 x 11 cm). Elution with a mixture of toluene and EtOAc (8:2) gave an oil which after trituration with a mixutre of ether and hexanes (7:3) gave 9.35 g (87%) of the title azetidinone as a white solid. Recrystallization from a mixture of ether and hexanes gave white needles: mp 56-57°C; $[\alpha]_D^{22}$ + 33° (c 1.0, $CDCl_3$); uv (EtOH) $\lambda_{max}$: 224 (10,630) and 258 nm (7,547);
ir (KBr) $\nu_{max}$: 3200 (NH), 2156, 2120 and 2080 ($N_2$ and $N_3$), 1755 (C=O of β-lactam), 1720 (C=O of β-ketoester) and 1640 $cm^{-1}$ (amide); $^1$Hmr (200 MHz, $CDCl_3$) δ: 1.19 (d, J=6.59 Hz, 3H, $CH_3$-1 of diazopropyl), 1.45 (d, J=6.72 Hz, 3H, $CH_3$ of azidoethyl), 3.04 (dd, $J_{H3,H4}$= 1.93 Hz, $J_{H3,H1}$=4.24 Hz, 1H,

H-3), 3.8-4.0 (m, H-4, H-1 of diazopropyl and $CH_3CHN_3$ overlapping), 4.73 (m, 2H, $CH_2$ of allyl), 5.3-5.4 and 5.9-6.1 ppm (2 x m, 2 x 2H, CH of allyl and NH).

| | | | |
|---|---|---|---|
| Anal. calcd. for $C_{13}H_{16}N_6O_4$: | C 48.75, | H 5.03, | N 26.24; |
| found: | C 48.25, | H 5.08, | N 25.94. |

Procedure 31

(5R,6S) Allyl (1'S-Azidoethyl)-4R-methyl-3,7-dioxo-1-azabicyclo[3.2.0]heptane-2-carboxylate

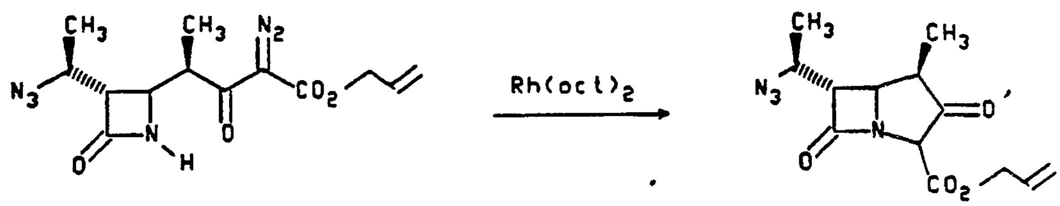


A solution of 3S-(1S-azidoethyl)-4R-(1R-methyl-3-allyloxycarbonyl-2-oxo-3-diazopropyl)azetidin-2-one (0.50 g, 1.56 mmol) in ethyl acetate (5 mL) was treated under $N_2$ with rhodium octanoate dimer and heated to 60°C for 30 min. the solvent was then evaporated under vacuum and the residual oil was used as such for the next step:
ir (NaCl, film)$\nu_{max}$: 2120 ($N_3$), 1768 (C=O of b=lactam) and 1745 $cm^{-1}$ (C=O of $\beta$-ketoester); 'Hmr (200 MHz, $CDCl_3$) $\delta$: 1.22 (d, J=7.81 Hz, 3H, $CH_3$-4), 1.49 (d, J=6.70 Hz, 3H, $CH_3CHN$), 2.81 (m, 1H, H-4), 3.39 (dd, $J_{H6,H5}$=2.34 Hz, $J_{H6,H1'}$=5.65 HZ, 1H, H-6), 4.03 (m, 1H, $CH_3CHN$), 4.17 (dd, $J_{H5,H6}$=2.34 Hz, $J_{H5,H4}$-7.85 Hz, 1H, H-5), 4.7 (m, 3H, $CH_2$ of allyl and H-2 overlapping), 5.2-5.4 and 5.9-6.1 ppm (2 x m, 2H and 1H, CH of allyl).

Procedure 32

(5S,6S) Allyl 6-(1'S-Azidoethyl)-3-(2-cyanoethylthio)-4R-methyl-7-oxo-1-1azabicyclo[3.2.0]hept-2-ene-2-carboxylate

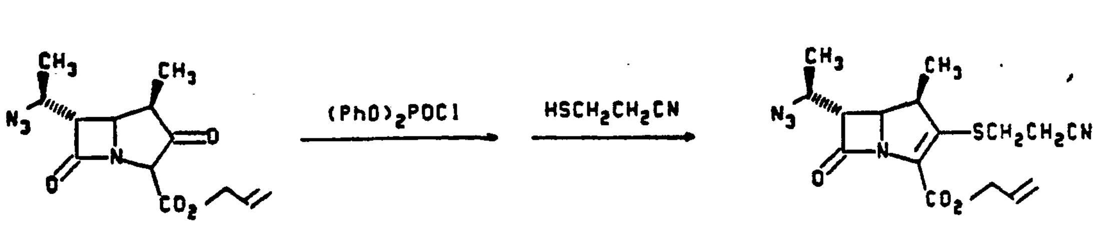


A solution of the crude bycyclic ketone (1.56 mmol, Procedure 31) in dry $CH_3CN$ (5 mL) was cooled to 0-5°C and treated simultaneously with diphenyl chlorophosphate (0.47 g, 1.73 mmol) and N,N-diisopropylethylamine (0.22 g, 1.72 mmol) added dropwise over 5 min. A small crystal of 4-N,N-dimethylaminopyridine was then added and the resulting mixture was then stirred for 30 min. The solution

was then cooled to -25°C and treated with N,N-diisopropylethylamine (0.22 g, 1.72 mmol) and β-mercaptopropionitrile (0.27 g, 3.12 mmol). After 1 h at -25°C, the reaction mixutre was quenched by addition of 0.2M pH 7.0 phosphate buffer and EtOAc (50 mL). The organic phase was washed with brine, dried (anhydros $MgSO_4$) and evaporated.

The residue was chromatographed on silica gel (4 x 11 cm) using a mixture of toluene and ethyl acetate (9:1 to 8:2) as eluent. The first fractions eluted (0.25 g) did not contain a β-lactam as determined by ir. The following fractions gave 0.10 g (18%) of the title carbapenem as an oil. The ir and 'Hmr of this material are identical to those of the product produced in Procedure 34.

## Procedure 33

(5S,6S) Allyl 3-(2-cyanoethylthio)-6-(1'R-hydroxyethyl)-4R-methyl-7-oxo-1-azabicyclo[3.2.0]hept-2-ene-2-carboxylate

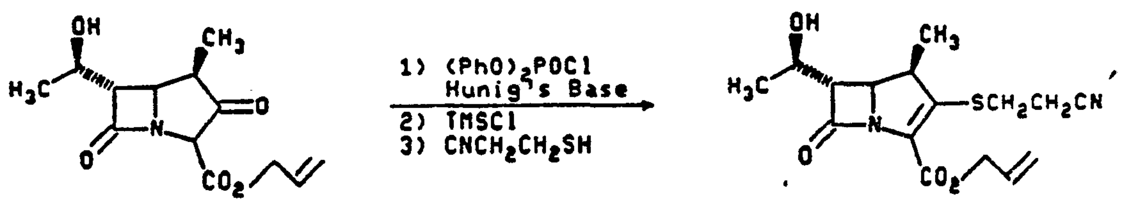


Allyl 6-(1'R-hydroxyethyl)-4R-methyl-3,7-dioxo-1-1azabicyclo[3.2.0]heptane-2-carboxylate was prepared by cyclization of 5.0 g (16.93 mmol) of the diazoketone employed as starting material in Procedure 1 with rhodium acetate catalyst in EtOAc (50 mL) at 60°C for 20 min.) This material, 16.9 mmol., in dry acetonitrile (50 mL) was cooled to 0-5°C and treated under $N_2$ with diphenyl chlorophosphate (5.0 g, 18.5 mmol) and N,N-diisopropylethylamine (3.35 mL, 19.3 mmol) added dropwise over 5 min. A trace of 4-N,N-dimethylaminopyridine (0.003 g) was added and the mixture was stirred for 2 h. Then N,N-diisopropylethylamine (3.0 mL, 17.2 mmol) followed by chlorotrimethylsilane (2.16 mL, 17.0 mmol) were added over 5 min and the resulting solution was stirred for 10 min. β-Mercaptopropionitrile (2.95 g, 33.9 mmol) followed by N,N-diisopropylethylamine (3.35 mL, 19.2 mmol) were added successively and the resulting mixture was stirred at 0-5°C for 18 h. The solution was then diluted with EtOAc (400 mL), washed with water, saturated $NaHCO_3$, brine and dried ($MgSO_4$). Evaporation of the solvent under reduced pressure gave an oil which was diluted with tetrahydrofuran (50 mL) and water (50 mL) and treated at 22°C with acetic acid (1.5 mL). After 30 min, the mixture was diluted with EtOAc (400 mL) washed successively with cold saturated $NaHCO_3$, brine and dried ($MgSO_4$). Evaporation of the solvent gave an oil which was chromatographed on silica gel (9 x 12 cm). Elution with a mixture of toluene and EtOAc (1:1) gave 1.26 g (22%) of the title material as an oil. Crystallization from a mixture of EtOAc and hexanes gave the title compound as a white solid: mp 79-80°C; $[\alpha]_D^{22}$ + 132° (c 1.0, $CHCl_3$); uv (EtOH) $\lambda_{max}$: 316 nm (10, 940); ir (KBr) $\nu_{max}$: 3470 (OH), 2250 (weak C=N), 1755 (C=O of β-lactam) and 1703 $cm^{-1}$ (C=O of ester); 'Hmr (200 MHz, $CDCl_3$) δ: 1.27 (d, J=7.29 Hz, 3H, $CH_3$-4), 1.35 (d, J=6.29 Hz, 3H, $CH_3CHO$), 1.87 (d, J=4 Hz, 1H, OH), 2.6-3.3 (m, 4H, $SCH_2CH_2CN$), 3.27 (dd, $J_{H6,H5}$= 2.61 Hz, $J_{H6,H1'}$= 7.0 Hz, 1H, H-6), 3.41 (dq, $J_{H4,CH3}$=7.29 Hz, $J_{H4,H5}$= 9.27 Hz, 1H, H-4), 4.26 (dd, $J_{H5,H6}$= 2.61 Hz, $J_{H5,H4}$= 9.27 Hz, 1H, H-5), 4.26 (m overlapping with H-5, 1H, $CH_3CHO$), 4.76 (m, 2H, $CH_2$ of allyl), 5.2-5.5 and 5.9-6.1 ppm (m, 2H and 1H, CH of allyl).

| | | | | |
|---|---|---|---|---|
| Anal. calcd. for $C_{16}H_{20}N_2O_4S$: | C 57.13, | H 5.99, | N 8.33, | S 9.53; |
| found: | C 57.03, | H 6.08, | N 8.28, | S 9.27. |

Procedure 34

(5S,6S) Allyl 6-(1′S-Azidoethyl)-3-(2-cyanoethylthio)-4R-methyl-7-oxo-1-azabicyclo[3.2.0]hept-2-ene-2-carboxylate

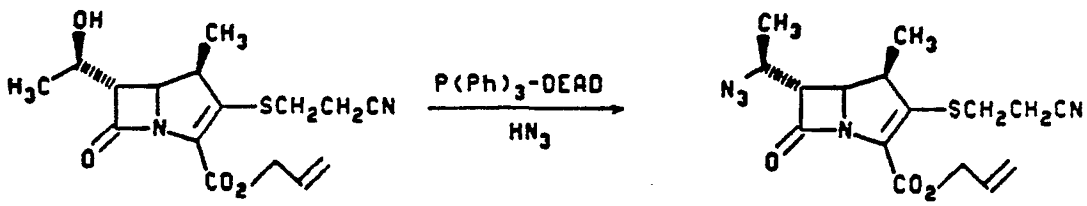


A solution of (5S,6S) allyl 3-(2-cyanoethylthio)-6-(1′R-hydroxyethyl)-4R-methyl-7-oxo-1-azabicyclo [3.2.0]hept-2-ene-2-carboxylate (3.00 g, 8.92 mmol, Procedure 33) in dry tetrahydrofuran (50 mL) was cooled to 0-5°C and treated under $N_2$ with triphenylphosphine (4.68 g, 17.8 mmol) and 25 mL (18.0 mmol) of a 0.72 M solution of hydrazoic acid in toluene. Then 2.80 mL (17.8 mmol) of diethyl azodicarboxylate was added dropwise over 5 min and the resulting solution was stirred at the same temperature for 30 min. The reaction mixture was then diluted with EtOAc (300 mL), washed with saturated aqueous $NaHCO_3$ and brine and dried ($MgSO_4$). Evaporation of the solvent gave an oil which was triturated with toluene and filtered to remove the crystalline diethyl hydrazinedicarboxylate. The filtrate was then chromatographed on silica gel (4.5 x 13 cm). Elution with a mixture of $CH_2Cl_2$ and $CH_3CN$ (0-5%) gave 1.24 g (38%) of the title carbapenem as an oil:

ir (NaCl film)$\nu_{max}$: 2255 (weak, C=N), 2120 ($_{N3}$), 1770 (C-O of β-lactam) and 1710 $cm^{-1}$ (C=O of ester); ′Hmr (200 MHz, $CDCl_3$) δ: 1.23 (d, J=7.31 Hz, 3H, $CH_3$-4), 1.47 (d, J=6.67 Hz, 3H, $CH_3CHN$), 2.6-3.3 (m, 4H, $SCH_2CH_2CN$), 3.4 (m, 2H, H-6 and H-4 overlapping), 4.0 (m, 1H, $CH_3$,CHN), 4.2 (dd, $J_{H5,H6}$= 2.5 Hz, 1H, H-5), 4.6-4.9 (m, 2H, $CH_2$ of allyl), 5.2-5.5 and 5.9-6.1 (m, 2H and 1H, CH of allyl). This product also contains some elimination product (olefin at position 6) in a ratio 7:3.

Procedure 35

(5S,6R) 6-(1′S-Aminoethyl)-3-(2-cyanoethylthio)-4R-methyl-7-oxo-1-azabicyclo[3.2.0]hept-2-ene-2-carboxylic Acid (41787)

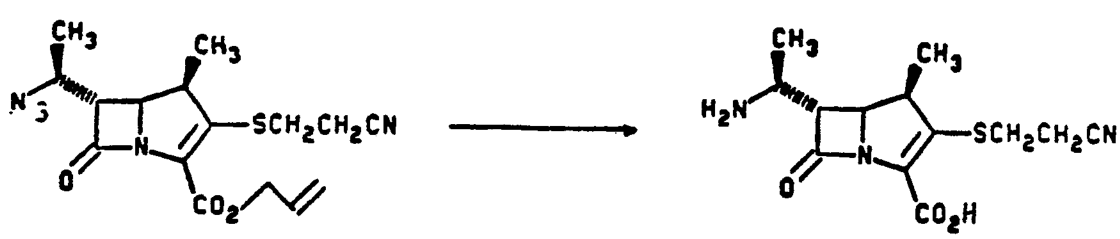

A solution of (5S,6S) allyl 6-(1′S-azidoethyl)-3-(2-cyanoethylthio)-4R-methyl-7-oxo-1-azabicyclo[3.2.0]-hept-2-ene-2-carboxylate (2.20 g, 6.08 mmol, Procedure 32 or 34) in dry benzene (125 mL) was treated under $N_2$ with triphenylphosphine (1.76 g, 6.71 mmol) and heated under reflux for 30 min. The solution of phosphinimine was then cooled to 22°C, treated with p-nitrobenzaldehyde (1.01 g, 6.68 mmol) and stirred at the same temperture for 20 h. Evaporation of the solvent gave the crude Schiff base mixed with triphenylphosphine oxide which was used as such for the next step.

A solution of the crude Schiff base in dry $CH_2Cl_2$ (100 mL) was treated at 22°C and under $N_2$ with tetrakis(triphenylphosphine)palladium (0.225 g) and 13.4 mL (6.7 mmol) of a 0.5 M solution of potassium 2-ethylhexanoate in EtOAc. After 20 min, 100 mL of 0.2 M pH 6.0 phosphate buffer was added and the mixture was stirred vigorously for 30 min. The aqueous phase was collected and the organic phase was extracted with water (2 x 50 mL). The combined aqueous phases were concentrated in vacuo (T < 10°C) and chromatogrphaed twice on reversed phase silica gel (μ-Bondapak c-18, 3.5 x 9 cm; first column $H_2O$ pH 6.8, 0.01 M phosphate buffer-$CH_3CN$ 5%; second column elution $H_2O$). Lyophilization of the uv active fractions gave 0.26 g (14%) of the title carbapenem as a white amorphous powder which was still contaminated by the elimination product at position 6. Final purification was done by hplc (μ-Bondapak c-18): $[\alpha]_D^{22}$ + 75°(C 1.0; $H_2O$), hplc on μ-Bondapak c-18, 5% $CH_3CN$-$H_2O$ pH 7.4 phosphate buffer, flow rate 1 mL/min, uv detector 304 nm, retention time 6.7 min, purity 99%; uv ($H_2O$, pH 7.4 phosphate buffer) $\lambda_{max}$: 300 nm (8,270);

ir (KBr)$\nu_{max}$: 2250 (CN), 1758 (C=O of β-lactam) and 1585 $cm^{-1}$ (C=O of ester); $'$Hmr (200 MHz, $D_2O$) δ: 1.24 (d, J=7.28 Hz, 3H, $CH_3$-4), 1.44 (d, J=6.69 Hz,3H, $CH_3CHN$), 2.8-3.3 (m, 4H, $SCH_2CH_2CN$), 3.54 (m, 1H, H-4) 3.65 (dd, $J_{H6,H5}$=2.48 Hz, $J_{H6,H1'}$=7.63 Hz, 1H, H-6), 3.89 (m, 1H, $CH_3CHN$) and 4.26 ppm (dd, $J_{H5,H6}$=2.48 Hz, $J_{H5,H4}$= 8.99 Hz, 1H, H-5).

By uv, at 37°C in a pH 7.4 phosphate buffer the half-life was 1.5 h. By hplc, at 37°C in a pH 2.0 citric acid, the half-life was evaluated to be 35 min.

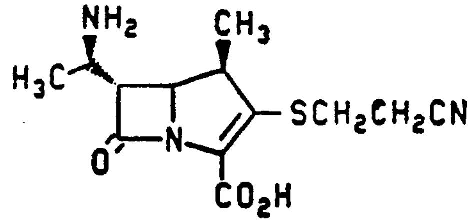

BMY-41787

M.I.C.

| | |
|---|---|
| Str. pneu. A9585 | 4.000 |
| Str. pyo. A9604 | 2.000 |
| Str. faec. A20688 | 125.000 |
| Staph. aur. A9537 | 8.000 |
| Staph. serum | 16.000 |
| Staph. aur. Pen+ | 16.000 |
| Staph. aur. MR | 125.000 |
| E. coli A15119 | 0.060 |
| E. coli A20341 | 0.060 |
| K. pneu. A9664 | 0.130 |
| K. pneu. A20468 | 2.000 |
| Ent. clo. A9659 | 0.250 |
| Ent. clo. A9656 | 0.250 |
| P. mir. A9900 | 0.130 |
| P. vul. A21559 | 0.060 |
| P. morg. A15153 | 2.000 |
| P. rett. A22424 | 2.000 |
| Ser. mars. A20019 | 2.000 |
| Ps. aeru. A9843 | 0.130 |
| Ps. aeru. CarbR | 0.130 |
| t1/2, pH 7.4 (h.): | 1.5 |
| t1/2, pH 2 (min.): | 35 |
| DHP: | |

Procedure 36

(5S,6R) 6-(1′R-N-trimethylammonium-ethyl-4R-methyl-3-methylthio-7-oxo-1-azabicyclo [3.2.0]hept-2-ene-2-carboxylate (40945)

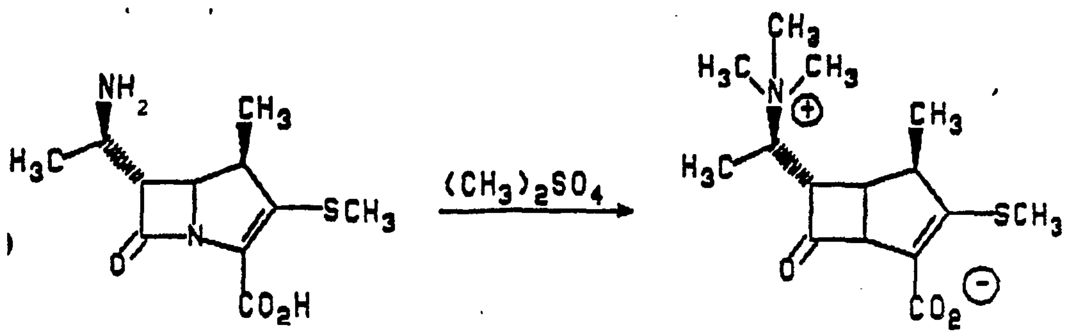


A suspension of (5S,6R) 6-(1′R-aminoethyl)-4R-methyl-3-methylthio-7-oxo-1-azabicyclo[3.2.0]hept-2-ene-2-carboxylic acid (0.506 g, 1.97 mmol, Procedure 9) in a mixture of water (20 mL) and dioxane (8 mL) at 0°C, was adjusted to pH 9.0 with 1M NaOH. Then dimethyl sulfate (1.0 mL, 10.54 mmol) was added all at once followed by additional 1M NaOH solution to provide a total addition of 2.0 mmol of NaOH. The pH was maintained at pH 9.0 by adding 1M NaOH during the reaction. After 2 h at 0°C, 0.2 M pH 7.0 phosphate buffer (30 mL) was added and the solution was concentrated to one-half the original volume under vacuum. The product was then chromatographed on reversed phase silica gel (μ-Bondapak c-18, 3 x 9 cm). Elution with water gave 0.310 g (52%) of the title material as a white amorphous solid after lyophilization: hplc on μ-Bondapak c-18, 4 mm x 30 cm, 5% $CH_3C$-$H_2O$ pH 7.0 phosphate buffer, flow rate 1 mL/min, μv detector at 304 nm, retention time 5.5 min, purity 99%; $[\alpha]_D^{22}$ + 131° (c 1.0, $H_2O$); uv ($H_2O$, pH 7.4 phosphate buffer) $\lambda_{max}$: 304 nm (9,180); ir (KBr) $\lambda_{max}$: 1750 (C=O of β-lactam), 1600 $cm^{-1}$ (C=O of carboxylate); ′Hmr (200 MHz, $D_2O$) δ: 1.28 (d, J=7.24 Hz, 3H, $CH_3$-4), 1.61 (d, J=6.57 Hz, 3H, $CH_3CHN$), 2.37 (s, 3H, $SCH_3$), 3.17 (s, 9H, $NCH_3$), 3.54 (m, 1H, H-4), 3.96 (broad, 1H, H-6), 4.06 (m, 1H, H-1′) and 4.15 ppm (dd, $J_{H5,H6}$ =2.38, $J_{H5,H4}$=8.53, 1H, H-5).

By uv this carbapenem was found to have a half-life of 143 h at 37°C in a pH 7.4 phosphate buffer. This half-life was also verified by ′Hmr. By hplc in a pH 2.0 citric acid buffer at 37°C, the half-life was evaluated to be 28 h. This half-life was also verified by ′Hmr.

| M.I.C. | |
|---|---|
| Str. penu. A 9585 | 8.000 |
| Str. pyo. A9604 | 16.000 |
| Str. faec. A20688 | 125.000 |
| Staph. aur. A9537 | 8.000 |
| Staph. serum | 63.000 |
| Staph aur. Pen + | 125.000 |
| Staph aur. MR | 125.000 |
| E. coli A15119 | 63.000 |
| E. coli A20341 | 63.000 |
| K. pneu. A9664 | 125.000 |
| K. pneu. A20468 | 125.000 |
| Ent. clo. A9659 | 125.000 |
| Ent. clo. A9656 | 63.000 |
| P. mir. A9900 | 32.000 |
| P. vul. A21559 | 63.000 |
| P. morg. A15153 | 125.000 |
| P. rett. A22424 | 125.000 |
| Ser. mars. A20019 | 125.000 |
| Ps. aeru. A9843 | 125.000 |
| Ps. aeru. CarbR | 125.000 |
| t1/2, pH 7.4 (h.): | 143 |
| t1/2, pH 2 (h): | 28 |

## Procedure 37

(5S,6R) Allyl 6-(1′R-(N-Carboallyloxy-L-leucylglycyl)amino-ethyl)-4R-methyl-3-methylthio-7-oxo-1-azabicyclo[3.2.0]hept-2-ene-carboxylate

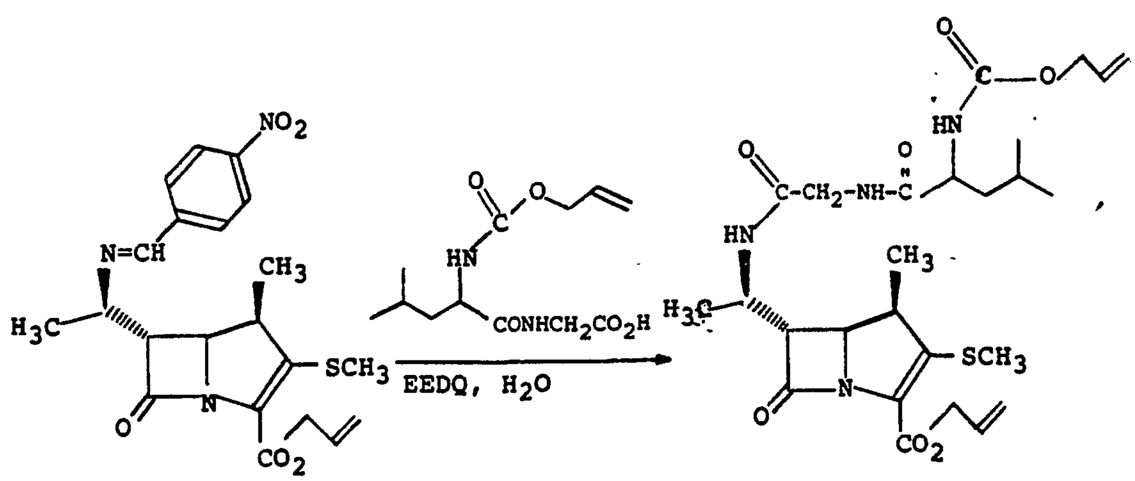


A solution of the crude (5S, 6R) allyl 4R-methyl-3-methylthio-6-(1′R-p-nitrobenzylideneamino-ethyl)-7-oxo-1-azabicyclo[3.2.0]-hept-2-ene-2-carboxylate (3.10 mmol), prepared as in Procedure 9, Method B in tetrahydrofuran (15 mL) at 23°C was treated with EEDQ (0.90 g, 3.63 mmol)) and a solution of N-carboallyloxy-L-leucylglycine (0.95 g, 3.49 mmol) in THF (5 mL). Water (0.2 mL, 11.1 mmol) was added and the solution was stirred for 4.5 h. The reaction mixture was then diluted with EtOAc (200 mL), washed successively with pH 7.0 phosphate buffer and brine dried ($MgSO_4$) and evaporated. The residual oil was then chromatographed on silacagel (3x13 cm) using a toluene-EtOAc gradient (1:1 to EtOAc). The title carbapenem was obtained as a solid (0.76 g, 45%) which was recrystallized from a mixture of EtOAc-hexanes to give needles: mp 148-149° C; $[\alpha]_D^{23}$ 137° (c 1.0, $CHCl_3$); uv $(EtOH)_{max}$: 320nm (10,560);
ir $(KBr)\nu_{max}$: 1765 (C=O of $\beta$-lactam), 1705 (sh, C=O of ester), 1962 (sh), 1682, 1642, 1545 and 1535 $cm^{-1}$ (amide);
Hmr (200 MHz, $CDCl_3$) $\delta$: 0.93 and 0.96 (2 x d, J=5.2 Hz, and J=5.7 Hz, 2 x 3H, $CH_3$ of leucine), 1.18 (d, J=7.25 Hz, 3H, $CH_3$-4), 1.38 (d, J=6.77 Hz, 3H, $CH_3CHN$), 1.5 - 1.7 (m, 3H, H-3 and H-4 of leucine), 2.36 (s, 3H, $SCH_3$), 3.23 (dd, $J_{H6,H1}$ = 8.90 Hz, 1H, H-6), 3.37 (dd, $J_{H4,CH3}$ = 7.25 Hz, $J_{H4,H5}$ = 9.0 Hz, 1H, H-4), 3.37 (dd, 3.90 (AB part of ABX system, $J_{AX}$ = 6.64 Hz, $J_{BX}$ = 5.57 Hz, $J_{AB}$ = 16.92 Hz, $\Delta v$=53.6 Hz, 2H, $CH_2$ of glycine), 4.0 (m overlapping with $CH_2$ of glycine, 1H, H-2 of leucine), 4.15 (dd, $J_{H5,H6}$ = 2.49 Hz, $J_{H5,H4}$ = 9.0 Hz, 1H, H-5), 4.40 (m, 1H, $CH_3CHN$), 4.56 and 4.74 (2 m, 2 x 2H, $CH_2$ of allyl), 5.1-5.5 and 5.8-6.1 (m, 6H, CH of allyl) 6.6 and 6.8 ppm (broad m, NH).

| | | | | |
|---|---|---|---|---|
| Anal. calcd. for $C_{26}H_{38}N_4O_7S$: | C 56.71, | H 6.96, | 10.17, | S 5.82; |
| found: | C 56.27, | H 7.0, | N 9.94, | S 6.19. |

This substance is then deallylated to remove the allyloxycarbonyl protecting group and the allyl ester group by treatment with triphenylphosphine and Pd(0) as described in Procedure 38. The resulting product exhibits the following biological activity.

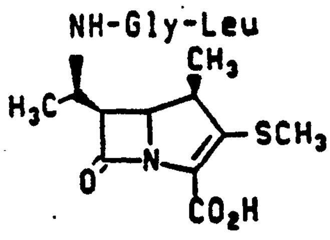

BMY-41144

| | |
|---|---|
| M.I.C. | |
| Str. pneu. A9585 | 8.000 |
| Str. pyo. A9604 | 2.000 |
| Str. faec. A20688 | 125.000 |
| Staph. aur. A9537 | 4.000 |
| Staph. serum | 16.000 |
| Staph. aur. Pen+ | 8.000 |
| Staph. aur. MR | 63.000 |
| E. coli A15119 | 0.250 |
| E. coli A20341 | 0.250 |
| K. pneu. A9664 | 1.000 |
| K. pneu. A20468 | 1.000 |
| Ent. clo. A9659 | 2.000 |
| Ent. clo. A9656 | 0.500 |
| P. mir. A9900 | 0.500 |
| P. vul. A21559 | 2.000 |
| P. morg. A15153 | 0.500 |
| P. rett. A22424 | 16.000 |
| Ser. mars. A20019 | 16.000 |
| Ps. aeru. A9843 | 63.000 |
| Ps. aeru CarbR | 125.000 |

| PD50, | IM | PO |
|---|---|---|
| S. aur. | | |
| E. coli | 7.20 | 25.00 |

t1/2, pH 7.4 (h.): 40
t1/2, pH 2 (min.): 5

Cmax, PO/50: 2

Procedure 38

(5S,6R) 6-(1′R-(L-alanylglycyl)aminoethyl)-4R-methyl-3-methylthio-7-oxo-1-azabicyclo[3.2.0]hept-2-ene--2-carboxylic Acid (41145)

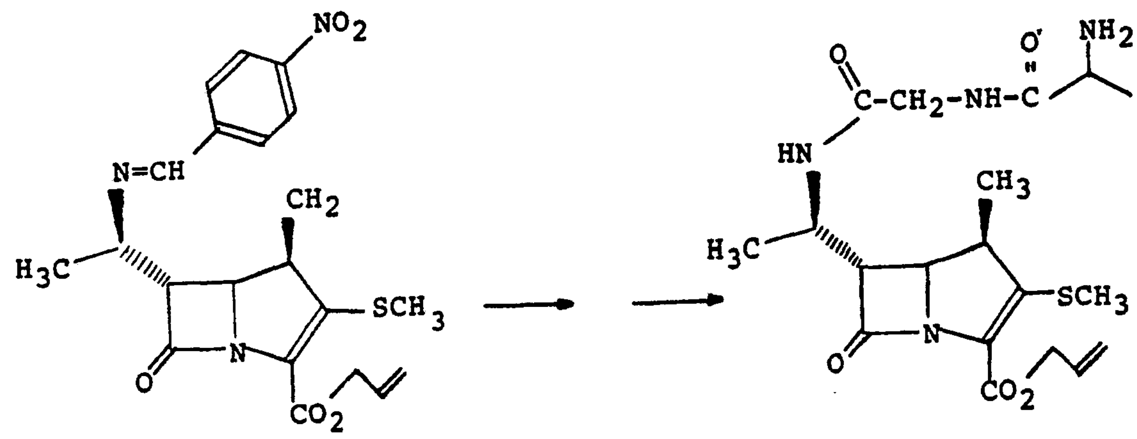

A solution of L-alanyglycine (1.0 g, 6.84 mmol) in water (20 mL) was cooled to 0-5° C and treated with $NaHCO_3$ (1.43 g, 17.1 mmol) and allyl chloroformate (0.8 mL, 7.53 mmol). The resulting mixture was stirred vigorously for 1 h at 0-5° C and for 30 min at 22° C. The reaction mixture was then washed with ether (20 mL) and the cooled aqueous phase was acidified to pH 3.0 concentrated HCl. The solution was then extracted with ether ( 5 x 20 mL). The combined ether extracts were dried ($MgSO_4$) and evaporated to give 0.42 g (27%) of N-carboallyloxy-L-alanylglycine as an oil:

ir (NaCl, film)$\nu_{max}$: 1720 (C=O of acid), 1670 and 1530 cm $^{-1}$ (C=O of amide); Hmr (200 MHz, $CDCl_3$) $\delta$: 1.40 (d, J=7.04 Hz, 3H, $CH_3$ of alanine, 4.05 (m, 2H, $CH_2$ of glycine), 4.35 (m, 1H, H-2 of alanine), 4.56 (m, 2H, $CH_2$ of allyl), 5.2-54 and 5.8-6.1 (m, 3H, OH of allyl), 5.72 (d, J=5.25 Hz, 1H, NH of alanine) and 7.10 ppm (t, J=5.13 Hz, 1H, NH of glycine).

A solution of the crude (5S,6R) allyl 4R-methyl-3-methylthio-6-(1'R-p-nitrobenzylideneamino-ethyl)-7-oxo-1-azabicyclo[3.2.0]hept-2-ene-2-carboxylate (1.86 mmol, prepared as in Procedure 9, Method B) in THF (10 mL) was treated at 22° C with a solution of N-carboallyloxy-L-alanylglycine (.0.42 g, 1.83 mmol) in THF (10 mL) and EEDQ (0.50 g, 2.02 mmol). Water (0.2 mL, 11.1 mmol) was added and the reaction mixture was stirred for 3 h. The solution was then diluted with EtOAc (50 mL), washed successively with pH 7.0 phosphate buffer and brine and dried ($MgSO_4$). Evaporation of the solvent gave an oil which was chromatographed on silicagel (2 x 10 cm). Elution with a mixture of EtOAc and EtOH (95:5) gave 0.430 g (45%) of the title carbapenem as a white solid. Recrystallizatin from EtOAc-hexane gave (5S,6R)allyl 6-(1'R-(N-carboallyloxy-L-alanylglycyl)aminoethyl)-4R-methyl-3-methylthio-7-oxo-1-azabicyclo[3.2.0]hept-2-ene-2-carboxylate as white prisms: mp 150-151° C; $[\alpha]_D^{22}$ + 80° (c 1.0, $CHCl_3$); uv (EtOH) $\lambda_{max}$: 320 nm (11,500); ir (KBr)$\nu_{max}$: 1750 (C=O of $\beta$-lactam), 1700 (sh), 1697, 1680, 1664 and 1535 $cm^{-1}$ (amide); Hmr (200 MHz, $CDCl_3$) $\delta$: 1.19 (d, J=7.25 Hz, 3H, $CH_3$-4), 1.38 and 1.42 (2 x d, J=6.83 Hz and J=7.11 Hz, 2 x 3H, $CH_3$CHN-6 and $CH_3$CHN of alanine), 2.37 (s, 3H, $SCH_3$), 3.27 (dd, $J_{H6,H5}$ = 2.41 Hz, $J_{H6,H1}$ = 8.71 Hz, 1H, H-6), 3.38 (dq, $J_{H4,CH3}$ - 7.25 HZ, $J_{H4,H5}$ = 8.95 Hz, 1H, H-4), 3.91 (AB part of ABX system, $J_{AX}$ = 6.36 Hz, $J_{BX}$ = 5.75 Hz, $J_{AB}$ = 16.94 Hz, $\Delta\nu$ = 42.6 Hz, 2H, $CH_2$ of glycine), 4.1 (m overlapping with $CH_2$ of glycine, 1H, H-2 of alanine), 4.14 (dd, $J_{H5,H6}$ = 2.41 Hz, $J_{H5,H4}$ = 8.95 Hz, 1H, H-5), 4.40 (m, 1H, $CH_3$CHN-6), 4.6 and 4.75 (2 x m, 2 x 2H, $CH_2$ of allyl), 5.2-5.5 and 5.8-6.1 (m, 6H, CH of allyl), 6.2 and 6.8 ppm (broad m, NH).

| | | | | |
|---|---|---|---|---|
| Anal. calcd. for $C_{23}H_{32}N_4O_7S$: | C 54.32, | H 6.34, | N 11.02, | S 6.30; |
| found: | C 54.21, | H 6.46, | N 10.75, | S 6.22. |

A solution of the foregoing carballyloxy carbapenem (0.300 g, 0.59 mmol) in dry $CH_2Cl_2$ (5 mL) was deallylated by treatment at 22° C and under $N_2$ with triphenylphosphine (0.030 g), tetrakis (triphenylphosphine)palladium [0] (0.030 g) and N-methylaniline (0.20 mL, 1.84 mmol). After a few minutes, a solid began to precipitate and the mixture was stirred for 2 h. The solid was then collected by filtration and washed with $CH_2Cl_2$ to give 0.180 g of the crude title carbapenem. Chromatography of this material on reversed phase silica gel ($\mu$-Bondapak c-18, 2.5 x 12 cm, elution $H_2O$-$CH_3CN$ 0.10%) gave 0.112 g (50%) of the pure carbapenem as a white powder after lyophilization: hplc on $\mu$-Bondapak c-18, 4 mm, x 30 cm,

8% $CH_3CN$-$H_2O$ pH 7.4 phosphate buffer, flow rate 1 mL/min, uv detector at 302 nm, retention time 6.0 min, purity 98.5%; uv ($H_2O$, pH 7.4 phosphate buffer) $\lambda_{max}$: 304 nm (8,800);
ir (KBr)$\nu_{max}$: 1750 (C=O of $\beta$-lactam), 1660 and 1540 (broad, amide) and 1560 $cm^{-1}$ (broad, C=O of carboxylate);
Hmr (200 MHz, $D_2O$) $\delta$: 1.18 (d, J=7.22 Hz, 3H, $CH_3$-4), 1.30 (d, J=6.81 Hz, 3H, $CH_3$CH-6), 1.55 (d, J=711 Hz, $CH_3$ of alanine), 2.36 (s, 3H, $SCH_3$), 3.45 (m overlapping with H-6, 1H, H-4), 3.49 (dd, $J_{H6,H5}$ = 2.20 Hz, $J_{H6,H1}$ = 6.4, 3.97 (ABq, $J_{AB}$ = 16.58 Hz, $\Delta$v= 10.4 Hz, 2H, $CH_2$ of glycine), 4.07 (dd, $J_{H5,H6}$ = 2.20 Hz, $J_{H5,H4}$ = 8.80 Hz, 1H, H-5), 4.13 (q, J=7.11 Hz, 1H, H-2 of alanine) and 4.39 ppm (m, 1H, $CH_3$CHN-6).

By uv, at 37 °C in a pH 7.4 phosphate buffer, the carbapenem has a half-life of 29 h. By hplc at 37 °C in a pH 2.0 citric acid buffer, the half-life was evaluated to be less than 5 min.

| M.I.C. | | |
|---|---|---|
| Str. penu. A9585 | 4.000 | |
| Str. pyo. A9604 | 4.000 | |
| Str. faec. A20688 | 32.000 | |
| Staph. aur. A9537 | 16.000 | |
| Staph. serum | 16.000 | |
| Staph. aur. Pen+ | 32.000 | |
| Staph. aur. MR | 125.000 | |
| E. coli A15119 | 0.500 | |
| E. coli A20341 | 0.500 | |
| K. pneu. A9664 | 2.000 | |
| K. pneu. A20468 | 2.000 | |
| Ent. clo. A9659 | 2.000 | |
| Ent. clo. A9656 | 2.000 | |
| P. mir. A9900 | 0.500 | |
| P. vul. A21559 | 2.000 | |
| P. morg. A15153 | 2.000 | |
| P. rett. A22424 | 32.000 | |
| Ser. mars. A20019 | 4.000 | |
| Ps. aeur. A9843 | 32.000 | |
| Ps. aeur. CarbR | 125.000 | |
| PD50, | IM | PO |
| S. aur. | | |
| E. coli | 12.50 | 25.00 |
| t1/2, pH 7.4 (h.): | | 29 |
| t1/2, pH 2 (min.): | | 5 |
| Cmax, PO/50: | | 2 |

## Procedure 39.

(5S,6R)-6-{1′R-(L-Alanyl-L-leucyl)aminoethyl}-4R-methyl-3-methylthio-7-oxo-1-azabicyclo[3.2.0]hept-2-ene-2-carboxylic Acid (41248)

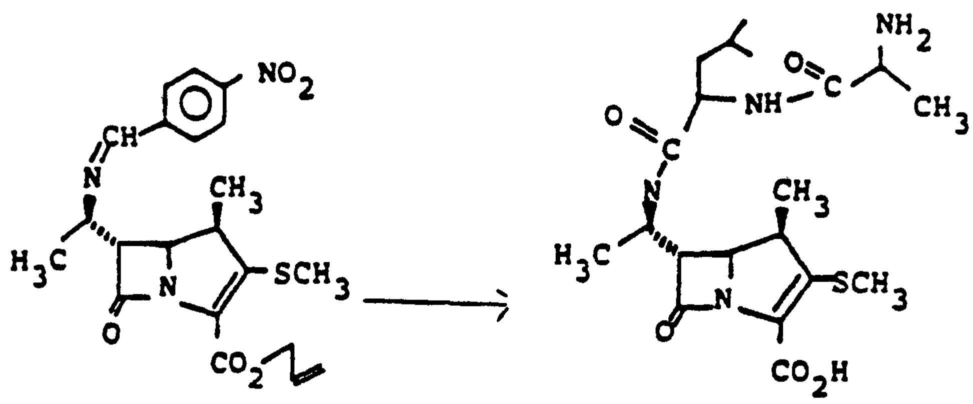

A mixture of L-alanyl-L-leucine (1.00 g, 4.36 mmol) and $NaHCO_3$ (0.92 g, 11.0 mmol) in water (10 mL) was treated at 0°C with allyl chloroformate (0.47 mL, 4.42 mmol). The resulting mixture was stirred vigorously at 0°C for 30 min and at 22°C for 2.5 h. The reaction mixture was then washed with ether. The aqueous phase was cooled to 0°C, adjusted to pH 3.0 with concentrated HCl, saturated with NaCl and extracted with ether (3 x 20 mL). The combined organic extracts were dried ($MgSO_4$) and evaporated to yield 1.08 g (86%) of N-carboallyloxy-L-alanyl-L-leucine as a foam. Crystallization from ether gave a white solid: mp 109-111°C;

ir (KBr) $\nu_{max}$: 1710 and 1700 (C=O), 1650 and 1550 $cm^{-1}$ (amide); 'Hmr (200 MHz, $CdCl_3$) δ: 0.93 and 0.95 2 x d, J= 5.80 Hz and J=5.93 Hz, 2 x 3H, $CH_3$ of leucine), 1.38 (d, J=7.04, 3H, $CH_3$ of alanine), 1.6-1.8 (m, 3H, H-3 and H-4 of leucine), 4.28 (m, 1H, H-2 of leucine), 4.57 (m, 3H, H-2 of alanine and $CH_2$ of allyl), 5.2-5.4 and 5.8-6.0 (m, 3H, CH of allyl), 5.5 and 6.6 ppm (broad d, 2 x 1H, NH).

| | | | |
|---|---|---|---|
| Anal. calcd. for $C_{13}H_{22}N_2O_5$: | C 54.53, | H 7.74, | N 9.78; |
| found: | C 54.62, | H 7.89, | N 9.76. |

A solution of crude (5S,6R) allyl 4R-methyl-3-methylthio-6-(1'R-(p-nitrobenzylideneamino)ethyl-7-oxo-1-azabicyclo[3.2.0]hept-2-ene-2-carboxylate (2.10 mmol, prepared as described in Procedure 9, Method B) in THF (20 mL) was treated at 22°C with N-carboallyloxy-L-alanyl-L-leucine (0.93 g, 3.24 mmol) and EEDQ (0.84 g, 3.39 mmol). Water (0.23 mL, 12.8 mmol) was then added and the mixture was stirred at 22°C for 6 h. The reaction mixture was then diluted with EtOAc (200 mL) washed with pH 7.0 aqueous phosphate buffer and brine and dried ($MgSO_4$). Evaporation of the solvent gave an oil which was chromatographed twice on silica gel (3 x 14 cm, first column $CH_2Cl_2$-$CH_3CN$, 1:1 and second column toluene: EtOAc 1:1) to give 0.72 g (41%) of the desired acylated allyl ester as an oil. The ester was crystallized with difficulty from a mixture of EtOAc and hexane: mp 67-75°C; $[\alpha]_D^{22}$ 0° (c 1.0, CHCl3); uv (EtOH)$\lambda_{max}$: 320 nm (11,690)

ir (KBr)$\nu_{max}$: 1770 (C=O of β-lactam), 1705 (C=O of ester), 1650 and 1535 $cm^{-1}$ (amide); 'Hmr (200 MHz, $CDCl_3$ δ: 0.90 and 0.94 (2 x d, J=6.16 Hz and J=6.16 Hz, 2 x 3H, $CH_3$ of leucine), 1.21 (d, J=7.26 Hz, 3H, $CH_3$-4), 1.37 and 1.40 (2 x d, J=6.52 Hz and J=6.91 Hz, 2 x 3H, $CH_3CHN$-6 and $CH_3$ of alanine);1.4-1.8 (m, 3H, H-3 and H-4 of leucine), 2.37 (s, 3H, $SCH_3$-3), 3.35 (dd, $J_{H6,H5}$=2.45 Hz, $J_{H6,H1'}$=8.06 Hz, 1H, H-6), 3.35 (m overlapping with H-6, 1H, H-4), 4.08 (dd, $J_{H5,H6}$=2.45 Hz, $J_{H5,H4}$= 8.85 Hz, 1H, H-5), 4.13 and 4.3 (2 x m, 1H and 2H, $CH_3CHN$, H-2 of alanine and H-2 of leucine), 4.58 and 4.75 (2 x m, 2 x 2H, $CH_2$ of allyl), 5.2-5.5 and 5.8-6.1 (m, 6H, CH of allyl), 5.11, 6.41 and 6.5 ppm (broad d, 3 x 1H, NH).

| | | | | |
|---|---|---|---|---|
| Anal. calcd. for $C_{27}H_{40}N_4O_7S$: | C 57.43, | H 7.14, | N 9.92, | S 5.68: |
| found: | C 56.79, | H 7.00 | N 9.70, | S 5.68. |

A solution of the (5S,6R) allyl 6-{1'R(N-carboallyloxy-L-alanyl-L-leucyl)aminoethyl]-4R-methyl-3-methylthio-7-oxo-1-azabicyclo[3.2.0]hept-2-ene-2-carboxylate (0.60 g, 1.06 mmol) prepared above in dry $CH_2Cl_2$ (10 mL) was treated at 22°C and under $N_2$ with triphenylphosphine (0.060 g), tetrakis (triphenylphosphine) palladium (0) (0.060 g) and N-methylaniline (0.46 mL, 4.24 mmol). After 3 h at 22°C, the reaction mixture was extracted with water (3 x 20 mL). The combined aqueous extracts were maintained

under vacuum to remove traces of organic solvent and then chromatographed on reversed phase silica gel (μ-Bondapak c-18, 3.5 x 8 cm). Elution with a mixture of water and acetonitrile (8:2) gave 0.16 g (34%) of the title carbapenem as a white amorphous solid after freeze drying: $[\alpha]_D^{22}$ + 138° (c 1.0, $H_2O$); hplc on μ-Bondapak c-18, 30 cm x 4 mm, 15% $CH_3CN$-$H_2O$ pH 7.4 phosphate buffer, flow rate 1.5 mL/min, uv detector 304 nm, retention time 7.5 min, purity 99%; uv ($H_2O$), pH 7.4 phosphate buffer)$\lambda_{max}$: 304 n, (10,800);
ir (KBr)$\nu_{max}$: 1750 (C=O of B-lactam, 1655 and 1540 (amide) and 1580 $cm^{-1}$ (broad, C=O of carboxylate); ′Hmr (200 MHz, $CDCl_3$) δ: 0.91 and 0.95 (2 x d, J=6.44 Hz and J=7.17 Hz, 2 x 3H, $CH_3$ of leucine), 1.18 (d, J=7.22 Hz, 3H, $CH_3$-4), 1.30 (d, J=6.81 Hz, 3H, $CH_3CHN$-6), 1.52 (d, J=7.10 Hz, 3H, $CH_3$ of alanine), 1.6 (m, 3H, H-3 and H-4 of leucine), 2.35 (s, 3H, $SCH_3$-$\bar{3}$), 3.4 (m,1H, H-4), 3.47 (dd, $J_{H6,H5}$=2.38 Hz, $J_{H6,H1'}$ = 7.28 Hz, 1H, H-6), 4.04 (dd, $J_{H5,H6}$=2.38 Hz, $J_{H5,H4}$=8.91 Hz, 1H, H-5), 4.06 (q, J=7.10 Hz, 1H, H-2 of alanine) and 4.40 ppm (m, 2H, H-2 of leucine and $CH_3C\underline{H}N$-6).

By uv at 37° C in a pH 7.4 phosphate buffer, this carbapenem has a half-life of 46 h. By hplc, at 37° C in a pH 2.0 citric acid buffer, the half-life was evaluated to be less than 5 min.

| M.I.C. | | |
|---|---|---|
| Str. pneu. A9585 | 1.000 | |
| Str. pyo. A9604 | 2.000 | |
| Str. faec. A20688 | 125.000 | |
| Staph. aur. A9537 | 16.000 | |
| Staph. serum | 16.000 | |
| Staph. aur. Pen + | 32.000 | |
| Staph. aur. MR | 125.000 | |
| E. coli A15119 | 16.000 | |
| E. coli A20341 | 63.000 | |
| K. pneu. A9664 | 125.000 | |
| K. pneu. A20468 | 63.000 | |
| Ent. clo. A9659 | 63.000 | |
| Ent. clo. A9656 | 32.000 | |
| P. mir. A9900 | 8.000 | |
| P. vul. A21559 | 32.000 | |
| P. morg. A15153 | 32.000 | |
| P. rett. A22424 | 125.000 | |
| Ser. mars. A20019 | 125.000 | |
| Ps. aeur. A9843 | 125.000 | |
| Ps. aeur. CarbR | 125.000 | |
| PD50, | IM | PO |
| S. aur. | | |
| E. coli | 25.00 | 25.00 |
| t1/2, pH 7.4 (h.): | | 46 |
| t1/2, pH 2 (min.): | | 5 |
| DHP: | | |
| Cmax, PO/50: | | 2 |

Procedure 40

3S,4R-3-[(1R)-1-t-Butyldimethylsilyloxyethyl]-4-(1R)-1-ethyl-3-allyloxycarbonyl-2-oxypropyl]-2-azetidin-2-one

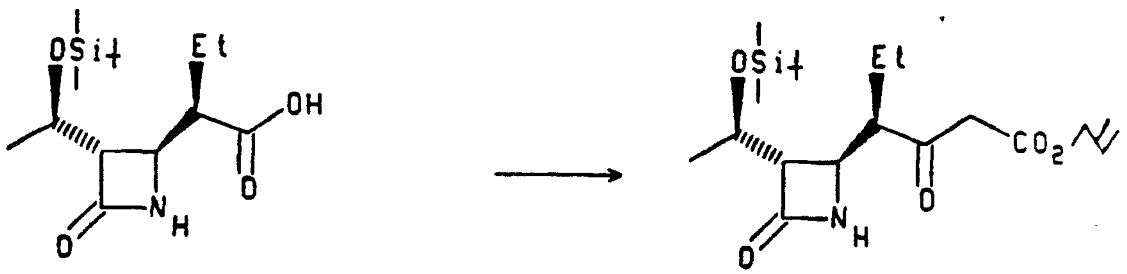

A suspension of 3S,4S-3-[(1R)-1-t-butyldimethylsilyloxyethyl]-4-[1R)-1-carboxypropyl]-azetidin-2-one (4.7 g, 14.9 mmol) in $CH_2CN$ 70 mL) was treated with carbonyl diimidazole (2.66 g, 16.4 mmol) and then stirred for 30 min at ca 22° C allyl acetate (20.0 mmol) was added, and the mixture was then stirred for 20 h at 22° C and at 60° C for 24 h. It was concentrated (30 mL), diluted with EtOAc (100 mL), washed with HCl (1N, 1 x 100 mL), water (2 x 100 mL), aqueous $NaHCO_3$ (1M, 1 x 100 mL), water (2 x 100 mL) and brine. The organic phase was dried ($MgSO_4$) and evaporated to give a residue (5.5 g) that was purified on a silica gel (22.5 g, 25% EtOAc/$CH_2Cl_2$) column. The title material was obtained in 37% yield (2.2 g);
ir ($CH_2Cl_2$)$\nu_{max}$: 3400 (NH), 1750, 1740 and 1715 $cm^{-1}$ (C=O); 'Hmr ($CDCl_3$) $\delta$: 6.0-5.8 (2H, m, NH, allyl), 5.36-5.22 (2H, m, allyl), 5.05 (0.5 H, s, olefinic H), 4.6-4.59 (2H, m, $CH_2$), 4.18-4.13 (1h, M, H-1'), 3.87, 3.86, 3.84, 3.83, 3.82, 3.81, 3.78, 3.77 (1H, 1 dd, J=2, J-8, H-4), 3.5 (1H, s, $CH_2$), 2.95, 2.94, 2.92: 2.92, 2.91, 2.88 (1H, 2m, H3), 2.8-2.6 (0.35 H, m, H-1"), 2.2-2 (0.45, m, H-1"), 1.8-1.4 (2H, m, $CH_2$), 1.16, 1.13, 1.10, 1.07 (3H, 2d, J=6.4, 6.3, $CH_3$), 0.964, 0.946, 0.928, 0.91, 0.89, 0.87 (3H, 2t, J=7.3, 7.2 $CH_3$), 0.84 (9H, s, t-butyl) and 0.04, 0.03 ppm (6H, 2s, dimethyl).

## Procedure 41

(3S,4R)-3-[(1R)-1-t-Butyldimethylsilyloxyethyl]-4-[(1R)-1-ethyl-3-diazo-3-allyloxycarbonyl-2-oxopropyl]-azetidin-2-one

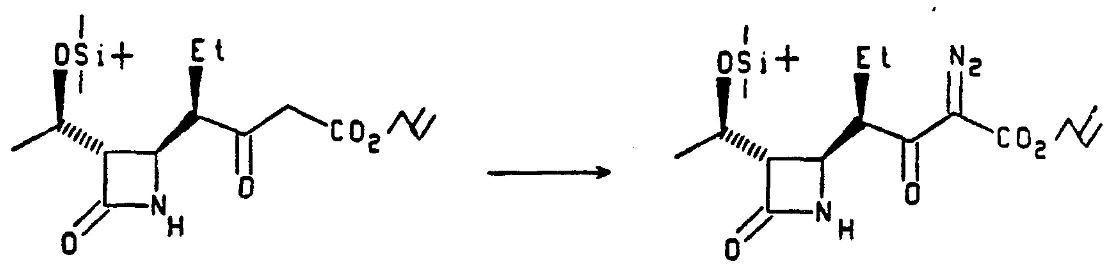

A solution of 3S, 4R-3-[(1R)-1-t-butyldimethylsilyloxyethyl]-4-[(1R)-1-ethyl-3-allyloxycarbonyl-2-oxopropyl]-2-azetidin-2-one (2.2 g, 5.5 mmol) in $CH_3C$ (25 mL) was treated at 5° C with triethyl amine (0.78 mL, 5.5 mmol) and p-toluenesulfonylazide (1.09 g, 5.5 mmol), and stirred at 5° C for 45 min. The ice bath was removed and the mixture was stirred for 30 min more. Evaporation of the solvent left a residue that was triturated with pet-ether. Filtration of the sulfonyl amide left the title material contaiminated with 19% of corresponding $\alpha$-ethyl derivative; (2.3 g, 100%);
ir ($CH_2Cl_2$)$\nu_{max}$: 3400 (NH), 2140 ($N_2$), 1760, 1710 and 1645 $cm^{-1}$ C=O); 'Hmr ($CDCl_3$) $\delta$: 6-5.8 (1H, m, allyl), 5.83 (1H, s, NH), 5.4-5.2 (2H, m, allyl), 4.7-4.6 (2H, m $CH_2$), 4.2-4.0 (1H, m, H-1'), 3.95 (1H, m, H-1;), 3.86 (1H, dd, J=2.0, J=5.5, H-4), 3.05-3.00 (1H, m, H-3), 1.9-1.6 (1H, m, $CH_2$), 1.6-1.3 (1H, m, $CH_2$), 1.6-1.3 (1H, m, $CH_2$), 1.14 (3H, d, J=6.3, $CH_3$), 0.89 (3H, t, J=7.4, $CH_3$), 0.85 (9H, s, t-butyl) and 0.047, 0.041 ppm 6H, 2s, dimethyl.

Procedure 42

(3S,4R)-3-[(1R)-1-Hydroxyethyl]-4-[(1R)-1-ethyl-3-diazo-3-allyloxycarbonyl-2-oxopropyl]-azetidin-2-one

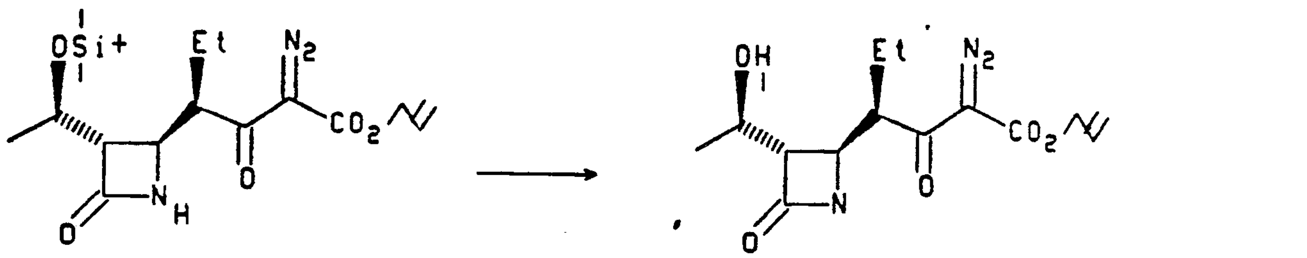

A solution of (3S,4R)-3-[(1R)-1-hydroxyethyl]-4-[(1R)-1-ethyl-3-diazo-3-allyloxycaarbonyl-2-oxopropyl]-azetidin-2-one (2.0 g, 4.7 mmol) in $CH_3CN$ (10 mL) was treated with 1N aqueous HCl (2.36 mL, 2.236 mmol) and stirred for 7 h at ca 22° C. The mixture was diluted with $H_2O$ (30 mL), washed with heptane (3 x 30 mL), and with saturated with NaCl. The aqueous solution was then extracted with EtOAc (3 x 30 mL). The EtOAC extracts were combined, washed with sat. $NaHCO_3$ (1x 50 mL), water (1 x 50 mL), $NH_4Cl_4$ (50 mL) water (1 x 50 mL) and dried ($MgSO_4$). Evaporation of the solvent gave a residue that was purified on a silica gel column (45 g, ethyl acetate) to give title material (1.2 g, 77%, mp 72-75° C);
ir ($CH_2Cl_2$)$\nu_{max}$: 3600, 3500 (OH), 3400 (NH), 2240 ($N_2$), 1760, 1715 and 1635 $cm^{-1}$ (C=O); ′Hmr ($CDCl_3$) δ: 5.97 (1H, s, NH), 6.02-5.82 (1H, m, allyl), 5.39-5.27 (2H, m, allyl), 4.73-4.69 (2H, m, $CH_2$), 4.15-4.05 (1H, m, H-1′), 3.88-3.76 (2H, m, H-4 and H~1″), 2.96, 2.925, 2.920 (1H, dd, Y-3), 2.41 (1H, d, J-3.5, OH), 1.82-56 (2H,m, $CH_2$), 1.28 (3H, d, J=5.8, $CH_3$) and 0.90 (3H, t, J=7.5, $CN_3$).

Procedure 43

Allyl (5S,6S)-4R-Ethyl-6-(1′R-hydroxyethyl)-3,7-dioxo-1-azabicyclo [3.2.0]heptane-2-carboxylate

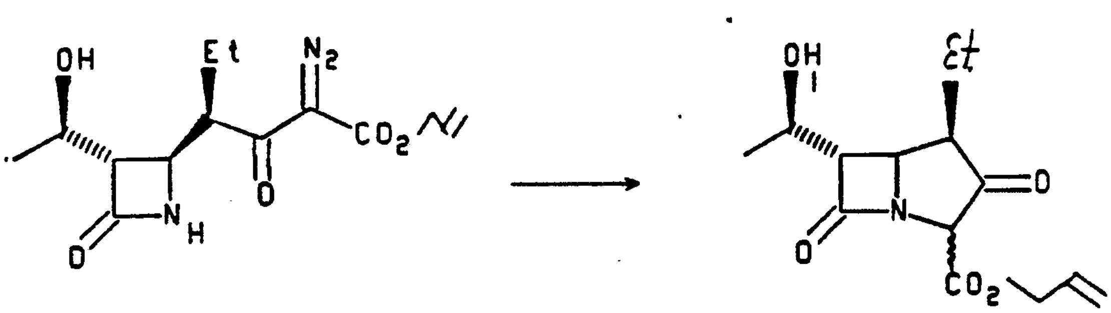

A solution of (3S, 4R-3-[(R)-1-hydroxyethyl]-4-[(1R)-1-ethyl-3-diazo-3-allyloxycarbonyl-2-oxopropyl]-azetidin-2-one (1.1 g, 3.6 mmol) and rhodium octanoate (3 mg) in 75% EtOAc/hexane was heated under reflux for 3 h. Evaporation of solvent gave title derivative as an oil (1.0 g, 100%);
ir ($CH_2Cl_2$)$\nu_{max}$: 3600 (OH), 1765 and 1740 $cm^{-1}$ (C=O); ′Hmr ($CDCl_3$) δ: 60-5.8 (1H, m, allyl), 5.4-5.2 (2H, m, allyl), 5.7-5.6 (2H, m, $CH_2$), 5.62 (1H, s, CH), 4.33 (1H, m, H-1′), 2.8 (1H, dd, J=2.5, J=8.0, H-4), 3.28 (1H, dd, J=2.5,J=7.0, H-6), 2.7-2.5 (1H, m, H-1″), 2.1-1.8 (1H, bs, Oh), 1.8-1.4 (2H, m $CH_2CH_3$), 1.38 (3H, d, J=6.5; $CH_3$), and 1.10 ppm (3H, t, J-7.4, $CH_2CH_3$).

Procedure 44

Allyl (5S,6S)4R-Ethyl-6-[1R-hydroxyethyl]-3-[(propan-2-yl-thio]-7-oxo-1-azabicyclo[3.2.0]hept-2-ene-2-carboxylate

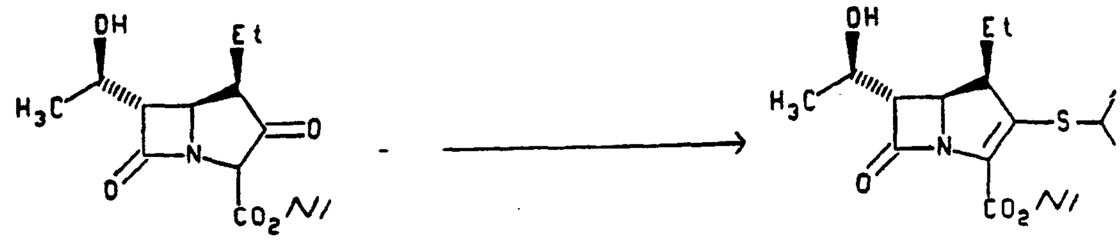

A cold (ice bath) solution of the product of Procedure 43 (1.13 g, 4 mmol) in $CH_3CN$ (30 mL) was treated dropwise with diphenylchlorophosphate (0.91 mL, 4.4 mmol), DIPEA (0.84 Ml, 4.8 mmol) and DMAP (5 mg). The mixture was stirred for 1 h at 22° C, cooled to -10° C and treated with isopropyl mercaptan (0.46 mL, 4.8 mmol) and DIPEA (0.91 mL, 5.2 mmol). The mixture was allowed to stir for 23 h at 0° C, diluted with EtOAc, washed with water (3 x 100 mL) and brine and dried ($MgSO_2$). The residue obtained upon evaporation of solvent (1.7 g) was passed through a silica gel (80 g) column to give title material which was found to be unstable (0.44 g, 32%);
ir ($CH_2Cl_2$)$\nu_{max}$: 3680, 3600 (OH), 1775 and 1710 $cm^{-1}$ (C=O); 'Hmr ($CDCl_{23}$) $\delta$: 6.0-5.7 (1H, m, allyl-H, 5.4-5.1 (2H, m, allyl-H), 4.6 (2H, center of m, $CH_2$), 4.3-4.1 (2H, m, H-1' and H-5), 3.3-3.05 (2H, m, H-6 and H-4), 1.7 3H, m, OH, H-CH, H isopropyl), 1.4-1.1 (11H, m, H-CH, $CH_3$ isopropyl, $CH_3$) and 0.9 ppm (3H, t, $CH_3$).

Procedure 45

Allyl (5S,6S)-(1'S-Azidoethyl)-4R-ethyl-3-[(propan-2-yl)thio]-7-oxo-1-azabicyclo[3.2.0]hept-2-ene-2-carboxylate

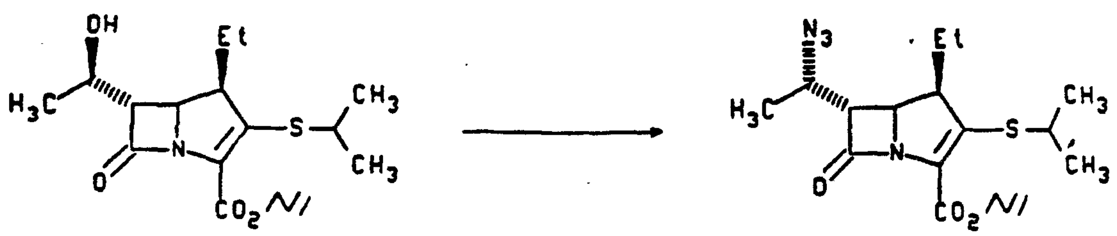

A cold (0° C) THF (45 mL, dry) solution of allyl (4R,5S,6S)-4-ethyl-6-(1'R-hydroxyethyl)-3-[(propan-2-yl)-thio]-7-oxo-1-azabicyclo[3.2.0]hept-2-ene-2-carboxylate (440 mg, 1.3 mmol) was treated first with triphenylphosphine (682 mg, 2.6 mmol), and then dropwise with a solution of $HN_3$ in toluene (4.5 mL, 2.6 mmol), and diethyl azodicarboxylate (0.414 mL, 2.5 mmol). The mixture was stirred for 30 minutes at 0° C, diluted with ethyl acetate (200 mL), washed with $NaHCO_3$ (1M, 2 x 150 mL), brine (1 x 150 Ml) and dried ($MgSO_4$). The residue (~1g) upon evaporation of solvent was purified on silica gel (50 g) with 1:1 hexanes/ethyl acetate to give the title material (370 mg, 78%) as a yellow oil;
IR ($CH_2Cl_2$)$\nu_{max}$: 2120 ($N_3$), 1775 (C=O B-lactam) and 1710 $cm^{-1}$ (C=) ester); 'Hmr (200 MHz, $CDCl_3$) $\delta$:

6.07-5.85, 5.49, 5.40, 5.30, 5.27, 5.22 (3H, m, olefine H), 4.88-4.62 (2H, m, allylic H), 4.15 (1H, dd, J=9.4, J=2.8), 4.00 (1H, center of lines or dq, J=6.7, J=5.6, H-1′), 3.39 (1H, dd, J=2.9, J=5.5, H-6), 3.27 (1H, m, SCH), 3.10 (1H, m, J=9.0, J=2.5, H-4), 1.94-1.73 (1H, m, $CH_2$ of β-ethyl), 1.63-1.4 (1H, m, $CH_2$ of β-ethyl), 1.46 (3H, d, J= =7.1, $CH_3$, 1.41-1.29 (6H, m, $HCCH_3$-$CH_3$), 1.02 (3N, t, J=7.3, $CH_3$ of β-ethyl).

## Procedure 46

(4R,5S,6R)6-(1′S-Aminoethyl)-4-ethyl-3-[(propan-2-yl)thio]-7-oxo-1-azabicylco[3.2.0]hept-2-ene-2-carboxylic Acid (41280)

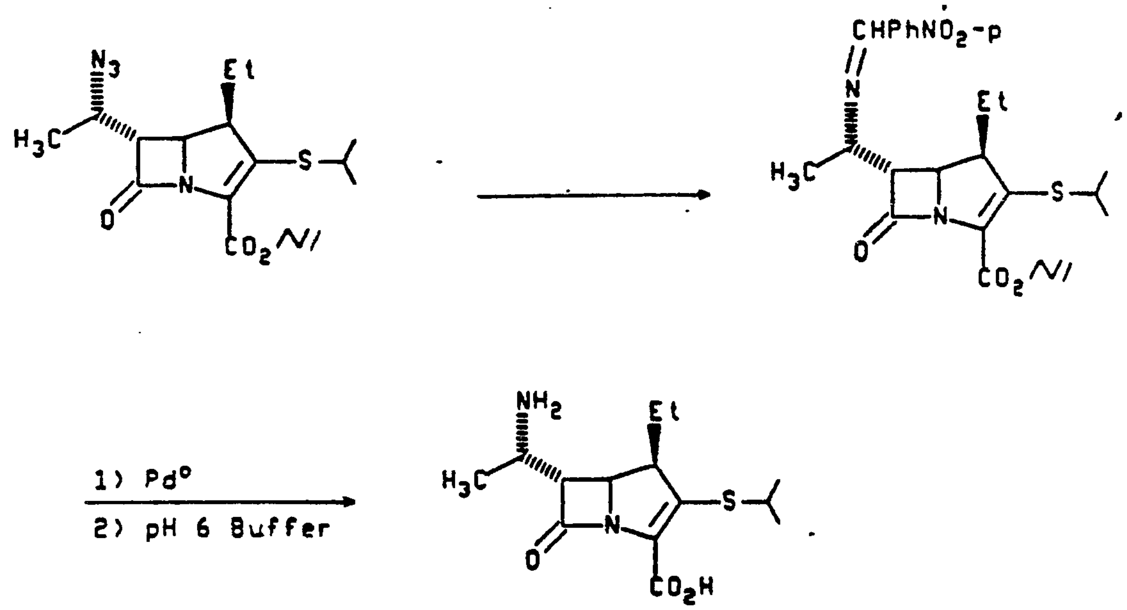


A benzene (10 mL) solution of allyl (4R,5S,6R)-6- (1′S-azidoethyl)-4-ethyl-3-[(propan-2-yl)thio]-8-oxo-1-azabicylco[3.2.0]hept-2-ene-2-carboxylate (370 mg, 1.00 mmol) and triphenylphosphine (370 mg, 1.4 mmol) was heated under reflux for 1 h and allowed to cool to room temperature (22° C). The mixture was then treated with p-nitrobenzaldehyde (160 mg, 1.00 mmol), stirred for 18 h and then concentrated to give allyl (4R,5S,6R)-4-ethyl-3-[propan-2-yl)thio]-6-(1′S-p-nitrobenzylideneaminoethyl)-7-oxo-1-azabicyclo[3.2.0]hept-2-ene-2-carboxylate [0.9 g, 100%);

ir ($CH_2Cl_2$)$\nu_{max}$: 1765 (C=O, β-lactam) and 1710 $cm^{-1}$ (C=0, ester)] which was used as such for the next step.

A methylene chloride (10 mL) solution of the foregoing Schiff base (0.9 g, 1 mmol) at room temperature (ca 22° C) was successively treated with $Pd(PPh_3)_4$ (40 mg) and with a 0.5 M solution of potassium ethyl hexanoate (21.0 mL, 1 mmol) in methyl acetate and then was stirred for 90 min. The mixture was treated with a 0.05 M pH 6 phosphate buffer and stirred for 30 min. More buffer (2 mL) was added and stirring was continued for 30 more minutes. The aqueous layer was collected, concentrated under vacuum and then chromatographed on a μ-Bondapak $C_{18}$ (20 g) column ($H_2O$, 25% $CH_3CN/H_2O$) to give crude title material as a lyophilized powder (50 mg, 17% impure). The crude powder was again purified on a μ-Bondapak $C_{18}$ - (5 g) pad ($H_2O$, 10% $CH_3CN/H_2O$) to give pure title material (35 mg, 12%) on a white powder; uv ($H_2O$, pH 7.4 phosphate buffer)$\lambda_{max}$: 306 (5300);

ir (nujol)$\nu_{max}$: 1760 (C=O, β-lactam) and 1585 $cm^{-1}$ (C=O, ester); ′Hmr (200 MHz, $D_2O$) δ: 4.24 (1H, dd, J=2.4,J=9.0, H-5), 3.91 (1H, center of 5 lines, dq, J=6.8,J=7.4,H-1′), 3.59 (1H, dd, J=2.4, J=8.2, H-6), 3.41-3.2 (2H, m, H-4 and SCH), 1.9-1.7 (1H, m, $CH_2$ of β-ethyl), 1.7 (1H, m, hidden part of $CH_2$ of β-ethyl), 1.47 (3N, d, J=6.7, $CH_3$), 1.29 (6H, center of 2d, J=6.4, J=6.8,$CH_3$) and 0.98 ppm (3H, t, J=7.4, $CH_3$ of β-ethyl); purity 85.95 %, hplc on μ-Bondapak $C_{18}$, 4 mm x 30 cm, elution 10% $CH_3CN$/buffer 7.4, 0.01M,

flow rate 2 mL/min, uv detector 306 nm, retention time 9.98 min.

Procedure 47

Allyl (5S,6S)-6-(1R-Hydroxyethyl)-4R-ethyl-3-methylthio-7-oxo-1-azabicyclo[3.2.0]hept-2-ene-2-carboxylate

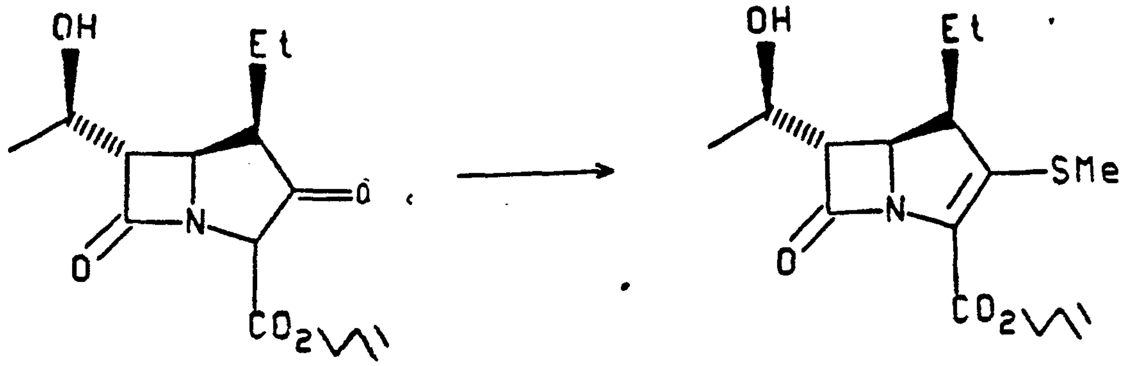

The bicyclic ketone product of Procedure 43, about 5 g., was allowed to react with diphenyl chlorophosphate, DIPEA, and TMS-Cl in a fashion similar to Method B of Procedure 3. The product was recovered by evaporation of the ethyl actetate extract at the conclusion of the reaction to yield an oil, 6.8 g., which was chromatographed on a silica gel (300 g) column ($CH_2Cl_2$ ---- EtOAc) to give title material (2.6 g, 50%);

ir ($CH_2Cl_2$)$\nu_{max}$: 3680, 3600 (OH), 1775 and 1710 $cm^{-1}$ (C=O); ′Hmr ($CDCl_3$) δ:6.04-5.85 (1H, m, allyl-H), 5.47-5.19 (2H, m, allyl-H), 4.85-4.59 (2H, m, $CH_{2Y}$), 4.24 (1H, center of m, H-1′), 4.18 (1H, dd, J=2.6, J=9.4, H-5), 3.20 (1H, dd, J=2.5, J-7.0, H-6), 3.11 (1H, center of m, H-4) 2.34 (3H, s, $SCH_3$), 1.90 (1H, bs, OH), 1.9-1.65 (1H, m, H-CH), 1.61-1.4 (1H, m, H-CH), 1.36 (3H, d, J=6.3, $CH_3$), and 1.01 ppm (3H, t, J=7.3, $CH_3$).

Procedure 48

Allyl (5S,6S)-6-(1′S-azidoethyl)-4R-ethyl-3-methylthio-7-oxo-1-azabicyclo[3.2.0]hept-2-ene-2-carboxylate

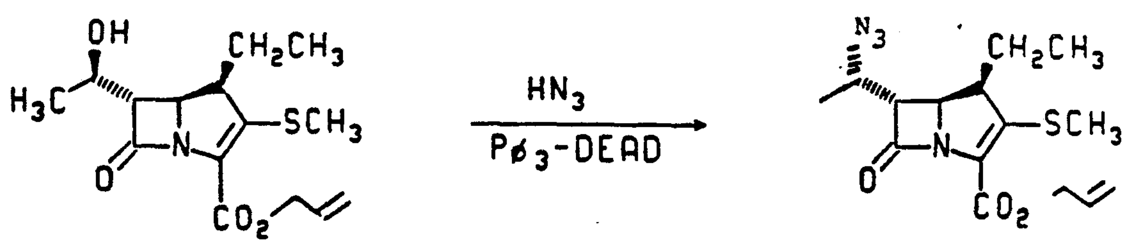


To a cold (0°C) THF (30 mL, dry) solution of allyl (5S,6S)-6-(1′R-hydroxyethyl)-4R-ethyl-3-methylthio-7-oxo-1-azabicyclo[3.2.0]hept-2-ene-2-carboxylate (311 mg, 1 mmol, Procedure 47) was added first triphenylphosphine (525 mg, 2 mmol), then a 0.32 M solution of $HN_3$ in toluene (6.25 mL, 2 mmol, dropwise, and finally diethyl azodicarboxylate (0.32 mL, 1.9 mmol), dropwise. The mixture was stirred for 15 min at 0°C, diluted with ethyl acetate (150 mL), washed with aqeous $NaHCO_3$ (1M, 2x 100 mL), washed with aqeous $NaHCO_3$ (1M, 2x100 mL), brine (1 x 100 mL) and dried ($MgSO_4$). The residue (1.3 g) upon evaporation of solvent was purified on a flash silica gel column (659 g, 1/1: hexane/ethylacetate) to give title material (275 mg, 82%) as a yellow oil;

ir ($CH_2Cl_2$)$\nu_{max}$: 2120 (N3), 1775 (C=o β-lactam) and 1705 $cm^{-1}$ (C=O ester); ′Hmr (200 MHz, $CDCl_3$) δ: 6.02-5.88, 5.47, 5.38, 5.26, 5.21 (3H, M, olefinic H), 4.85-4.61 (2H, m, allylic H), 4.12 (1H, dd, J=9.2, J=2.7, H-5), 3.98 (1H, center of 6 lines or dq, J=6.6, J=5.7, H-1′), 3.37 (1H, dd, J=2.8, J=5.5, H-6), 3.10 (1H, m, J=2.4, J=9.2, H-1), 2.35 (3H, s, $SCH_3$), 1.89-1.74 (1H, m, $CH_2$ of β-ethyl), 1.62-1.4 (1H, m, $CH_2$ of β-ethyl), 1.46 (3H, d, J=6.7, $CH_3$) and 1.0 ppm (3H, t, J=7.4, $CH_3$ of β-ethyl).

## Procedure 49

(5S,6R) 6-(1′S-Aminoethyl)-4R-ethyl-3-methylthio-7-oxo-1-azabicyclo[3.2.0]hept-2-ene-2-carboxylic Acid - (40767)

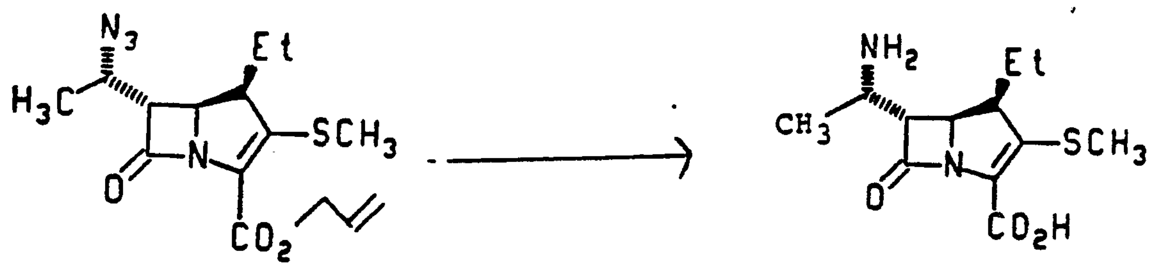

A benzene (4mL) solution of allyl (5S,6S) 6-(1′S-azidoethyl)-4R-ethyl-3-methylthio-7-oxo-1-azabicyclo-[3.2.0]hept-2-ene-2-carboxylate (170 mg, 0.5 mmol) from Procedure 48 and triphenylphosphine was treated under reflux for 1 h and allowed to cool to room temperature. The mixture was then treated with p-nitrobenzaldehyde (80 mg, 0.53 mmol), stirred for 6 h and then the solvent evaporated to give the Schiff base (450 mg, 100%) as an oil which was used as such for the next step:
ir (neat)$\nu_{max}$: 1770 (C=O, β-lactam) and 1705 $cm^{-1}$ C=O, ester); ′Hmr (200 MHz, $CDCl_3$) δ: 8.40-7.90 (5H, m, aromatic H and CH=N), 6.1-5.9, 5.47-5.45, 5.38-5.37 and 5.26-5.19 (3H, m, olefinic-H), 4.9-4.6 (2H, m, allylic H), 4.18 (1H, dd, J=2.7, J=9.3, H-5), 3.92 (1H, center of 5 lines, Jj=6.5, H=1′), 3.47 (1H, dd, J=2.7, J-6.8, H-6), 3.12 1H, m, H-4), 2.34 (3H, s, $SCH_3$), 1.9-1,4 (2H, complex AB, $CH_2$ of β-ethyl), 1.42 (3H, d, J=6.4, $CH_3$) and 0.99 (3H, t, J=7.3, $CH_3$ of β-ethyl.

A cold (ice bath) methylene chloride (4 mL) solution of the Schiff base (450 mg, 0.5 mmol) was successively treated with tetrakis (triphenylphosphine Pd (0) (20 mg) and with a 0.5M solution of potassium ethylhexanoate (1 mL, 0.5 mmol) in ethyl acetate and stirred for 1 h. The mixture was treated with a 0.05M pH6 buffer (4 mL), stirred for 90 min at ca 22° C, then treated with more pH6 buffer (4 mL) and stirred (30 min). The aqueous solution was collected and passed through a reversed phase $C_{18}$ column (20 g, $H_2O$-10% $CH_3CN/H_2O$) to give the title material. The lyophilized powder was then purified through a reversed phase $C_{18}$ column (20 g, $H_2O$-5% $CH_3CN/H_2O$) to give pure title material (40 mg, 30%) as a white powder; ir (Nujol)$\nu_{max}$: 1750 (C=O, β-lactam) and 1585 $cm^{-1}$ (C=O carboxylate); ′Hmr (200 MHz, $D_2O$) δ: 4.18 (1H, dd, J-2.4, J-8.9, H-5), 3.83 (1H, dd, J=6.8, J=8, H-1′), 3.53 (1H, dd, J=2.4, J=8.1, H-6), 3.32 (1H, m, J=2.4, J-8.9, H-4), 2.35 (3H, s, $SCH_3$), 1.91-1.72, 1.57-2.6 (2H, m, $CH_2$ of β-ethyl), 1.43 (3H, d, J=6.7, $CH_3$) and 0.98 ppm (3H, t, J=7.4, $CH_3$ of β-ethyl); uv ($H_2O$, pH 7.4 phosphate buffer) $\lambda_{max}$: 306 (10453: purity 98.9%, hplc on μ-Bondapak $C_{18}$, 4 mm x 30 cm, elution 2% $CH_3CN$/buffer pH 7.0, 0.01M, flow rate 2 mL/min, uv detector 306 nm.

| M.I.C. | |
|---|---|
| Str. pneu. A9585 | 0.250 |
| Str. pyo. A9604 | 1.000 |
| Str. faec. A20688 | 125.000 |
| Staph. aur. A9537 | 2.000 |
| Staph. serum | 63.000 |
| Staph aur. Pen + | 8.000 |
| Staph aur. MR | 125.000 |
| E. coli A15119 | 2.000 |
| E. coli A20341 | 2.000 |
| K. pneu. A9664 | 2.000 |
| K. pneu. A20468 | 2.000 |
| Ent. clo. A9659 | 2.000 |
| Ent. clo. A9656 | 2.000 |
| P. mir. A9900 | 0.500 |
| P. vul. A21559 | 0.500 |
| P. morg. A15153 | 2.000 |
| P. rett. A22424 | 32.000 |
| Ser. mars.A20019 | 2.000 |
| Ps. aeru. A9843 | 8.000 |
| Ps. aeru. CarbR | 8.000 |
| t1/2, pH 7.4 (h.): | 8 |
| t1/2, pH 2 (min.): | 154 |

## Procedure 50

Allyl (5S-6S)-4R-Ethyl-6-(1R-hydroxyethyl)-3-[(1-methyl-1,2,3-triazol-4-yl)methylthio]-7-oxo-1-azabicyclo-[3.2.0]hept-2-ene-2-carboxylate

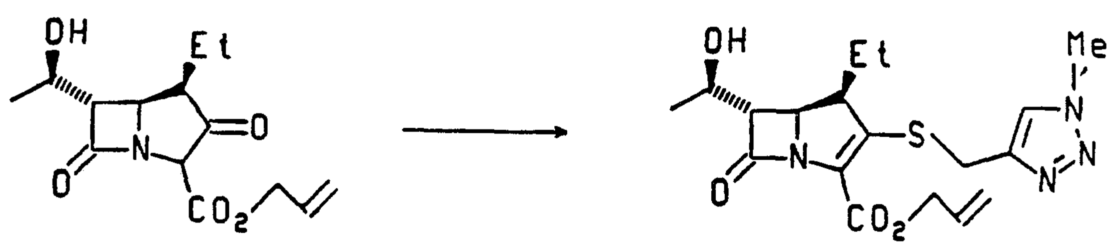

A cold (ice bath) solution of allyl (5S,6S)-4R-ethyl-6-(1R-hydroxyethyl)-3,7-dioxo-1-azabicycl[3.2.0]hept-2-ene-2-carboxylate (1.17 g, 4 mmol, Procedure 43) in $CH_3CN$(20 mL) was treated with diphenylchlorophosphate (0.84 mL, 4.8 mmol), DIPEA (0.91 mL, 4.4 mmol) and DMAP (5 mg). The ice bath was removed and the mixture was stirred for 1 h. It was then cooled in an ice-MeOH bath and treated with (1-methyl-1,2,3-triazol-4-yl)methanethiol (620 mg, 4.8 mmol) and DIPEA (0.91 mL, 5.2 mmol). The bath was removed and the mixture was stirred for 4 h after which it was diluted with ethyl acetate (100 mL), washed with water (3 x 50 mL), brine (1 x 50 mL) and dried ($MgSO_4$). The residue, (2.5 g) upon solvent evaporation, was passed through a silica gel (150 g) column to give title material (0.53 g, 34%, 1:1 hexane-EtOAc, EtOAc);

ir ($CH_2Cl_2$)$\nu_{max}$: 3680, 3600 (OH), 1775 and 1710 $cm^{-1}$ (C=O); 'Hmr ($CDcl_3$) $\delta$: 7.48 (1H, s, aromatic-H), 6.0-5.8 (1H, m, allyl-H), 5.5-5.2 (2H, m, allyl-H), 4.8-4.6 (2H, m, $CH_2$), 4.2-4.07(3H, part of ABq, H-', H-5),

4.07 (3H, s, N-Me), 3.97, 3.9 (1h, part of ABq, J = 14.9, S-$CH_2$) 3.42 (1H, m, H-4), 2.77 (1H, dd, J = 2.6, J = 6.8, H-6), 1.95-1.7 (2H, m, OH and H-CH), 1.65-1.4 (1H, m, HC-H), 1.35 (3H, d, J = 6.3, $CH_3$) and 1.05 ppm (3H, t, J = 7.4, $CH_3$).

Procedure 51

Allyl (5S,6S) 6-(1′S-Azidoethyl)-4R-ethyl-3-[(1-methyl-1,2,3-triazol-4-yl)methylthio]-7-oxo-1-azabicyclo[3.2.0]-hept-2-ene-2-carboxylate

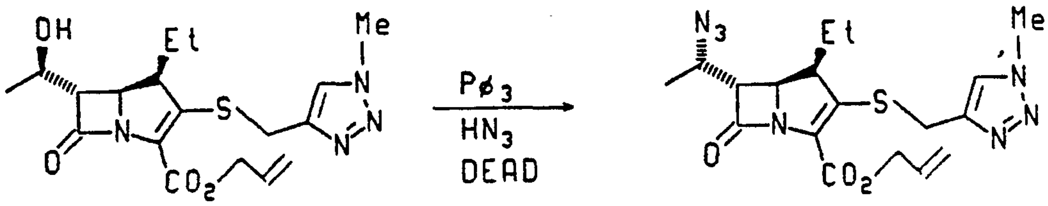


To a cold (ice bath) THF (60 mL) solution of allyl (5S,6S)-6-(1′-R-hydroxyethyl)-4R-ethyl-3-[(1-methyk-1,2,3-triazol-4-yl)methylthio]-7-oxo-1-azabicyclo[3.2.0]hept-2-ene-2-carboxylate (530 mg, 1.3 mmol, Procedure 50) was added successively triphenylphosphine (710 mg, 2.7 mmol), a 0.39M solution of $HN_3$ in toluene (6.9 mL, 2.7 mmol) and diethyl azodicarboxylate (425 µl, 2.6 mmol). The mixture was stirred for 15 min at 0° C, diluted with ethyl acetate (150 mL), washed with aqueous $NaHCO_3$ (1M, 2 x 100 mL), brine (1 x 100 mL) and dried ($MgSO_4$). The residue (2.1 g) upon solvent evaporation was purified through a flash silica gel (80 g) column (1/1: hexane/ethyl acetate) to give title material as an oil (920 mg, 89%);
ir ($CH_2Cl_2$)$\nu_{max}$: 2120 ($N_3$), 1780 (C=O, β-lactam) and 1720 $cm^{-1}$ (C=O, ester); Hmr (200 MHz, $CDCl_3$) δ: 6.1-5.85, 5.5-5.2 (3H, m, vinylic-H), 4.9-4.6 (2H, m, allylic-H), 4.25, 4.18 (1H, 2 lines of AB, J = 14.9, $SCH_2$), 4.1-4.0 (1H, hidden m, H-5), 4.08 (3H, s, N-$CH_3$), 3.96, 3.88 (1H, 2 lines of ABq, J = 15, $SCH_2$) 3.96 (1H, hidden m, H-1′), 3.46 (1H, center of m, J = 9.5, J = 2.5, H-4), 3.35 (1H, dd, J = 2.8, J = 5.4, H-6), 2.0-1.8 (1H, m, $CH_2$ of β-ethyl), 1.7-1.4 (1H, hidden m, $CH_2$ of β-ethyl), 1.46 (3H, d, J = 6.7, $CH_3$) and 1.03 ppm (3H, t, J = 7.3, $CH_3$ of β-ethyl).

Procedure 52

(5S,6R) 6-(1′S-Aminoethyl)-4R-ethyl-3-[(1-metyl-1,2,3-triazol-4-yl)-methylthio]-7-oxo-1-azabicyclo[3.2.0]hept-2-ene-2-carboxylate (41282)

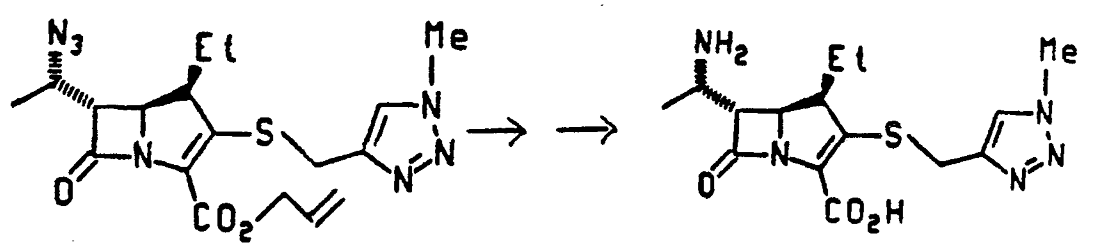

A toluene (10 mL) solution of allyl (5S,6S) 6-(1′S-azidoethyl)-4R-ethyl-3-[(1-methyl-1,2,3-triazol-4-yl)-methylthio]-7-oxo-1-azabicyclo[3.2.0]hept-2-ene-2-carboxylate (920 mg, 2.2 mmol, Procedure 51) and

triphenyl phosphine (920 mg, 3.5 mmol) vas heated under reflux for 1 h and allowed to cool to room temperature. The mixture was then treated with p-nitrobenzaldehyde (290 mg, 2.2 mmol), stirred for 6 h and evaporated to give the intermediate Schiff base (1.6 g, 100%) which was used as such for the next step; ir ($CH_2Cl_2$)$\nu_{max}$: 1775 (C=O, β-lactam) and 1710 $cm^{-1}$ (C=O, ester); ′Hmr (200 MHz, $CDCl_3$) δ: 8.45 (1H, s, CH=N), 8.3, 8.25, 7.95, 7.9 (2H, 2d, aromatic-H), 6.06-5.87 (1H, m, vinylic-H), 5.48, 5.39, 5.27, 5.22 (2H, m, vinylic H), 4.86-4.61 (2H, m, allylic-H), 4.26, 4.18 (1H, part of ABq, J=15, $SCH_2$), 4.11 (1H, dd, J=2.8, J=9.4, H-5), 4.07 (3H, s, N-$CH_3$), 3.95, 3.87 (1H, part of ABq, J=15, $SCH_2$), 3.89 (1H, center of 5 lines, J=6.3, H-1′), 3.55-3.43 (2H, m, H-4 and H-6), 2.1-1.8 (1H, m, $CH_2$ of β-ethyl), 1.7-1.4 (1H, m, $CH_2$ of β-ethyl), 1.7-1.4 (1H, m, $CH_2$ of β-ethyL), 1.41 (3H, d, J=6.4, $CH_3$) and 1.02 ppm (3H, t, J=7.3, $CH_3$ of β-ethyl.

A methylene chloride (10 mL) solution of the Schiff base (1.6 g, 1.2 mmol) was successively treated with tetrakis triphenylphosphine palladium (40 mg) and with a 0.5M solution of potassium ethylhexanoate (2.6 mL, 1.3 mmol) in ethyl acetate and was stirred for 30 min at 22°C. The mixture was treated with a 0.05 M pH6 phosphate buffer (10 mL), stirred for 90 min. The aqueous solution was collected, concentrated in vacuo and passed through a μ-Bondapak $C_{18}$ 930 g) column ($H_2O$, $C_{H3}CN/H_2$)) to give impure title material (230 mg, 55%) as a powder after lyophilization. The crude powder was passed again through a Bondapak $C_{18}$ (12 g) column to give pure title material (180 mg, 43%) as a white powder; uv ($H_2O$, pH 7.4 phosphate buffer) $\lambda_{max}$: 304 (7775);
ir (Nujol)$\nu_{max}$: 1755 (C=O, b-lactam) and 1585 $cm^{-1}$ (C=O, ester); ′Hmr (200 MHz, $D_2$)) δ: 7.87 (1H, s, triazole-H), 4.21, 4.13 (1H, part of ABq, J=15, $SCH_2$), 4.12 (part of dd, H-5), 4.06 (2H, s, $NCH_3$), 4.06 (hidden part of dd, H-5), 4.04, 3.96 (1H, part of ABq, J=15, $ScH_2$), 3.84 (1H center of 5 lines, J=6.7, J-8.1, H-1′), 3.55 (1H, dd, J=2.3, J=7.1, H_6, 3.22 (1H, m, H-4), 1.9-1.7 (1H, m, $CH_2$ of β-ethyl), 1.6-1.3 (1H, m, $CH_2$ of β-ethyl), 1.42 (3H, d, J=6.6, $CH_3$ and 0.92 ppm (3H, t, J=7.2, $CH_3$ of β-ethyl); purity 99.93%, hplc on μ-Bondapak C-18, 4 mm x 30 cm, elution 9% $CH_3CN$-$H_2O$, pH 7.4 phosphate buffer, flow rate 1 mL/min uv detector 304 nm, retention time 7.5 min.

| M.I.C. | |
|---|---|
| Str. pneu. A9585 | 8.000 |
| Str. pyo. A9604 | 16.000 |
| Str. faec. A20688 | 125.000 |
| Staph. aur. A9537 | 32.000 |
| Staph. serum | 63.000 |
| Staph. aur. Pen+ | 125.000 |
| Staph. aur. MR | 125.000 |
| E. coli A15119 | 0.500 |
| E. coli A20341 | 0.500 |
| K. pneu. A9664 | 1.000 |
| K. pneu. A20468 | 2.000 |
| Ent. clo. A9659 | 2.000 |
| Ent. clo. A9656 | 1.000 |
| P. mir. A9900 | 0.250 |
| P. vul. A21559 | 0.250 |
| P. morg. A15153 | 0.500 |
| P. rett. A22424 | 4.000 |
| Ser. mars. A20019 | 1.000 |
| Ps. aeur. A9843 | 32.000 |
| Ps. aeru. CarbR | 0.000 |
| t1/2, pH 7.4 (h.): | |
| t1/2, pH 2 (min.): | |

Procedure 53

(5R,6S) Allyl 6-(1′-R-Azidoethy)-3H-4R-methyl-7-oxo-1-azabicyclo[3.2.0]hept-2-ene-2-carboxylate

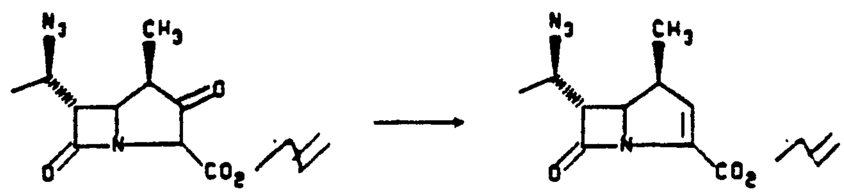

A solution of (5R, 6S) allyl (1′R-azidoethy)-4R-methyl-3,7-dioxo-1-azabicyclo[3.2.0]hept-2-carboxylate (3.42 g, 11.7 mmol, procedure 28) in THF (36 mL) and MeOH (4 mL) was cooled to -25°C and treated, in one portion, with $NaBH_4$ (487 mg, 12.88 mmol). After stirring for 15 min at -25°C the reaction mixture was diluted with EtOAc and washed with cold $H_2O$, cold dilute HCl and finally brine. The organic phase was dried ($MgSO_4$) and evaporated. The resulting crude alcohol was dissolved in $CH_2Cl_2$ (50 mL) and, after cooling in ice, treated with MsCl (2.68 g, 23.4 mmol) and dropwise with TEA (2.37 g, 23.4 mmol). After stirring for 15 min, the solution was washed twice with cold salted $H_2O$ and then dried ($MgSO_4$). The drying agent was removed by filtration, and after cooling in ice, DBN (1.45 g, 11.7 mmol) was added to the filtrate. Elimination of the mesylate took place in 15 min. Then the reaction mixture was washed with cold diluted HCl, brine and finally dried ($MgSO_4$). After evaporation of the solvent, the crude carbapenem was purified by column chromatography ($SiO_2$, 5% $CH_3CN$ in $CH_2Cl_2$).

Y: 1.65 g (51.0% overall).

′Hmr ($CDCl_3$) δ: 6.41 (1H, d, J:2.86, H-3), 6.05-4.60 (5H, allylic pattern), 4.22 (1H, dd, J:3.12, J:10.03, H-5), 3.91 (1H, dq, J:9.67, J:6.56, H-1′), 3.31-3.17 [2H, (dd, J:3.12, J:9.67, H-6), (ddq, J:7.39, J:2.86, J:10.03, H-4)-], 1.48 (3H, d, J:6.56, 1′-$CH_3$), 1.14 (3H, d; J:7.39, 4-$CH_3$).

## Procedure 54

(5R,6R) 6-(1′-R-Aminoethyl)-3H-4R-methyl-7-oxo-1-azabicyclo[3.2.0]hept-2-ene-2-carboxylic Acid (42425)

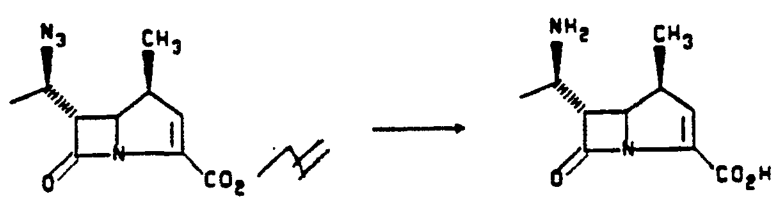

A solution of (5R, 6S) allyl (1′R-azidoethyl)-3H-4R-methyl-7-oxo-1-azabicyclo[3.2.0]hept-2-ene-2-carboxylate (1.65 g, 5.97 mmol, procedure 52) and $PØ_3$ (1.64 g, 6.27 mmol) in benzene (30 mL) was refluxed for 1 h. After cooling to R.T. the reaction mixture was treated with p-nitrobenzaldehyde (0.9 g, 5.97 mmol), stirred for 16 h and finally refluxed for 1 h to form the Schiff base. Benzene was evaporated and replaced by $CH_2Cl_2$ (50 mL). To this solution was added at R.T. $Pd(PØ_3)_4$ (150 mg) and dropwise N-methylaniline (1.28 g, 11.94 mmol). Deprotection was completed in 30 min. A 0.2M buffer solution (pH: 6.0, 25 mL) was added with vigorous stirring to the Schiff base solution. After 1 h, the organic phase was separated, the aqueous solution extracted with $CH_2Cl_2$ and then chromatographed on reversed phase $SiO_2$ eluting with $H_2O$.

Y: 600 mg (47.8%).

ir (Nujol) $\nu_{max}$:1757 (β-lactam), 1575 ($CO_2$-)

′HMr ($D_2O$) δ:6.26 (1H, d, J:2.63, H-3), 4.28 (1H, dd, J:2.44, J:9.24, H-5), 3.88 (1H, dq, J:8.40, J:6.61, H-1′), 3.63 (1H, dd, J:2.44, J:8.40, H-6), 3.26 (1H, ddq, J:2.63, J:7.33, J:9.24, H-4), 1.49 (3H, d, J:6.61, 1′-$CH_3$), 1.10 (3H, d, J:7.33, 4-$CH_3$)

uv $\lambda_{max}$:262 (4053).

Procedure 55

(5R,6S) Allyl 6-(1′S-Azidoethyl)-3H-4R-methyl-7-oxo-1-azabicyclo[3.2.0]hept-2-ene-2-carboxylate

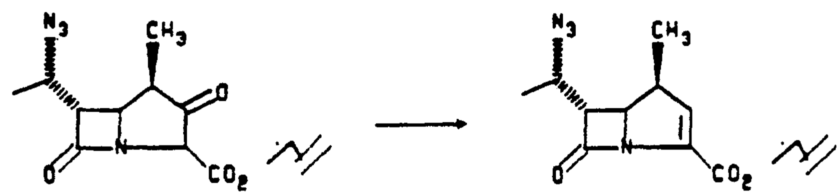

A solution of (5R,6S) allyl (1′S-azidoethyl)-4R-methyl-3,7-dioxo-1-azabicyclo[3.2.0]hept-2-carboxylate (3.88 g, 13.27 mmol, Procedure 31) in THF (36 mL) and MeOH (4 mL) was cooled to -25° C and treated, in one portion, with $NaBH_4$ (552 mg, 14.6 mmol). After stirring for 15 min at -25° C, the reaction mixture was diluted with EtOAc and washed with cold $H_2O$, cold dilute HCl and finally brine. The organic phase was dried ($MgSO_4$) and evaporated. The resulting crude alcohol was dissolved in $CH_2Cl_2$ (50 mL) and, after cooling in ice, treated with MsCl (1.89 g, 16.5 mmol) and dropwise with TEA (1.67 g, 16.5 mmol). After stirring for 60 min, the solution was washed with $H_2O$ and dried ($MgSO_4$). The drying agent was removed by filtration and after cooling in ice, DBN (1.49 g, 12 mmol) was added to the filtrate. Elimination of the mesylate took place in 60 min. Then the reaction mixture was washed with cold diluted HCl, brine and finally dried ($MgSO_4$). After evaporation of the solvent, the crude carbapenem was purified by column chromatography ($SiO_2$, 5% $CH_3CN$ in $CH_2Cl_2$).

Y: 2.09 g (57.1%).

ir ($CH_2Cl_2$) $\nu_{max}$:2120 $cm^{-1}$ ($-N_3$), 1781 ($\beta$-lactam), 1730 (ester).

′Hmr ($CDCl_3$) $\delta$:6.40 (1H, d, J:2.68, H-3), 6.05-4.60 (5H, allylic pattern), 4.23 (1H, dd, J:3.28, J:10.07, H-5), 3.97 (1H, dq, J:5.22, J:6.68, H-1′), 3.48 (1H, dd, J:3.28, J:5.22, H-6), 3.20 (1H, ddq, J:2.68, J:10.07, J:7.36, H-4), 1.45 (3H, d, J:6.68, 1′-$CH_3$), 1.14 (3H, d, J:7.36, 4-$CH_3$).

The material obtained as described in Procedure 54 is contaminated by about 30% of the corresponding 4S-methyl-carbapenem.

Procedure 56

(5R, 6R) 6-(1′S-Aminoethyl)-3H-4R-methyl-7-oxo-1-azabicyclo[3.2.0]hept-2-ene-2-carboxylic Acid (42426)

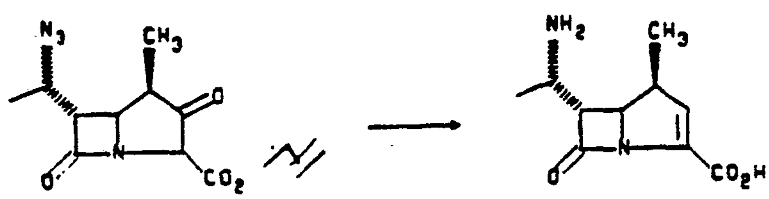

A solution of (5R,6S) allyl 6-(1′S-azidoethyl)-3H-4R-methyl-7-oxo-1-azabicyclo[3.2.0]hept-2-ene-2-carboxylate (2.07 g, 7.5 mmol, Procedure 55) and PØ₃ (2.07 g, 7.88 mmol) in benzene (35 mL) was refluxed for 1 h. After cooling to room temperature, the reaction mixture was heated with p-nitrobenzaldehyde (1.13 g, 7.5 mmol), stirred at room temperature for 20 h and then evaporated to dryness. The residual Schiff base was dissolved in $CH_2Cl_2$ (50 mL) and treated with $Pd(PØ_3)_4$ (150 mg) followed by the dropwise addition of N-methylaniline (1.61 g, 15 mmol). After stirring at room temperature for 1 h, a 0.2M buffer solution (pH:6.0, 25 mL) was added and a vigorous stirring maintained for 2 h. Then the organic phase was separated, the aqueous solution was extracted with $CH_2Cl_2$ and finally chromatographed on reversed phase silica gel

($H_2O$). Only the fractions containing the pure 4-R methyl carbapenem were saved.
Y: 36 mg (2.3%).
ir (Nujol) $\nu_{max}$:1755 ($\beta$-lactam), 1585 ($CO_2$-).
'Hmr ($D_2O$) $\delta$:6.26 (1H, unresolved d, H-3), 4.29 (1H, unresolved dd, H-5), 3.89 (1H, unresolved dq, H-1'), 3.65 (1H, unresolved dd, H-6), 3.28 (1H, unresolved ddq, H-4), 1.44 (3H, d, J:6.20, 1'-$CH_3$), 1.11 (3H, d, J:7.20, 4-$CH_3$).
uv: $\lambda_{max}$:262 (2428).

## Procedure 57

(5S,6R) 6-(1S-Aminoethyl)-4R-methyl-3-methylthio-7-oxo-1-1-azabicyclo[3.2.0]hept-2-ene-2-carboxylate - (40383)

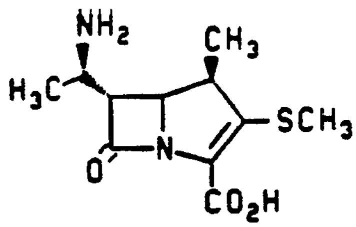

This compound may be prepared from 3S-(1R-hydroxyethyl)-4R-(1R-methyl-3-allyloxycarbonyl-2-oxo-3-diazopropyl)azetidin-2-one by applying thereto the methods of Procedures 2, 3, 8, and 9. Crystallization from water gave the pure product as white prisms: hplc on $\mu$-Bondapak c-18, 4 mm x 30 cm, 2% $CH_3CN$-$H_2O$ pH 7.4 phosphate buffer, flow rate 1 mL/min, uv detector at 304 nm, retention time 10.1 min, purity 99%; uv ($H_2O$, pH 7.4 phosphate buffer) $\lambda_{max}$: 304 nm (11,840);
ir (KBr) $\nu_{max}$: 1748 (C=O of $\beta$-lactam), 1645, 1572 and 1550 $cm^{-1}$; $^1$Hmr ($D_2O$) $\delta$: 1.21 (d, J=7.25, 3H, $CH_3$-4), 1.42 (d, J= 6.70, 3H,$CH_3CHNH_2$), 2.36 (s, 3H, $SCH_3$-3), 3.51 (m, 1H, H-4), 3.59 (dd, $J_{H6,H5}$ = 2.30, $J_{H6,H1'}$ = 7.56, 1H, H-6), 3.87 (m,1H,H-1'), 4.18 ppm (dd, $J_{H5,H6}$ = 2.30, $J_{H5,H4}$ = 8.69, 1H, H-5).

By uv, this carbapenem was found to have a half-life of 4.5 h at 37°C in a pH 7.4 phosphate buffer. At 22°C in the same conditions, the half-life increases to 21 h. By hplc in a pH 2.0 citric acid buffer at 37°C, the half-life was evaluated to 36 min.

| M.I.C. | | |
|---|---|---|
| Str. pneu. A9585 | 4.000 | |
| Str. pyo. A9604 | 4.000 | |
| Str. faec. A20688 | 125.000 | |
| Staph. aur. A9537 | 8.000 | |
| Staph. serum | 16.000 | |
| Staph. aur. Pen+ | 16.000 | |
| Staph. aur. MR | 125.000 | |
| E. coli A15119 | 0.130 | |
| E. coli A20341 | 0.130 | |
| K. pneu. A9664 | 0.500 | |
| K. pneu. A20468 | 0.250 | |
| Ent. clo. A9659 | 0.500 | |
| Ent. clo. A9656 | 0.500 | |
| P. mir. A9900 | 0.060 | |
| P. vul. A21559 | 0.060 | |
| P. morg. A15153 | 0.250 | |
| P. rett. A22424 | 4.000 | |
| Ser. mars. A20019 | 0.250 | |
| Ps. aeru. A9843 | 0.500 | |
| Ps. aeru. CarbR | 1.000 | |
| Ps. aeru. Range: | 1-16 | |
| MIC 50 | 0.9 | |
| MIC 90 | 2.0 | |
| PD50, | IM | PO |
| S. aur. | | |
| E. coli | 12.00 | 25.00 |
| t1/2, pH 7.4 (h.): | | 4.5 |
| t1/2, pH 2 (min.): | | 36 |
| DHP: | | 19.20 |
| Cmax, PO/50: | | 5 |

Procedure 58

(5S,6S)-6-[1′(S)-Aminoethyl)-(4R)-methyl-3-[pyridin-3-yl)methylthio]-7-oxo-1-azabicyclo[3.2.0]-hept-2-ene-2-carboxylic Acid (40732)

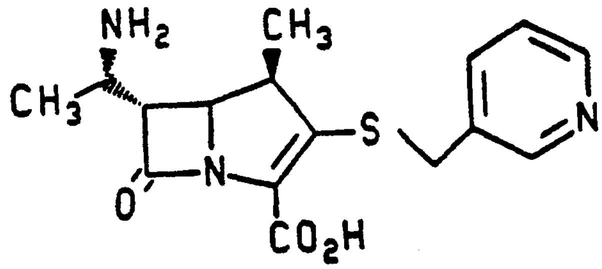

This product is the 1′S-isomer of the product of Procedure 21, and may be prepared in similar fashion

to Procedures 19, 20 and 21 using allyl (5S,6S) 6-(1′R-hydroxyethyl)-4R-methyl-3,7-dioxo-1-azabicyclo-[3.2.0]hept-ane-2-carboxylate as starting material. The crude title compound was passed through a column (3.5 x 8.5 cm) of reverse phase silica (μ-Bondapak C-18), and eluted with a mixture of $H_2O$ and $CH_3CN$ (9:1). There was obtained a white fluffy solid after lyophilization of the eluate; purity 98.99% by HPLC (u.v. detection at 304 nm) on μ-Bondapak C-18 (4 mm x 30 cm); 15% $CH_3CN$ in pH 7.4 phosphate buffer; flow rate 1 mL/min; retention time: 4.46 min. uv (pH 7.4): $\lambda_{max}$266 (5461), 304 (8011).
ir (nujoL): $\nu_{max}$1760 $cm^{-1}$ (C=O, β-lactam). $t_{1/2}$(37 °C); pH 7.4 = 1.7 h; pH 2.0 = 31 min.
′H-nmr (200 MHz, $D_2O$) δ: 1.17 (d, $CH_3$-4), 1.39 (d, $CH_3$), 3.36 (m, H-4, $J_{4,5}$=8.05 Hz, $J_{4,CH3}$=7.22 Hz), 3.58 (dd, H-6, $J_{5,6}$=2.41 Hz, $J_{6,1'}$=7.6 Hz), 3.83 (m, H-1′, $J_{1'}$, CH3= 6.67 Hz), 4.04, 4.19 (AB, $SCH_2$, $J_{A,B}$ = 14.07 Hz), 4.07 (dd, H-5), 7.44 (dd, H-5, Py, $J_{4\text{-}5}$ = 7.93 Hz, $J_{5,6}$ = 3.85 Hz), 7.89 (d, H-4, Py),8.43 (d, H-6, Py), 8.52 (d, H-2, py, $J_{meta}$ = 1.71 Hz).

| M.I.C. | | |
|---|---|---|
| Str. pneu. A9585 | 2.000 | |
| Str. pyo. A9604 | 0.250 | |
| Str. faec. A20688 | 2.000 | |
| Staph. aur. A9537 | 0.500 | |
| Staph. serum | 4.000 | |
| Staph. aur. Pen+ | 2.000 | |
| Staph. aur MR | 8.000 | |
| E. coli A15119 | 0.030 | |
| E. coli A20341 | 0.030 | |
| K. pneu. A9664 | 0.060 | |
| K. pneu. A20468 | 0.130 | |
| Ent. clo. A9659 | 0.060 | |
| Ent. clo. A9656 | 0.130 | |
| P. mir. A9900 | 0.016 | |
| P. vul. A21559 | 0.030 | |
| P. morg. A15153 | 0.030 | |
| P. rett. A22424 | 0.130 | |
| Ser. mars. A20019 | 0.030 | |
| Ps. aeru. A9843 | 0.500 | |
| Ps. aeru. CarbR | 2.000 | |
| Ps | | |
| PD50, | IM | PO |
| S. aur. | | |
| E. coli | | 25.00 |
| P. mir. | | |
| P. aeru. | | |
| t1/2, pH 7.4 (h.): | | 2 |
| t1/2, pH 2 (min.): | | 31 |
| Cmax, PO/50: | | 1.29<br>3 |

Procedure 59

(5S,6R) 6-(1′S-N-Trimethylammonium-ethyl)-4R-methyl-3-methylthio-7-oxo-1-azabicyclo[3.2.0]hept-2-ene-2-carboxylate (40806)

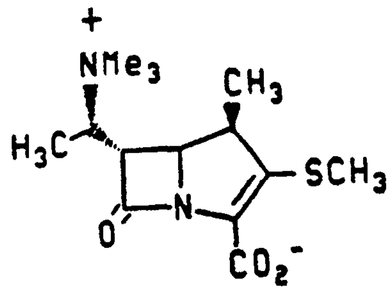

This substance is the 1'S-isomer of the product of Procedure 36 and may be prepared in substantially similar fashion employing the product of Procedure 56 as starting material. The product was chromatographed on reversed phase silica gel (Waters μ-Bondapak c-18, 4 x 10 cm. The uv active fractions were concentrated under vacuum and chromatographed a second time to give the title material as a white powder after lyophilization: hplc on μ-Bondapak c-18, 4 mm x 30 cm, 5% $CH_3CN$-$H_2O$ pH 7.0 phosphate buffer, flow rate 1 mL/min, uv detector at 300 nm, retention time 7.4 min, purity 97.5%; $[\alpha]_D^{22}$ + 129° (c 1.0, $H_2O$); uv ($H_2O$), pH 7.4 phosphate buffer) $\lambda_{max}$: 304 nm (9,834);
ir (KBr) $\nu_{max}$: 1750 (C=O of β-lactam) and 1600 $cm^{-1}$ (C=O of carboxylate);
'Hmr (200 MHz, $D_2O$) δ: 1.28 (d, J = 7.25 Hz, 3H, $CH_3$-4), 1.57 (d, J=6.61 Hz, 3H, $CH_3CHN$), 2.37 (s, 3H, $SCH_3$-3), 3.18 (s, 9H, $NCH_3$), 3.57 (m, 1H, H-4), 3.91 (dd, $J_{H6,H5}$ = 2.41 Hz, $J_{H6,H1'}$ = 6.22 Hz, 1H, H-6), 4.12 (m, 1H, H-1'), and r.30 ppm (dd, $J_{H5,H6}$ = 2.41 Hz, $J_{H5,H4}$ = 8.63 Hz, 1H, H-5).

By uv this carbapenem was found to have a half-life of 20 h at 30° C in a pH 7.4 phosphate buffer. In a pH 2 citric acid buffer at 37° C, the half-life was evaluated to be 4.8 h.

| M.I.C. | |
|---|---|
| Str. pneu. A9585 | 0.500 |
| Str. pyo. A9604 | 1.000 |
| Str. faec. A20688 | 125.000 |
| Staph. aur. A9537 | 2.000 |
| Staph. serum | 8.000 |
| Staph aur. Pen+ | 8.000 |
| Staph aur. MR | 125.000 |
| E. coli A15119 | 8.000 |
| E. coli A20341 | 8.000 |
| K. pneu. A9664 | 16.000 |
| K. pneu. A20468 | 32.000 |
| Ent. clo. A9659 | 32.000 |
| Ent. clo. A9656 | 8.000 |
| P. mir. A9900 | 4.000 |
| P. vul. A21559 | 8.000 |
| P. morg. A15153 | 16.000 |
| P. rett. A22424 | 16.000 |
| Ser. mars. A20019 | 16.000 |
| Ps. aeru. A9843 | 16.000 |
| Ps. aeru. CarbR | 63.000 |
| t1/2, pH 7.4 (h.): | 20 |
| t1/2, pH 2 (h.): | 4.8 |

## Procedure 60

(5S,6R) 6-(1'S-aminoethyl)-3-ethylthio-4R-methyl-7-oxo-1-azabicyclo[3.2.0]hept-2-ene-2-carboxylic acid

(40833)

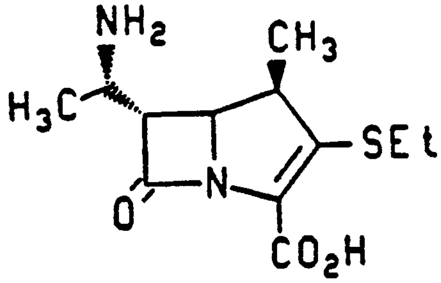

This compound is the 1′S-isomer of the compound of Procedure 7. Allyl (5S,6S) 3-ethylthio-6-(1′R-hydroxyethyl)-4R-methyl-7-oxo-1-azabicyclo[3.2.0]hept-2-ene-2-carboxylate may be transformed to the 1′S-azido compound as described in Procedure 6 for its isomer, and thence to the desired product as described in Procedure 7 for the isomer. Crystallization from water gave needles: hplc on μ-Bondapak c-18, 4 mm x 30 cm, 5% $CH_3CN$-$H_2O$ pH 7.0 phosphate buffer flow rate 1 mL/min, uv detector at 304 nm, retention time 8.9 min, purity 99.7%; uv ($H_2O$ pH 7.4 phosphate buffer) $\lambda_{max}$: 304 nm (12,356);
ir (KBr)$\nu_{max}$: 1760 (C=O of β-lactam) and 1580 $cm^{-1}$ (broad, C=O of carboxylate);
′Hmr (200 MHz, $D_2O$) δ: 1.22 (d, J = 7.01 Hz, 3H, $CH_2$-4), 1.27 (t, J = 7.39 Hz, 3H, $SCH_2CH_3$), 1.43 (d, J = 6.71 Hz, 3H, $CH_3CHN$), 2.84 (m, 2H, $SCH_2CH_3$), 3.49 (m, 1H, H-4), 3.61 (dd, $J_{H6,H5}$- 2.38 Hz, $J_{H6,H1'}$ = 7.58 Hz, 1H, H-6), 3.88 (m, 1H, H-1′) and 4.22 ppm (dd, $J_{H5,H6}$ = 2.38 Hz, $J_{H5,H4}$ = 8.78 Hz, 1H, H-5).

| | | | | | |
|---|---|---|---|---|---|
| Anal. calcd. for $C_{12}H_{18}N_2O_3S$: | C 53.31, | H 6.71, | N 10.36, | S 11.86; | |
| Found: | C 51.22, | H 6.85, | N 10.01, | S 11.06; | $H_2O$ 2.9%. |

By uv, this carbapenem was found to have a half-life of 3.0 h at 37° C in a pH 7.4 phosphate buffer. By hplc in a pH 2.0 citric acid buffer at 37° C, the half-life was evaluated to be 32 min.

| M.I.C. | | |
|---|---|---|
| Str. pneu A9585 | 2.000 | |
| Str. pyo A9604 | 2.000 | |
| Str. faec. A20688 | 125.000 | |
| Staph. aur. A9537 | 8.000 | |
| Staph. serum | 8.000 | |
| Staph aur. Pen + | 16.000 | |
| Staph aur. MR | 125.000 | |
| E. coli A15119 | 0.030 | |
| E. coli A20341 | 0.030 | |
| K. pneu. A9664 | 0.060 | |
| K. pneu. A20468 | 0.130 | |
| Ent. clo. A9659 | 0.060 | |
| Ent. clo. A9656 | 0.030 | |
| P. mir. A9900 | 0.016 | |
| P. vul. A21559 | 0.030 | |
| P. morg. A15153 | 0.030 | |
| P. rett. A22424 | 2.000 | |
| Ser. mars. A20019 | 0.250 | |
| Ps. aeru. A9843 | 0.250 | |
| Ps. aeru. CarbR | 2.000 | |
| PD50, | IM | PO |
| S. aur. | | |
| E. coli | | 25.00 |
| t1/2, pH 7.4 (h.): | | 3 |
| t1/2, pH 2 (min.): | | 32 |
| DHP: | | 14:40 |

Procedure 61

(5S,6R) 6-(1′S-(L-Leucylglycyl)aminoethyl)-4R-methyl-3-methylthio-7-oxo-1-azabicyclo[3.2.0]hept-2-ene-2-carboxylic Acid (41062)

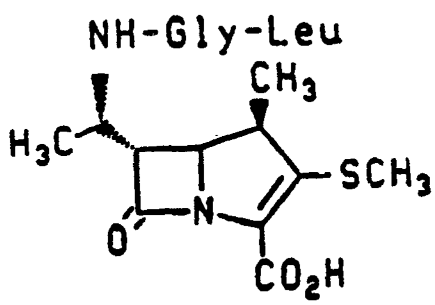

This product is the 1′S-isomer of the product of Procedure 37 and may be prepared in similar fashion using as starting material intermediates used in the synthesis of the product of Procedure 56. Chromatography of an aqueous solution of the product on reversed phase silica gel (μ-Bondapak c-18, 3 x 11 cm) using a mixture of water and acetonitrile (9:1) as eluent gave 0.410 g (64%) of the title carbapenem as a white amorphous powder after freeze drying: $[\alpha]^{22}_{D}$ + 67.8° (c 1.0, $H_2O$);hplc on μ-Bondapak C-18, 4 mm x

30 cm, 10% $CH_3CN$-$H_2O$ pH 7.4 phosphate buffer, flow rate 2 mL/min, uv detector 304 nm, retention time 5.5 min, purity 96%; uv ($H_2O$), pH 7.4 phosphate buffer) $\lambda_{max}$: 304 nm (11,100);
ir (KBr) $\nu_{max}$: 1750 (C=O of $\beta$-lactam), 1660 and 1540 (amide) and 1580 $cm^{-1}$ (broad, C+O of carboxylate); $^1$Hmr (200 MHz, $D_2O$) $\delta$: 0.96 and 0.97 (two overlapping d, J=5.91 Hz and J=5.80 Hz, 2 x 3H, $CH_3$ of lysine), 1.18 (d, J=7.18 Hz, 3H, $CH_3$-4), 1.31 (d, J=6.88 Hz, 3H, $CH_3CHN$), 1.72 (m, 3H, H-3 and H-4 of lysine), 2.35 (s, 3H, $SCH_3$-3), 3.45 (m, 1H, H-4), 3.51 (dd, $J_{H6,H5}$ = 2.05, $J_{H6,H1'}$ = 5.88 Hz, 1H, H-6), 3.97 (dd, overlapping with $CH_2$ of glycine, $J_{H5,H6}$ = 2.05 Hz, 1H, H-5), 3.99 (s, 2H, $CH_2$ of glycine), 4.05 (t, J=7.4 Hz, H-2 of lysine) and 4.35 ppm (m, 1H, $CH_3CHN$).

By uv at 37°C in a pH 7.4 phosphate buffer, this carbapenem was found to have a half-life of 30 h. By hplc, at 37°C in a pH 2.0 citric acid buffer, the half-life was evaluated to be less than 5 min.

| M.I.C. | |
|---|---|
| Str. pneu. A9585 | 63.000 |
| Str. pyo. A9604 | 63.000 |
| Str. faec. A20688 | 125.000 |
| Staph. aur. A9537 | 125.000 |
| Staph. serum | 63.000 |
| Staph aur. Pen+ | 125.000 |
| Staph aur. MR | 125.000 |
| E. coli A15119 | 125.000 |
| E. coli A20341 | 125.000 |
| K. pneu A9664 | 125.000 |
| K. pneu A20468 | 125.000 |
| Ent. clo. A9659 | 125.000 |
| Ent. clo. A9656 | 125.000 |
| P. mir. A9900 | 125.000 |
| P. vul. A21559 | 125.000 |
| P. morg. A15153 | 125.000 |
| P. rett. A22424 | 125.000 |
| Ser. mars. A20019 | 125.000 |
| Ps. aeru. A9843 | 125.000 |
| Ps. aeru. CarbR | 125.000 |
| t1/2, pH 7.4 (h.): | 30 |

## Claims

1. The 6-(1-aminomethyl)-1-azabicyclo-[3,2,0]-hept-2-ene having Formulas V or Formula XII

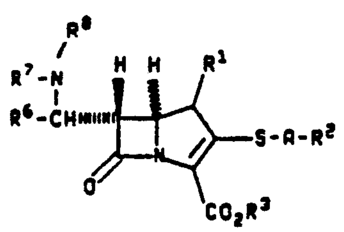

(V)

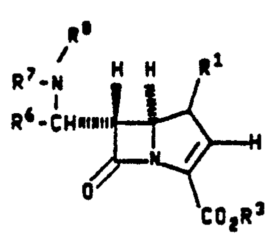

(XII)

wherein
$R^1$ is lower alkyl having 1 to 6 carbon atoms, R- or S- orientation,
A is a bond or it is a straight or branched alkylene group having 1 to 6 carbon atoms connecting the sulfur atom and $R^2$,

$R^2$ is hydrogen or a substituent other than amino but including methyl, hydroxy, cyano, $-CO_2R^3$, halo, carboxamido, carbamyl, phenyl, B-substituted phenyl, heterocyclo, or B-substituted heterocyclo, each of said heterocyclo having 4 to 8 ring members including at least one carbon, and from 1 to 4 heteroatoms selected from O, S, and N,
$R^3$ is H, lower alkyl having 1 to 6 atoms, aralkyl having 7 to 17 carbon atoms, an anionic charge, a salt forming group, a protective ester group, or a physiologically hydrolyzable ester group,
$R^6$ is hydrogen, lower alkyl having 1 to 6 carbon atoms, or phenyl, wherein each of said alkyl and said phenyl optionally has one or two substituents B,
$R^7$ and $R^8$ are selected independently from hydrogen, alkyl having 1 to 6 carbon atoms, aralkyl having 7 to 17 carbon atoms, said alkyl and aralkyl optionally having one or two substituents B, or one of $R^7$ and $R^8$ is carbacyl, sulfonyl, or phosphonyl having 1 to 17 carbon atoms, and the other is hydrogen
wherein said substituents B are selected from halogen, nitro, methoxy, hydroxy, methyl, cyano, carboxy, carboxamido, sulfo, sulfonamido, carbamyl, and guanyl, and the pharmaceutically acceptable salts of the compounds of Formulas V and XII.

2. The compound of Claim 1 wherein the carbon atom bearing $R^1$ and the carbon atom bearing $R^6$, when $R^6$ is other than hydrogen, are independently in either the R-configuration or the S-configuration.

3. The compound of Claim 1 wherein the carbon atom bearing $R^1$ is in the R-configuration.

4. The compound of Claim 1 wherein each of the carbon atoms bearing $R^1$ and $R^6$, when $R^6$ is other than hydrogen, are in the R-configuration.

5. The compound of Claims 1, 3, or 4 wherein each of $R^1$ and $R^6$ is methyl.

6. The compound of Claim 1, 3, or 5 wherein each of $R^7$ and $R^8$ is hydrogen.

7. The compound claimed in Claim 1 and having Formula V, with the following meanings for $R^1$, A, $R^2$, $R^3$, $R^6$, $R^7$, and $R^8$, and the stereochemical configurations specified in parentheses relative to the carbon atoms bearing $R^1$ and $R^6$

| Compound Number | $R^1$ | $R^2$ | A | $R^3$ | $R^6$ | $R^7$ | $R^8$ |
|---|---|---|---|---|---|---|---|
| 40383 | $CH_3$(R) | $CH_3$ | bond | H | $CH_3$(S) | H | H |
| 40591 | $CH_3$(R) | $CH_3$ | bond | H | $CH_3$(R) | H | H |
| 40592 | $CH_3$(R) | $CH_3$ | bond | K | $CH_3$(S) | H | * |
| | | * $R^8$ is phenoxyacetyl | | | | | |
| 40593 | $CH_3$(R) | $CH_3$ | bond | K | $CH_3$(S) | H | * |
| | | * $R^8$ is acetyl | | | | | |
| 40732 | $CH_3$(R) | 3-pyr-idyl | $CH_2$ | - | $CH_3$(S) | H | H |
| 40741 | $CH_3$(R) | $CH_3$ | bond | H | $CH_3$(S) | H | glycyl |
| 40767 | $C_2H_5$(R) | $CH_3$ | bond | H | $CH_3$(S) | H | H |
| 40806 | $CH_3$(R) | $CH_3$ | bond | - | $CH_3$(S) | $CH_3$ | $(CH_3)_2$* |
| | | *methyl quaternary ammonium | | | | | |
| 40833 | $CH_3$(R) | $C_2H_5$ | bond | H | $CH_3$(S) | H | H |
| 40849 | $CH_3$(R) | $CH_3$ | bond | H | $CH_3$(S) | $CH_3$ | $CH_3$ |
| 40850 | $CH_3$(R) | $CH_3$ | bond | H | $CH_3$(S) | H | $CH_3$ |
| 40886 | $CH_3$(R) | 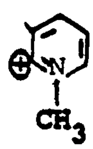 | $CH_2$ | - | $CH_3$(S) | H | H |
| 40931 | $CH_3$(R) | 3-pyridyl | $CH_2$ | H | H | H | H |
| 40945 | $CH_3$(R) | $CH_3$ | bond | - | $CH_3$(R) | $CH_3$ | $(CH_3)_2$* |
| | *methyl quaternary ammonium | | | | | | |
| 40992 | $CH_3$(R) | phenyl | bond | H | $CH_3$(S) | H | H |
| 41004 | $CH_3$(R) | $CH_3$ | bond | H | $CH_3$(S) | H | * |
| | *$R^8$ is L-alanyl | | | | | | |

| Compound Number | $R^1$ | $R^2$ | A | $R^3$ | $R^6$ | $R^7$ | $R^8$ |
|---|---|---|---|---|---|---|---|
| 41062 | $CH_3$(R) | $CH_3$ | bond | H | $CH_3$(S) | H | * |
| | *$R^8$ is L-leucylglycyl | | | | | | |
| 41070 | $CH_3$(R) | $CH_3$ | bond | H | $CH_3$(S) | H | L-leucyl |
| 41116 | $CH_3$(R) | $C_2H_5$ | bond | H | $CH_3$(R) | H | H |
| 41117 | $C_2H_5$(R) | $CH_3$ | bond | H | $CH_3$(R) | H | H |
| 41118 | $CH_3$(R) | phenyl | $CH_2$ | H | $CH_3$(R) | H | H |
| 41144 | $CH_3$(R) | $CH_3$ | bond | H | $CH_3$(R) | H | * |
| | *$R^8$ is L-leucyl-glycyl | | | | | | |
| 41145 | $CH_3$(R) | $CH_3$ | bond | H | $CH_3$(R) | H | * |
| | *$R^8$ is L-alanylglycyl | | | | | | |
| 41178 | $CH_3$(R) | * | $CH_2$ | H | $CH_3$(R) | H | H |
| | *$R^2$ is 1-methyl-1,2,3-triazol-4-yl | | | | | | |
| 41212 | $CH_3$(R) | 2-pyridyl | $CH_2$ | H | $CH_3$(R) | H | H |
| 41214 | $CH_3$(R) | 3-pyridyl | $CH_2$ | H | $CH_3$(R) | H | H |
| 41248 | $CH_3$(R) | $CH_3$ | bond | H | $CH_3$(R) | H | * |
| | *$R^8$ is L-alanyl-L-leucyl | | | | | | |
| 41280 | $C_2H_5$(R) | $-CH(CH_3)_2$ | bond | H | $CH_3$(S) | H | H |
| 41282 | $C_2H_5$(R) | * | $-CH_2-$ | H | $CH_3$(S) | H | H |
| | *$R^2$ is 1-methyl-1,2,3-tetrazol-4-yl | | | | | | |

| Compound Number | $R^1$ | $R^2$ | A | $R^3$ | $R^6$ | $R^7$ | R |
|---|---|---|---|---|---|---|---|
| 41330 | $CH_3$(R) | 2-pyridyl | $CH_2$ | H | $CH_3$(S) | H | H |
| 41376 | $CH_3$(R) | 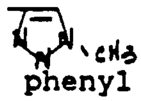 | $CH_2$ | H | $CH_3$(S) | H | H |
| 41385 | $CH_3$(R) | phenyl | $CH_2$ | H | $CH_3$(S) | H | H |
| 41416 | $CH_3$(R) | F | $CH_2CH_2$ | H | $CH_3$(R) | H | H |
| 41443 | $CH_3$(R) | F | $CH_2CH_2$ | H | $CH_3$(S) | H | H |
| 41646 | $CH_3$(R) | CN | $CH_2CH_2$ | H | $CH_3$(R) | H | H |
| 41656 | $C_2H_5$(R) | F | $CH_2CH_2$ | H | $CH_3$(S) | H | H |
| 41787 | $CH_3$(R) | CN | $CH_2CH_2$ | H | $CH_3$(S) | H | H |
| 41843 | $CH_3$(R) | NHCHO | $CH_2CH_2$ | H | $CH_3$(S) | H | H |
| 41930 | $CH_3$(R) | NHCHO | $CH_2CH_2$ | H | $CH_3$(R) | H | H |
| 42174 | $CH_3$(R) | OH | $CH_2CH_2$ | H | $CH_3$(S) | H | H |
| 42271 | $CH_3$(R) | OH | $CH_2CH_2$ | H | $CH_3$(R) | H | H |
| 42484 | $CH_3$(R) | CN | $CH_2$ | H | $CH_3$(R) | H | H |
| 42485 | $CH_3$(R) | CN | $CH_2$ | H | $CH_3$(S) | H | H |

8. The compound claimed in Claim 1 and having Formula XII with the following meanings for $R^1$, $R^3$, $R^6$, $R^7$, and $R^8$ and the stereochemical configurations specified in parenthesis relative to the carbon atoms bearing $R^1$ and $R^6$.

| Compound Number | $R^1$ | $R^3$ | $R^6$ | $R^7$ | $R^8$ |
|---|---|---|---|---|---|
| 42425 | $CH_3$(R) | H | $CH_3$(R) | H | H |
| 42426 | $CH_3$(R) | H | $CH_3$(S) | H | H |

9. The process for the preparation of a compound defined in Claim 1 wherein $R^7$ and $R^8$ are hydrogen which comprises (a) reacting a first beta lactam compound of Formulas VI, VII, VIII, IX, or X

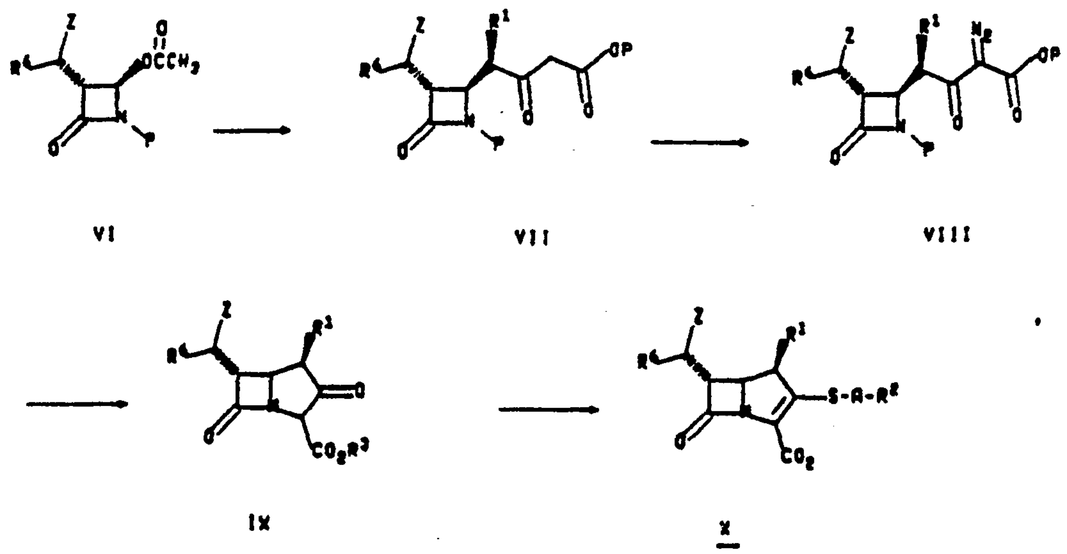

VI VII VIII

IX X

wherein Z is OH with triphenylphosphine, hydrazoic acid, and dialkyl azodicarboxylate under reaction conditions which are known to bring about displacement of -OH with $-N_3$ with inversion of configuration at the carbon atom to which $R^6$ is attached and provide a second beta lactam compound of Formulas VI, VII, VIII, IX, or X wherein Z is $-N_3$ and having the opposite configuration at the carbon atom to which $R^6$ is attached with respect to said first beta lactam compound; (b) reducing the azido group of said second beta lactam compound to the amino group and provide a third beta lactam compound of Formulas VI, VII, VIII, IX, or X wherein Z is $-NH_2$; and (c) when a compound defined in Claim 1 by Formula V is desired converting either said second beta lactam compound, or said third beta lactam compound through that portion of the sequence of intermediates having the Formula Numbers shown above as VI →, VII →, VIII →, IX → X in which each of said intermediates has a Formula Number higher than that of said second or said third beta lactam coupound, or wherein Z is azido, amino, or protected amino, and converting resulting compound of Formula X to the desired product of Formula V, or said resulting compound of Formula IX to the desired product of Formula XII.

10. The use of at least one compound of anyone of claims 1 to 8 for preparing a pharmaceutical composition for the treatment of an infection in a plant or an animal caused by a self-replicating infectious agent.

11. A pharmaceutical composition, preferably in dosage unit form, comprising at least one compound according to anyone of claims 1 to 9 and a pharmaceutical carrier therefore.

12. A process for preparing the pharmaceutical composition of claim 11 which comprises incorporating at least one compound of anyone of claims 1 to 9 into a pharmaceutical carrier.